# EUROPEAN PATENT APPLICATION

(11) **EP 3 628 160 A1**
(43) Date of publication of application: **01.04.2020**
(21) Application number: 18196615.1
(22) Date of filing: 25.09.2018
(51) Int. Cl.: A01N 57/20, A01N 25/00, A01P 13/00, C12N 15/82

(54) **USE OF GLYPHOSATE HERBICIDE FOR CONTROLLING UNWANTED VEGETATION IN BETA VULGARIS GROWING AREAS**

(71) Applicant: KWS SAAT SE & Co. KGaA, 37574 Einbeck (DE)
(72) Inventor: Czarnecki, Olaf, 13055 Berlin (DE); Gertz, Maik, 30167 Hannover (DE); Lein, Jens Christoph, 37085 Göttingen (DE); Wurbs, David, 37574 Einbeck (DE)

(57) **Abstract**

The present invention relates to the use of a glyphosate herbicide for controlling unwanted vegetation in Beta vulgaris growing areas in which the Beta vulgaris plants comprise an endogenous allele encoding an epsp synthase having at position 179 an amino acid different from proline.

SEQUENCE LISTING

<110>  KWS SAAT SE

<120>  USE OF GLYPHOSATE HERBICIDE FOR CONTROLLING UNWANTED VEGETATION
       IN BETA VULGARIS GROWING AREAS

<130>  KWS0288EP2

<160>  21

<170>  PatentIn version 3.5

<210>  1
<211>  7001
<212>  DNA
<213>  Artificial Sequence

<220>
<223>  genomic DNA of mutated epsps gene (P179S)

<400>  1
aggaagtatt tgaatttgat atagatattg tgtctttgtg tgtgttgaat ttcaattccc      60

agttccctaa aaaaaattta caattgcaat ttcgagatta tgatgtaaat taaatttgag     120

agactagaaa gtatttggtc aacccaaaaa aaaaatatca atacttatat aaatcaaaaa     180

cataatagag aatccaattt tactaaaaat attagtaatt ttgattaaaa taatctatta     240

aaatgaactc taaccttcac ataatttcca catattatta atcaacaaaa taagcatcac     300

aaattattag aataggcgat ctaattttaa cataaaatta gacgaattca aattgaattt     360

ttctaacaag ctcattccat ttcacgcaac ccaaaattat cctagtcagt agtcatccat     420

tcttttctca ttcctttatt cttgattatc gaactacaac agataatttc aaaaaaaaac     480

taaattggta gtcttaactg attaaactac ttactaaatg gattaaagaa tgtcattact     540

gaatagatta aactgattac gaaatagatt aacttggtcc ctaaatagat taaattagtt     600

actatattaa aattaggcga tctcttacaa aaccaactga ataagcatag ctctgtatat     660

tacctagatt tcaactaaat caaaacccct tacagttcaa tctagagctg atcattttgg     720

ctcggcccgt cccatttttg ggccgggttt tagtcagatt tttttggccc gcggtcgggc     780

ccggcccgat ttttttggct ttgggcaagc caaaaacgac ttttcagttt attttttggc     840

ccgacccgtt tttacccgca aaagcccgct aatttaggtc cgcactttgg gcacaaaaat     900

ttagcccgaa cttaaacctg gcccgaccca tgatcacctc tagtttaatc caaactaaaa     960

aactacacaa gttagccaaa aattatgtct actttgtaca actttataaa atacacacag    1020

tagttgatat cttgatgatt aactcctttt gaagtttgac tacacaccaa ccccaaacac    1080

acccactttt tcccccctct tgtcaccaac cccccctcct ctttagccac caaagtttgg    1140

ttggtgagtc ctccataact gctaaattct ctcttttttc tctctcctaa aaaactaaaa    1200

cccaccaaaa tttcagacat caaaaaaatt acaagtgaag gaaacaataa tggctcaagc    1260

tagcaccata aacaatggtg tcaaaagcac ccaattatgc cccaatttac ccaaaaccca 1320

cttatccaaa tcttcaaaat ctgttaaatt tggatcaaat ttgagatttt ctccaaagtt 1380

gaaatctttt aacaatgaaa gagttggtgg gaattcatca gttgttttca gggttagggc 1440

ttcagttgca gcagcagctg aaaaatcatc aactgtacca gaaattgtgt tacaacccat 1500

caaagagatc tcgggtacca ttcaattgcc cggatccaag tcattatcta atcggattct 1560

acttcttgct gccctttctc aggtactttt caattgtttg atttcttttt ttcttagaac 1620

ttgtgaattt gtatacttta tccgtttcta aatacgtgca acatttgaat agtaacgagt 1680

atttatctac caacttattt aatattctct cacgaatgta tatgaaaaaa tatagtcatg 1740

cgtggtttta tttgattgat ctgcggactt ttataatatc aactttttat aatttagagg 1800

acaaagtagt gtattgggta gcgtgtaagg aggttgggaa actggaggaa ttttttaaca 1860

attcaagttt gatatttttc atagtgaaat gtttattagc atagaatcat gcttttagtt 1920

tttagtggag tgtgcattta ttctttaact tgttggatgg ctatgattaa gaattggatt 1980

ctggttattt gcttgagtat ttagaaatta attgtgggtg ttggtgataa tgtaacaaaa 2040

ttgttttgaa ggtgtgagaa tgtgatttta attatatgag gaaatgatgg gttatttatt 2100

gtaatgtggg aatttatgat aatatgttgg aggatgaaac aattgatgat tttgaagtgg 2160

ggtatgaagg ctttccccca tttttttacc tttcatgtgt tgtatgtagc agactgccaa 2220

agcaattctt ctgagcagct tggcatattt ccattgcaac tcataatctc aatccaagca 2280

caatcaatta cctgccacgc tagcacttta agtagcaaaa tcgcgcttga agaaaagata 2340

ataccacagg cttctatagt tctattcctt gtgatatagg acacaagtat catcctagat 2400

gacttcccta gccctgcaaa taattcctgg aaggaagtcc gttgagtatc cttgatcaag 2460

taaaattagc gctttctttt gggtaacaca aactatactc gaaccacttt atgctaaggg 2520

atcacattgc gtctcttgca gaacttttaa catttctaac atggcataag cacggaaaca 2580

ccctgtgaag ttgctagtga ccatcttttt tgataaatca agaatccctg ttcttatttg 2640

catacaaagg gttaagtctt gtctgagagg gattgacata tctcctgctg gatagacttt 2700

gtgttcatct ctaatgttat tctgagtttg cattgtatgt gttgtgtttt cgtttttctt 2760

cttgtttgtg tgtgtgtgtg tgtgtgtgtg tgtgtgttac ccaaacttgt acggaaattt 2820

ccatcctaac gagaattatg ctgcagggaa caacagtggt tgacaacttg ctatatagtg 2880

atgatatccg ttacatgctg gatgctttga gaactcttgg gttgaatgtg gaggatgata 2940

atatagccaa aagggcaatc gtggagggtt gcggtggttt gtttcctgtt ggaaaagatg 3000

ggaaagaaat tgaacttttt cttggaaatg caggaacagc aatgcgctca ttgacagctg 3060

cagttgctgt tgctggagga aattctaggt ttgtctacta aggccttcca tgtcatcgtg 3120

aggctgttaa tattctttta ttttgtgtgg agacttgttt cctcttgcta caattgcaaa 3180
gggggctggg attgattaac aatgcagaac tcaaacacta tctttggtat caccgtctgg 3240
caggtggcat ctgatcttgc aaaacagata tggagggttt tgccggaaag gggttggggg 3300
tgggacttgg gagtatagcc tcaatgttat agaagattgt gctatactac tgtaaaatta 3360
acttttggtt ggaaccagat atagttaata aagtattttt ttatctggtg aggggtgctt 3420
ctaggtcctg aaaagttcca ccatagaaaa aaattgaatt tcattgtgaa aagctaaggt 3480
gaattcatta aattcaatac atgttgttga ttgcttgaac tagaatactt cagatatcag 3540
ttaattgaat ttatttcttt tggagaacat gcatgaagag cttttaactc gcctctacta 3600
tcctgctctt aattattttt ataaatagat ttagcaattg ggaatatcct gtaatttgct 3660
ggttgctttt tcatatggta tatattgaat tggtgtggag gcgttttaca aagagtgaag 3720
gtaattgttg atgggtggat attgaatgga aatactttga tggcggatag catattcatc 3780
tgattggatg tctgaaattt gatgagctct agtaaatgtt cctccatgta tgcttgtgta 3840
ttgggaattt gcaatccagt agtagagaac acccctgata attgcactat tattcctgag 3900
ttgtgagagc tctcaagaag atagatgggg ttcttcaagt tttttaattg tctttaggta 3960
gtgtaaaggt tgcaatattt caaaggatct gtctgatcca acaagtctgc taaatccttc 4020
accaatctga aaggggagaa gatactgctc tctctgacct gcttgcatgc atataaagct 4080
tctaaaacaa ataggttaaa tgtaaaacaa gcgcagaagt tttcacttct cttgggaaat 4140
gtgtgccaag atctattgca tgacatgaat tcatatttat tgattatatg ctcaacatga 4200
atgggattaa gaatgaaata aagtagcatg agtaggagac aatccgcctg ctcttttaat 4260
aggcatatac aatcagtcat caacatcatt gctgctttag aatttgattt atgttgatct 4320
tggtttttgc agctatgtac ttgatggagt gccgagaatg agggagcgac ccattgggga 4380
tttggtagcg ggtctaaagc aacttggcgc cgatgttgac tgttatcttg gcacaaattg 4440
tcctcctgtt cgagtgaatg ctaaaggagg ccttcccggg ggcaaggtga attctcttgt 4500
tttgattaga gaaaaattat aagttagtgt tctatcatgc ttgacacgaa caagtttttt 4560
tatctgaaag agaaagaagt gtatgcctaa tgattaacag tcatattcaa aatgatataa 4620
aagagggtgg aaggttactt gctctatgat gatttatggt agtaccgtgt taccaatagc 4680
agaattcaag caatattctt tgaactggta ataacaagtt caagcaatca agcatagttt 4740
agattagatt ctatcaatct cttagcatag tttaaattcg cttccatcaa tctcttttgt 4800
gacgtaagaa gttctgttcc attatactta tgctttttgt gaaattttat ttgcaggtca 4860
agctctctgg atcagttagt agccaatatt tgactgcact gcttatggct actcctttgg 4920
gtcttggaga cgtggaggtt gaaatcattg ataaattgat ttctgtacca tatgtggaga 4980
tgacaataaa gctaatggaa aggtttggag tgtctgtaga gcatagtgct gactggggta 5040

ggttcttgat ccgaggtggt cagaagtaca agtaagtctc tcttttttac tacgtgtcct 5100

gaaaaatcta tgttttagta gactaagata ctataaaata catcagatct cctggaaatg 5160

cgtatgttga gggtgatgct tcaagtgcta gttacttcat aggaggggcc gcagtcactg 5220

gtgggactgt gactgttgag ggttgtggaa caagtagttt acaggtatga tttaaagcct 5280

tatttcacat ccttttactt ctctccttac tgcatctcag cttaattctg aagaactctt 5340

gttgctcaat ctctaggcat aagcttgttc tttcgacctt ctaaatttgc aaaatcattc 5400

tgttaatgta acaaacagaa gatgagatgg tgtttttgat cctgataaaa aaaattagga 5460

cattcatgat tggtttatgt tctatgacaa tttcgatgat ctcttcaatt gtaacttgag 5520

gacccttttc tgctagtaaa gattgtgtat aagctattct agactgttgt atacacctca 5580

tattccggta ttgtttatac ctaataaact tcttatgatt agtatcttac ttgtctcatg 5640

tgttttgcat agggtgatgt aaaatttgct gaagttcttg agaagatggg ttgcaaggta 5700

tcctggacag agaacagtgt cactgtcact ggaccaccca gggatgcatc tggaagaaaa 5760

cacttgcgcg ccgttgatgt caacatgaac aaaatgccag atgttgcaat gactcttgct 5820

gttgttgctc tttatgcaga tggacccacc accattagag acggtatgct taattccttt 5880

ctgtgaacat gacactcttc ttgtgccata ggtagattcc gtagttattg gtcacaatcc 5940

aaatttgcat tggtatttaa agcaagtctt aggtggtgag accgcttgaa acccataata 6000

taaaagaaag gtcaccttgg tctcaaagtt aattcttgtg agaccatctc atgcaagatt 6060

ttgccgggta tctaatcctt gtttgcactg aggaaaagga aaaaaattaa catgacctat 6120

tagtaagcca aaaaaattct aacaagagaa gtagccgaat aggtaataat ttttgtgata 6180

taatgatcag tggctagctg gagagtgaag gaaacagaac ggatgattgc gatttgcaca 6240

gagctcagaa aggttagcca ttttggatat acacattctt gaaggttttc aaccttgtag 6300

aagattgtac tattgataaa aaaaattgat gtttatccgg tgtacttcga aatttctttt 6360

cagctggggg caacagttga ggaaggatca gattactgtg tgatcactcc acctgagaaa 6420

ctaaatgtga cggccattga tacatacgat gatcaccgaa tggccatggc attctctctt 6480

gctgcctgcg ccgatgttcc tgttaccatc aaggacccgg gttgcactcg caagactttc 6540

ccagactact ttgatgtgtt ggaaaggttt gcaaagcatt aagtggtctc ctacatattc 6600

tataaagcat aagctgagat tttttgagag aattaggaga tgaaaaatgc tttctgcttg 6660

agttatcatc acattctttg tattatgatt gtaagattat tatagtatag agtttacaaa 6720

gtactactaa taattgttat gtatccgatt gatcagaaat aagttaattg gaaggctgga 6780

ctttgaaaat gtgaccaaga cactagtgtg accaagtcat tttgttaatg tgagttcaat 6840

gttattgatt caacatgtag agccaaatct caattctatc gtcacttcat atgaccaaaa 6900

atctaaagat gaaaaagtaa aaaagagcat gttggatcaa actctagctg tatctctgaa 6960

attcaatcac gcagttagat caaatgagga ttaaagggag t 7001

<210> 2
<211> 1554
<212> DNA
<213> Artificial Sequence

<220>
<223> cDNA of mutated epsps gene (P179S)

<400> 2
atggctcaag ctagcaccat aaacaatggt gtcaaaagca cccaattatg ccccaattta 60

cccaaaaccc acttatccaa atcttcaaaa tctgttaaat ttggatcaaa tttgagattt 120

tctccaaagt tgaaatcttt taacaatgaa agagttggtg ggaattcatc agttgttttc 180

agggttaggg cttcagttgc agcagcagct gaaaaatcat caactgtacc agaaattgtg 240

ttacaaccca tcaaagagat ctcgggtacc attcaattgc ccggatccaa gtcattatct 300

aatcggattc tacttcttgc tgccctttct cagggaacaa cagtggttga caacttgcta 360

tatagtgatg atatccgtta catgctggat gctttgagaa ctcttgggtt gaatgtggag 420

gatgataata tagccaaaag ggcaatcgtg gagggttgcg gtggtttgtt tcctgttgga 480

aaagatggga aagaaattga actttttctt ggaaatgcag gaacagcaat gcgctcattg 540

acagctgcag ttgctgttgc tggaggaaat tctagctatg tacttgatgg agtgccgaga 600

atgagggagc gacccattgg ggatttggta gcgggtctaa agcaacttgg cgccgatgtt 660

gactgttatc ttggcacaaa ttgtcctcct gttcgagtga atgctaaagg aggccttccc 720

gggggcaagg tcaagctctc tggatcagtt agtagccaat atttgactgc actgcttatg 780

gctactcctt tgggtcttgg agacgtggag gttgaaatca ttgataaatt gatttctgta 840

ccatatgtgg agatgacaat aaagctaatg gaaaggtttg gagtgtctgt agagcatagt 900

gctgactggg gtaggttctt gatccgaggt ggtcagaagt acaaatctcc tggaaatgcg 960

tatgttgagg gtgatgcttc aagtgctagt tacttcatag gaggggccgc agtcactggt 1020

gggactgtga ctgttgaggg ttgtggaaca agtagtttac agggtgatgt aaaatttgct 1080

gaagttcttg agaagatggg ttgcaaggta tcctggacag agaacagtgt cactgtcact 1140

ggaccaccca gggatgcatc tggaagaaaa cacttgcgcg ccgttgatgt caacatgaac 1200

aaaatgccag atgttgcaat gactcttgct gttgttgctc tttatgcaga tggacccacc 1260

accattagag acgtggctag ctggagagtg aaggaaacag aacggatgat tgcgatttgc 1320

acagagctca gaaagctggg ggcaacagtt gaggaaggat cagattactg tgtgatcact 1380

ccacctgaga aactaaatgt gacggccatt gatacatacg atgatcaccg aatggccatg 1440

gcattctctc ttgctgcctg cgccgatgtt cctgttacca tcaaggaccc gggttgcact 1500

cgcaagactt tcccagacta ctttgatgtg ttggaaaggt ttgcaaagca ttaa 1554

<210> 3
<211> 517
<212> PRT
<213> Artificial Sequence

<220>
<223> protein of mutated epsps gene (P179S)

<400> 3

Met Ala Gln Ala Ser Thr Ile Asn Asn Gly Val Lys Ser Thr Gln Leu
1               5                   10                  15

Cys Pro Asn Leu Pro Lys Thr His Leu Ser Lys Ser Ser Lys Ser Val
            20                  25                  30

Lys Phe Gly Ser Asn Leu Arg Phe Ser Pro Lys Leu Lys Ser Phe Asn
        35                  40                  45

Asn Glu Arg Val Gly Gly Asn Ser Ser Val Val Phe Arg Val Arg Ala
    50                  55                  60

Ser Val Ala Ala Ala Ala Glu Lys Ser Ser Thr Val Pro Glu Ile Val
65                  70                  75                  80

Leu Gln Pro Ile Lys Glu Ile Ser Gly Thr Ile Gln Leu Pro Gly Ser
                85                  90                  95

Lys Ser Leu Ser Asn Arg Ile Leu Leu Leu Ala Ala Leu Ser Gln Gly
            100                 105                 110

Thr Thr Val Val Asp Asn Leu Leu Tyr Ser Asp Asp Ile Arg Tyr Met
        115                 120                 125

Leu Asp Ala Leu Arg Thr Leu Gly Leu Asn Val Glu Asp Asp Asn Ile
    130                 135                 140

Ala Lys Arg Ala Ile Val Glu Gly Cys Gly Gly Leu Phe Pro Val Gly
145                 150                 155                 160

Lys Asp Gly Lys Glu Ile Glu Leu Phe Leu Gly Asn Ala Gly Thr Ala
                165                 170                 175

Met Arg Ser Leu Thr Ala Ala Val Ala Val Ala Gly Gly Asn Ser Ser
            180                 185                 190

Tyr Val Leu Asp Gly Val Pro Arg Met Arg Glu Arg Pro Ile Gly Asp

195                 200                 205

Leu Val Ala Gly Leu Lys Gln Leu Gly Ala Asp Val Asp Cys Tyr Leu
    210                 215                 220

Gly Thr Asn Cys Pro Pro Val Arg Val Asn Ala Lys Gly Gly Leu Pro
225                 230                 235                 240

Gly Gly Lys Val Lys Leu Ser Gly Ser Val Ser Ser Gln Tyr Leu Thr
                245                 250                 255

Ala Leu Leu Met Ala Thr Pro Leu Gly Leu Gly Asp Val Glu Val Glu
            260                 265                 270

Ile Ile Asp Lys Leu Ile Ser Val Pro Tyr Val Glu Met Thr Ile Lys
        275                 280                 285

Leu Met Glu Arg Phe Gly Val Ser Val Glu His Ser Ala Asp Trp Gly
    290                 295                 300

Arg Phe Leu Ile Arg Gly Gly Gln Lys Tyr Lys Ser Pro Gly Asn Ala
305                 310                 315                 320

Tyr Val Glu Gly Asp Ala Ser Ser Ala Ser Tyr Phe Ile Gly Gly Ala
                325                 330                 335

Ala Val Thr Gly Gly Thr Val Thr Val Glu Gly Cys Gly Thr Ser Ser
            340                 345                 350

Leu Gln Gly Asp Val Lys Phe Ala Glu Val Leu Glu Lys Met Gly Cys
        355                 360                 365

Lys Val Ser Trp Thr Glu Asn Ser Val Thr Val Thr Gly Pro Pro Arg
    370                 375                 380

Asp Ala Ser Gly Arg Lys His Leu Arg Ala Val Asp Val Asn Met Asn
385                 390                 395                 400

Lys Met Pro Asp Val Ala Met Thr Leu Ala Val Val Ala Leu Tyr Ala
                405                 410                 415

Asp Gly Pro Thr Thr Ile Arg Asp Val Ala Ser Trp Arg Val Lys Glu
            420                 425                 430

Thr Glu Arg Met Ile Ala Ile Cys Thr Glu Leu Arg Lys Leu Gly Ala
        435                 440                 445

Thr Val Glu Glu Gly Ser Asp Tyr Cys Val Ile Thr Pro Pro Glu Lys
    450                 455                 460

Leu Asn Val Thr Ala Ile Asp Thr Tyr Asp Asp His Arg Met Ala Met
465                 470                 475                 480

Ala Phe Ser Leu Ala Ala Cys Ala Asp Val Pro Val Thr Ile Lys Asp
                485                 490                 495

Pro Gly Cys Thr Arg Lys Thr Phe Pro Asp Tyr Phe Asp Val Leu Glu
            500                 505                 510

Arg Phe Ala Lys His
        515

<210>  4
<211>  7001
<212>  DNA
<213>  Artificial Sequence

<220>
<223>  genomic DNA of mutated epsps gene (T175I and P179S)

<400>  4
aggaagtatt tgaatttgat atagatattg tgtctttgtg tgtgttgaat ttcaattccc      60

agttccctaa aaaaaattta caattgcaat ttcgagatta tgatgtaaat taaatttgag     120

agactagaaa gtatttggtc aacccaaaaa aaaaatatca atacttatat aaatcaaaaa     180

cataatagag aatccaattt tactaaaaat attagtaatt ttgattaaaa taatctatta     240

aaatgaactc taaccttcac ataatttcca catattatta atcaacaaaa taagcatcac     300

aaattattag aataggcgat ctaattttaa cataaaatta gacgaattca aattgaattt     360

ttctaacaag ctcattccat ttcacgcaac ccaaaattat cctagtcagt agtcatccat     420

tcttttctca ttcctttatt cttgattatc gaactacaac agataatttc aaaaaaaaac     480

taaattggta gtcttaactg attaaactac ttactaaatg gattaaagaa tgtcattact     540

gaatagatta aactgattac gaaatagatt aacttggtcc ctaaatagat taaattagtt     600

actatattaa aattaggcga tctcttacaa aaccaactga ataagcatag ctctgtatat     660

tacctagatt tcaactaaat caaaacccct tacagttcaa tctagagctg atcattttgg     720

ctcggcccgt cccatttttg ggccgggttt tagtcagatt tttttggccc gcggtcgggc     780

ccggcccgat ttttttggct ttgggcaagc caaaaacgac ttttcagttt attttttggc     840

ccgacccgtt tttacccgca aaagcccgct aatttaggtc cgcactttgg gcacaaaaat     900

ttagcccgaa cttaaacctg gcccgaccca tgatcacctc tagtttaatc caaactaaaa     960

aactacacaa gttagccaaa aattatgtct actttgtaca actttataaa atacacacag    1020

tagttgatat cttgatgatt aactcctttt gaagtttgac tacacaccaa ccccaaacac 1080

acccactttt tcccccctct tgtcaccaac cccccctcct ctttagccac caaagtttgg 1140

ttggtgagtc ctccataact gctaaattct ctcttttttc tctctcctaa aaaactaaaa 1200

cccaccaaaa tttcagacat caaaaaaatt acaagtgaag gaaacaataa tggctcaagc 1260

tagcaccata aacaatggtg tcaaaagcac ccaattatgc cccaatttac ccaaaaccca 1320

cttatccaaa tcttcaaaat ctgttaaatt tggatcaaat ttgagatttt ctccaaagtt 1380

gaaatctttt aacaatgaaa gagttggtgg gaattcatca gttgttttca gggttagggc 1440

ttcagttgca gcagcagctg aaaaatcatc aactgtacca gaaattgtgt tacaacccat 1500

caaagagatc tcgggtacca ttcaattgcc cggatccaag tcattatcta atcggattct 1560

acttcttgct gccctttctc aggtactttt caattgtttg atttcttttt ttcttagaac 1620

ttgtgaattt gtatacttta tccgtttcta aatacgtgca acatttgaat agtaacgagt 1680

atttatctac caacttattt aatattctct cacgaatgta tatgaaaaaa tatagtcatg 1740

cgtggtttta tttgattgat ctgcggactt ttataatatc aactttttat aatttagagg 1800

acaaagtagt gtattgggta gcgtgtaagg aggttgggaa actggaggaa ttttttaaca 1860

attcaagttt gatatttttc atagtgaaat gtttattagc atagaatcat gcttttagtt 1920

tttagtggag tgtgcattta ttctttaact tgttggatgg ctatgattaa gaattggatt 1980

ctggttattt gcttgagtat ttagaaatta attgtgggtg ttggtgataa tgtaacaaaa 2040

ttgttttgaa ggtgtgagaa tgtgatttta attatatgag gaaatgatgg gttatttatt 2100

gtaatgtggg aatttatgat aatatgttgg aggatgaaac aattgatgat tttgaagtgg 2160

ggtatgaagg ctttccccca tttttttacc tttcatgtgt tgtatgtagc agactgccaa 2220

agcaattctt ctgagcagct tggcatattt ccattgcaac tcataatctc aatccaagca 2280

caatcaatta cctgccacgc tagcacttta agtagcaaaa tcgcgcttga agaaaagata 2340

ataccacagg cttctatagt tctattcctt gtgatatagg acacaagtat catcctagat 2400

gacttcccta gccctgcaaa taattcctgg aaggaagtcc gttgagtatc cttgatcaag 2460

taaaattagc gctttctttt gggtaacaca aactatactc gaaccacttt atgctaaggg 2520

atcacattgc gtctcttgca gaacttttaa catttctaac atggcataag cacggaaaca 2580

ccctgtgaag ttgctagtga ccatcttttt tgataaatca agaatccctg ttcttatttg 2640

catacaaagg gttaagtctt gtctgagagg gattgacata tctcctgctg gatagacttt 2700

gtgttcatct ctaatgttat tctgagtttg cattgtatgt gttgtgtttt cgtttttctt 2760

cttgtttgtg tgtgtgtgtg tgtgtgtgtg tgtgtgttac ccaaacttgt acggaaattt 2820

ccatcctaac gagaattatg ctgcagggaa caacagtggt tgacaacttg ctatatagtg 2880

atgatatccg ttacatgctg gatgctttga gaactcttgg gttgaatgtg gaggatgata 2940

atatagccaa aagggcaatc gtggagggtt gcggtggttt gtttcctgtt ggaaaagatg 3000

ggaaagaaat tgaacttttt cttggaaatg caggaatagc aatgcgctca ttgacagctg 3060

cagttgctgt tgctggagga aattctaggt ttgtctacta aggccttcca tgtcatcgtg 3120

aggctgttaa tattctttta ttttgtgtgg agacttgttt cctcttgcta caattgcaaa 3180

gggggctggg attgattaac aatgcagaac tcaaacacta tctttggtat caccgtctgg 3240

caggtggcat ctgatcttgc aaaacagata tggagggttt tgccggaaag gggttggggg 3300

tgggacttgg gagtatagcc tcaatgttat agaagattgt gctatactac tgtaaaatta 3360

acttttggtt ggaaccagat atagttaata aagtattttt ttatctggtg aggggtgctt 3420

ctaggtcctg aaaagttcca ccatagaaaa aaattgaatt tcattgtgaa aagctaaggt 3480

gaattcatta aattcaatac atgttgttga ttgcttgaac tagaatactt cagatatcag 3540

ttaattgaat ttatttcttt tggagaacat gcatgaagag cttttaactc gcctctacta 3600

tcctgctctt aattattttt ataaatagat ttagcaattg ggaatatcct gtaatttgct 3660

ggttgctttt tcatatggta tatattgaat tggtgtggag gcgttttaca aagagtgaag 3720

gtaattgttg atgggtggat attgaatgga aatactttga tggcggatag catattcatc 3780

tgattggatg tctgaaattt gatgagctct agtaaatgtt cctccatgta tgcttgtgta 3840

ttgggaattt gcaatccagt agtagagaac acccctgata attgcactat tattcctgag 3900

ttgtgagagc tctcaagaag atagatgggg ttcttcaagt tttttaattg tctttaggta 3960

gtgtaaaggt tgcaatattt caaaggatct gtctgatcca acaagtctgc taaatccttc 4020

accaatctga aaggggagaa gatactgctc tctctgacct gcttgcatgc atataaagct 4080

tctaaaacaa ataggttaaa tgtaaaacaa gcgcagaagt tttcacttct cttgggaaat 4140

gtgtgccaag atctattgca tgacatgaat tcatatttat tgattatatg ctcaacatga 4200

atgggattaa gaatgaaata aagtagcatg agtaggagac aatccgcctg ctcttttaat 4260

aggcatatac aatcagtcat caacatcatt gctgctttag aatttgattt atgttgatct 4320

tggtttttgc agctatgtac ttgatggagt gccgagaatg agggagcgac ccattgggga 4380

tttggtagcg ggtctaaagc aacttggcgc cgatgttgac tgttatcttg gcacaaattg 4440

tcctcctgtt cgagtgaatg ctaaaggagg ccttcccggg ggcaaggtga attctcttgt 4500

tttgattaga gaaaaattat aagttagtgt tctatcatgc ttgacacgaa caagtttttt 4560

tatctgaaag agaaagaagt gtatgcctaa tgattaacag tcatattcaa aatgatataa 4620

aagagggtgg aaggttactt gctctatgat gatttatggt agtaccgtgt taccaatagc 4680

agaattcaag caatattctt tgaactggta ataacaagtt caagcaatca agcatagttt 4740

agattagatt ctatcaatct cttagcatag tttaaattcg cttccatcaa tctcttttgt 4800

gacgtaagaa gttctgttcc attatactta tgctttttgt gaaattttat ttgcaggtca 4860
agctctctgg atcagttagt agccaatatt tgactgcact gcttatggct actcctttgg 4920
gtcttggaga cgtggaggtt gaaatcattg ataaattgat ttctgtacca tatgtggaga 4980
tgacaataaa gctaatggaa aggtttggag tgtctgtaga gcatagtgct gactggggta 5040
ggttcttgat ccgaggtggt cagaagtaca agtaagtctc tcttttttac tacgtgtcct 5100
gaaaaatcta tgttttagta gactaagata ctataaaata catcagatct cctggaaatg 5160
cgtatgttga gggtgatgct tcaagtgcta gttacttcat aggaggggcc gcagtcactg 5220
gtgggactgt gactgttgag ggttgtggaa caagtagttt acaggtatga tttaaagcct 5280
tatttcacat ccttttactt ctctccttac tgcatctcag cttaattctg aagaactctt 5340
gttgctcaat ctctaggcat aagcttgttc tttcgacctt ctaaatttgc aaaatcattc 5400
tgttaatgta acaaacagaa gatgagatgg tgtttttgat cctgataaaa aaaattagga 5460
cattcatgat tggtttatgt tctatgacaa tttcgatgat ctcttcaatt gtaacttgag 5520
gacccttttc tgctagtaaa gattgtgtat aagctattct agactgttgt atacacctca 5580
tattccggta ttgtttatac ctaataaact tcttatgatt agtatcttac ttgtctcatg 5640
tgttttgcat agggtgatgt aaaatttgct gaagttcttg agaagatggg ttgcaaggta 5700
tcctggacag agaacagtgt cactgtcact ggaccaccca gggatgcatc tggaagaaaa 5760
cacttgcgcg ccgttgatgt caacatgaac aaaatgccag atgttgcaat gactcttgct 5820
gttgttgctc tttatgcaga tggacccacc accattagag acggtatgct taattccttt 5880
ctgtgaacat gacactcttc ttgtgccata ggtagattcc gtagttattg gtcacaatcc 5940
aaatttgcat tggtatttaa agcaagtctt aggtggtgag accgcttgaa acccataata 6000
taaaagaaag gtcaccttgg tctcaaagtt aattcttgtg agaccatctc atgcaagatt 6060
ttgccgggta tctaatcctt gtttgcactg aggaaaagga aaaaaattaa catgacctat 6120
tagtaagcca aaaaaattct aacaagagaa gtagccgaat aggtaataat ttttgtgata 6180
taatgatcag tggctagctg gagagtgaag gaaacagaac ggatgattgc gatttgcaca 6240
gagctcagaa aggttagcca ttttggatat acacattctt gaaggttttc aaccttgtag 6300
aagattgtac tattgataaa aaaaattgat gtttatccgg tgtacttcga aatttctttt 6360
cagctggggg caacagttga ggaaggatca gattactgtg tgatcactcc acctgagaaa 6420
ctaaatgtga cggccattga tacatacgat gatcaccgaa tggccatggc attctctctt 6480
gctgcctgcg ccgatgttcc tgttaccatc aaggacccgg gttgcactcg caagactttc 6540
ccagactact ttgatgtgtt ggaaaggttt gcaaagcatt aagtggtctc ctacatattc 6600
tataaagcat aagctgagat tttttgagag aattaggaga tgaaaaatgc tttctgcttg 6660
agttatcatc acattctttg tattatgatt gtaagattat tatagtatag agtttacaaa 6720

gtactactaa taattgttat gtatccgatt gatcagaaat aagttaattg gaaggctgga 6780

ctttgaaaat gtgaccaaga cactagtgtg accaagtcat tttgttaatg tgagttcaat 6840

gttattgatt caacatgtag agccaaatct caattctatc gtcacttcat atgaccaaaa 6900

atctaaagat gaaaaagtaa aaaagagcat gttggatcaa actctagctg tatctctgaa 6960

attcaatcac gcagttagat caaatgagga ttaaagggag t 7001

<210> 5
<211> 1554
<212> DNA
<213> Artificial Sequence

<220>
<223> cDNA of mutated epsps gene (T175I and P179S)

<400> 5
atggctcaag ctagcaccat aaacaatggt gtcaaaagca cccaattatg ccccaattta 60

cccaaaaccc acttatccaa atcttcaaaa tctgttaaat ttggatcaaa tttgagattt 120

tctccaaagt tgaaatcttt taacaatgaa agagttggtg ggaattcatc agttgttttc 180

agggttaggg cttcagttgc agcagcagct gaaaaatcat caactgtacc agaaattgtg 240

ttacaaccca tcaaagagat ctcgggtacc attcaattgc ccggatccaa gtcattatct 300

aatcggattc tacttcttgc tgccctttct cagggaacaa cagtggttga caacttgcta 360

tatagtgatg atatccgtta catgctggat gctttgagaa ctcttgggtt gaatgtggag 420

gatgataata tagccaaaag ggcaatcgtg gagggttgcg gtggtttgtt tcctgttgga 480

aaagatggga aagaaattga actttttctt ggaaatgcag gaatagcaat gcgctcattg 540

acagctgcag ttgctgttgc tggaggaaat tctagctatg tacttgatgg agtgccgaga 600

atgagggagc gacccattgg ggatttggta gcgggtctaa agcaacttgg cgccgatgtt 660

gactgttatc ttggcacaaa ttgtcctcct gttcgagtga atgctaaagg aggccttccc 720

gggggcaagg tcaagctctc tggatcagtt agtagccaat atttgactgc actgcttatg 780

gctactcctt tgggtcttgg agacgtggag gttgaaatca ttgataaatt gatttctgta 840

ccatatgtgg agatgacaat aaagctaatg gaaaggtttg gagtgtctgt agagcatagt 900

gctgactggg gtaggttctt gatccgaggt ggtcagaagt acaaatctcc tggaaatgcg 960

tatgttgagg gtgatgcttc aagtgctagt tacttcatag gaggggccgc agtcactggt 1020

gggactgtga ctgttgaggg ttgtggaaca agtagtttac agggtgatgt aaaatttgct 1080

gaagttcttg agaagatggg ttgcaaggta tcctggacag agaacagtgt cactgtcact 1140

ggaccaccca gggatgcatc tggaagaaaa cacttgcgcg ccgttgatgt caacatgaac 1200

aaaatgccag atgttgcaat gactcttgct gttgttgctc tttatgcaga tggacccacc 1260

accattagag acgtggctag ctggagagtg aaggaaacag aacggatgat tgcgatttgc 1320

acagagctca gaaagctggg ggcaacagtt gaggaaggat cagattactg tgtgatcact 1380

ccacctgaga aactaaatgt gacggccatt gatacatacg atgatcaccg aatggccatg 1440

gcattctctc ttgctgcctg cgccgatgtt cctgttacca tcaaggaccc gggttgcact 1500

cgcaagactt tcccagacta ctttgatgtg ttggaaaggt ttgcaaagca ttaa 1554

<210> 6
<211> 517
<212> PRT
<213> Artificial Sequence

<220>
<223> protein of mutated epsps gene (T175I and P179S)

<400> 6

Met Ala Gln Ala Ser Thr Ile Asn Asn Gly Val Lys Ser Thr Gln Leu
1               5                   10                  15

Cys Pro Asn Leu Pro Lys Thr His Leu Ser Lys Ser Ser Lys Ser Val
            20                  25                  30

Lys Phe Gly Ser Asn Leu Arg Phe Ser Pro Lys Leu Lys Ser Phe Asn
        35                  40                  45

Asn Glu Arg Val Gly Gly Asn Ser Ser Val Val Phe Arg Val Arg Ala
    50                  55                  60

Ser Val Ala Ala Ala Ala Glu Lys Ser Ser Thr Val Pro Glu Ile Val
65                  70                  75                  80

Leu Gln Pro Ile Lys Glu Ile Ser Gly Thr Ile Gln Leu Pro Gly Ser
                85                  90                  95

Lys Ser Leu Ser Asn Arg Ile Leu Leu Leu Ala Ala Leu Ser Gln Gly
            100                 105                 110

Thr Thr Val Val Asp Asn Leu Leu Tyr Ser Asp Asp Ile Arg Tyr Met
        115                 120                 125

Leu Asp Ala Leu Arg Thr Leu Gly Leu Asn Val Glu Asp Asp Asn Ile
    130                 135                 140

Ala Lys Arg Ala Ile Val Glu Gly Cys Gly Gly Leu Phe Pro Val Gly
145                 150                 155                 160

Lys Asp Gly Lys Glu Ile Glu Leu Phe Leu Gly Asn Ala Gly Ile Ala
                165                 170                 175

Met Arg Ser Leu Thr Ala Ala Val Ala Val Ala Gly Gly Asn Ser Ser
            180                 185                 190

Tyr Val Leu Asp Gly Val Pro Arg Met Arg Glu Arg Pro Ile Gly Asp
        195                 200                 205

Leu Val Ala Gly Leu Lys Gln Leu Gly Ala Asp Val Asp Cys Tyr Leu
    210                 215                 220

Gly Thr Asn Cys Pro Pro Val Arg Val Asn Ala Lys Gly Gly Leu Pro
225                 230                 235                 240

Gly Gly Lys Val Lys Leu Ser Gly Ser Val Ser Ser Gln Tyr Leu Thr
                245                 250                 255

Ala Leu Leu Met Ala Thr Pro Leu Gly Leu Gly Asp Val Glu Val Glu
            260                 265                 270

Ile Ile Asp Lys Leu Ile Ser Val Pro Tyr Val Glu Met Thr Ile Lys
        275                 280                 285

Leu Met Glu Arg Phe Gly Val Ser Val Glu His Ser Ala Asp Trp Gly
    290                 295                 300

Arg Phe Leu Ile Arg Gly Gly Gln Lys Tyr Lys Ser Pro Gly Asn Ala
305                 310                 315                 320

Tyr Val Glu Gly Asp Ala Ser Ser Ala Ser Tyr Phe Ile Gly Gly Ala
                325                 330                 335

Ala Val Thr Gly Gly Thr Val Thr Val Glu Gly Cys Gly Thr Ser Ser
            340                 345                 350

Leu Gln Gly Asp Val Lys Phe Ala Glu Val Leu Glu Lys Met Gly Cys
        355                 360                 365

Lys Val Ser Trp Thr Glu Asn Ser Val Thr Val Thr Gly Pro Pro Arg
    370                 375                 380

Asp Ala Ser Gly Arg Lys His Leu Arg Ala Val Asp Val Asn Met Asn
385                 390                 395                 400

Lys Met Pro Asp Val Ala Met Thr Leu Ala Val Val Ala Leu Tyr Ala
                405                 410                 415

Asp Gly Pro Thr Thr Ile Arg Asp Val Ala Ser Trp Arg Val Lys Glu

420                 425                 430

Thr Glu Arg Met Ile Ala Ile Cys Thr Glu Leu Arg Lys Leu Gly Ala
        435                 440                 445

Thr Val Glu Glu Gly Ser Asp Tyr Cys Val Ile Thr Pro Pro Glu Lys
    450                 455                 460

Leu Asn Val Thr Ala Ile Asp Thr Tyr Asp Asp His Arg Met Ala Met
465                 470                 475                 480

Ala Phe Ser Leu Ala Ala Cys Ala Asp Val Pro Val Thr Ile Lys Asp
                485                 490                 495

Pro Gly Cys Thr Arg Lys Thr Phe Pro Asp Tyr Phe Asp Val Leu Glu
            500                 505                 510

Arg Phe Ala Lys His
        515

<210>  7
<211>  4501
<212>  DNA
<213>  Artificial Sequence

<220>
<223>  genomic DNA of mutated als gene (W569L)

<400>  7
cgtggtaagg tttttcttcc tattgggcct aggaagtttt ccagccttgt aaaaatcttg      60

tgtgcttttt ctctttcgtt ttttagttat tttcattccg caatctaaat tcgaaaattt     120

tccctcaact acaattcaac cccttcttgt atttggtcta gtgttcatac tagaataaca     180

caaaatcgtg attaaatcta tgtattggat tgatagagaa acataaactc tttgaagaaa     240

cctaattatg ttaggacttc taatagtttc cgtcacgttt tcatttgtat tagaatttta     300

gaggtttaac tactataaag aattcatgtt ataatggaac ttgagtaata caagtcctgt     360

aagatgagaa gactaatcat gttagaagta taatcatgat agaagtctaa ttttattaat     420

agtcttttaa tgttagaatt ctaaagtttt taggagctta attaaggtag ttttcctacc     480

tatataagat tcctagtttc ctattacttg taataggatt cttagtttta taattatgaa     540

ataatttcta atcctaattg ttttcttata aataggctac ggctggtcac ctacaccaat     600

aaaattcaaa agtttactga ttaaatttga tagtttttct tcttatatga tctataagac     660

tagcttaatt agaatattaa gatttccccc ttattccacc ttcaaattag tgtacataac     720

tttctccatt aaaaatttca agaaaccttt tcctaattaa accatatatt ctaaaactcc     780

attaatagga acccctcgtt cctcatcaat tttttttttt taaaaggctt ttttttcttc     840

aacccatcat atccacattt acaagagcag ggtattttgg taagtttcca tatatagaaa 900

gtggaatcga gcgcctccac tcatttcctc ctcaaaagaa caagaacaag aacgagaaca 960

agaacaacca tcctcattct ctctccaaaa ctccaaacaa caacaatggc ggctaccttc 1020

acaaacccaa cattttcccc ttcctcaact ccattaacca aaaccctaaa atcccaatct 1080

tccatctctt caaccctccc cttttccacc cctcccaaaa ccccaactcc actctttcac 1140

cgtcccctcc aaatctcatc ctcccaatcc cacaaatcat ccgccattaa aacacaaact 1200

caagcacctt cttctccagc tattgaagat tcatctttcg tttctcgatt tggccctgat 1260

gaacccagaa aagggtccga tgtcctcgtt gaagctcttg agcgtgaagg tgttaccaat 1320

gtgtttgctt accctggtgg tgcatctatg gaaatccacc aagctctcac acgctctaaa 1380

accatccgca atgtcctccc tcgccatgaa caaggcgggg ttttcgccgc cgagggatat 1440

gctagagcta ctggaaaggt tggtgtctgc attgcgactt ctggtcctgg tgctaccaac 1500

ctcgtatcag gtcttgctga cgctctcctt gattctgtcc ctcttgttgc catcactggc 1560

caagttccac gccgtatgat tggcactgat gcttttcagg agactccaat tgttgaggtg 1620

acaaggtcta ttactaagca taattattta gttttggatg tagaggatat tcctagaatt 1680

gttaaggaag cctttttttt agctaattct ggtaggcctg gacctgtttt gattgatctt 1740

cctaaagata ttcagcagca attggttgtt cctgattggg ataggccttt taagttgggt 1800

gggtatatgt ctaggctgcc aaagtccaag ttttcgacga atgaggttgg acttcttgag 1860

cagattgtga ggttgatgag tgagtcgaag aagcctgtct tgtatgtggg aggtgggtgt 1920

ttgaattcta gtgaggagtt gaggagattt gttgagttga cagggattcc ggtggctagt 1980

actttgatgg ggttggggtc ttacccttgt aatgatgaac tgtctcttca tatgttgggg 2040

atgcacggga ctgtttatgc caattatgcg gtggataagg cggatttgtt gcttgctttc 2100

ggggttaggt ttgatgatcg tgtgaccggg aagctcgagg cgtttgctag ccgtgctaag 2160

attgtgcata ttgatattga ctctgctgag attgggaaga acaagcagcc ccatgtgtcc 2220

atttgtgctg atgttaaatt ggcattgcgg ggtatgaata agattctgga gtctagaata 2280

gggaagctga atttggattt ctccaagtgg agagaagaat taggtgagca gaagaaggaa 2340

ttcccactga gttttaagac atttggggat gcaattcctc cacaatatgc cattcaggtg 2400

cttgatgagt tgaccaatgg taatgctatt ataagtactg gtgttgggca gcaccaaatg 2460

tgggctgcgc agcattacaa gtacagaaac cctcgccaat ggctgacctc tggtgggttg 2520

ggggctatgg ggtttgggct accagccgcc attggagctg cagttgctcg accagatgca 2580

gtggttgtcg atattgatgg ggatggcagt tttattatga atgttcaaga gttggctaca 2640

attagggtgg aaaatctccc agttaagata atgctgctaa acaatcaaca tttaggtatg 2700

gttgtccaat tggaagatag gttctataaa gctaaccggg cacatacata ccttggaaac 2760

ccttccaaat ctgctgatat cttccctgat atgctcaaat tcgctgaggc atgtgatatt 2820

ccttctgccc gtgttagcaa cgtggctgat ttgagggccg ccattcaaac aatgttggat 2880

actccagggc cgtacctgct cgatgtgatt gtaccgcatc aagagcatgt gttgcctatg 2940

attccaagtg gtgccggttt caaggatacc attacagagg gtgatggaag aacctcttat 3000

tgatcggttt aatgacggtt ggaaccattt aaagagggta agctatatta ctgtatgtat 3060

attagtatgt tcctggataa tttagaagct tttgtctgtt gtcttttgca gtttatgaag 3120

ttagtttgct gttgtcatgt tacttgttac tttaaaaagc tttttgtagt ttttgagcaa 3180

ctagtatgga atgctcttcc tgtattgctt ggaaaattca caaaagtggt ttttcggcta 3240

tggatgttgt gttgcatcat gcatatatag cttgatatac tagttggctt ggtgcatctt 3300

taacatatac taatgagact acgacagcaa ttgccaatta gttggcttga cataattctt 3360

agtctgccac tagaaatctt gcttcttttt tttccctcat ttgttgaaag tccctgttgc 3420

gacctgacat gggagcattg gagattgttt agcaatagca gttgcagtta ggtgactaca 3480

gtcatcttcc aaatatgaat actctctgga ggggaggagg ttttacaaaa tatgagtttt 3540

taacacatga gaaacttata ttaaacaagg ttgagtcacc ccatattttt caaagttgac 3600

ttttgtctga tttgggtgac tatgcctgtt gtatgcaata aactcgatgt acataagact 3660

tgtataatcc aatctaaccc ttcctggctg attatgaaac cgagtcggct aatttgttgc 3720

ttgatcttca tgtgtgagcc tgatgccagg tgaccactag aggagtacct ttcattgaga 3780

tatcgattcg gttaattggt tcctcatatg ggtctcaaaa ctgaattttt ctcaggctct 3840

cttctaacca gttgttgaat tttatgaaac ttcagtccag ttaaaacttt gatcccggat 3900

cagaatttct ctgagttgtt cttccgcctc tcagttccac tgttccagtg ttcttggtcc 3960

tcaacctgta ctctgagcta tttctgcaaa cacctgaagt tcctgctgcc cttggacaaa 4020

tacagaatgc agcattagca tttatttaag acaagatgaa tacttgctcc cctgttagtt 4080

atgctaattc tgcattagta ttctttaatt ttaattagat actgcaatcc gtgaactctg 4140

cagtttcttg atctctctct gtttcaattt ctcctatctt atcgcccatt tctttaggct 4200

ttcgttatct atcgtctaat tcaagagtag gtaactaggt acgggataaa actttttcat 4260

gaagaaccta tgttcccgta ttatgctgtc ccacaagctt caactttcta ccttgttttc 4320

tatacgttgg acaacttctt ttgtgttgaa ctgatttaat tgataaatga ataattttgg 4380

ataaagaaaa ttgattgacc aataatttat ttattttagt ttacgttttg tatataccga 4440

ctgggctcat gtgagcacat ttatgtgcac aatttttttt atgtgaaaac aaaactaagc 4500

t 4501

<210> 8
<211> 1998
<212> DNA
<213> Artificial Sequence

<220>
<223> cDNA of mutated als gene (W569L)

<400> 8
atggcggcta ccttcacaaa cccaacattt tccccttcct caactccatt aaccaaaacc 60

ctaaaatccc aatcttccat ctcttcaacc ctcccctttt ccacccctcc caaaacccca 120

actccactct ttcaccgtcc cctccaaatc tcatcctccc aatcccacaa atcatccgcc 180

attaaaacac aaactcaagc accttcttct ccagctattg aagattcatc tttcgtttct 240

cgatttggcc ctgatgaacc cagaaaaggg tccgatgtcc tcgttgaagc tcttgagcgt 300

gaaggtgtta ccaatgtgtt tgcttaccct ggtggtgcat ctatggaaat ccaccaagct 360

ctcacacgct ctaaaaccat ccgcaatgtc ctccctcgcc atgaacaagg cggggttttc 420

gccgccgagg gatatgctag agctactgga aaggttggtg tctgcattgc gacttctggt 480

cctggtgcta ccaacctcgt atcaggtctt gctgacgctc tccttgattc tgtccctctt 540

gttgccatca ctggccaagt tccacgccgt atgattggca ctgatgcttt tcaggagact 600

ccaattgttg aggtgacaag gtctattact aagcataatt atttagtttt ggatgtagag 660

gatattccta gaattgttaa ggaagccttt tttttagcta attctggtag gcctggacct 720

gttttgattg atcttcctaa agatattcag cagcaattgg ttgttcctga ttgggatagg 780

ccttttaagt tgggtgggta tatgtctagg ctgccaaagt ccaagttttc gacgaatgag 840

gttggacttc ttgagcagat tgtgaggttg atgagtgagt cgaagaagcc tgtcttgtat 900

gtgggaggtg ggtgtttgaa ttctagtgag gagttgagga gatttgttga gttgacaggg 960

attccggtgg ctagtacttt gatggggttg ggtcttaccc cttgtaatga tgaactgtct 1020

cttcatatgt tggggatgca cgggactgtt tatgccaatt atgcggtgga taaggcggat 1080

ttgttgcttg ctttcggggt taggtttgat gatcgtgtga ccgggaagct cgaggcgttt 1140

gctagccgtg ctaagattgt gcatattgat attgactctg ctgagattgg gaagaacaag 1200

cagccccatg tgtccatttg tgctgatgtt aaattggcat tgcggggtat gaataagatt 1260

ctggagtcta gaatagggaa gctgaatttg gatttctcca agtggagaga agaattaggt 1320

gagcagaaga aggaattccc actgagtttt aagacatttg gggatgcaat tcctccacaa 1380

tatgccattc aggtgcttga tgagttgacc aatggtaatg ctattataag tactggtgtt 1440

gggcagcacc aaatgtgggc tgcgcagcat tacaagtaca gaaaccctcg ccaatggctg 1500

acctctggtg ggttgggggc tatggggttt gggctaccag ccgccattgg agctgcagtt 1560

gctcgaccag atgcagtggt tgtcgatatt gatggggatg gcagttttat tatgaatgtt 1620

caagagttgg ctacaattag ggtggaaaat ctcccagtta agataatgct gctaaacaat 1680

caacatttag gtatggttgt ccaattggaa gataggttct ataaagctaa ccgggcacat 1740

acataccttg gaaacccttc caaatctgct gatatcttcc ctgatatgct caaattcgct 1800

gaggcatgtg atattccttc tgcccgtgtt agcaacgtgg ctgatttgag ggccgccatt 1860

caaacaatgt tggatactcc agggccgtac ctgctcgatg tgattgtacc gcatcaagag 1920

catgtgttgc ctatgattcc aagtggtgcc ggtttcaagg ataccattac agagggtgat 1980

ggaagaacct cttattga 1998

<210> 9
<211> 665
<212> PRT
<213> Artificial Sequence

<220>
<223> protein of mutated als gene (W569L)

<400> 9

Met Ala Ala Thr Phe Thr Asn Pro Thr Phe Ser Pro Ser Ser Thr Pro
1               5                   10                  15

Leu Thr Lys Thr Leu Lys Ser Gln Ser Ser Ile Ser Ser Thr Leu Pro
            20                  25                  30

Phe Ser Thr Pro Pro Lys Thr Pro Thr Pro Leu Phe His Arg Pro Leu
        35                  40                  45

Gln Ile Ser Ser Ser Gln Ser His Lys Ser Ser Ala Ile Lys Thr Gln
    50                  55                  60

Thr Gln Ala Pro Ser Ser Pro Ala Ile Glu Asp Ser Ser Phe Val Ser
65                  70                  75                  80

Arg Phe Gly Pro Asp Glu Pro Arg Lys Gly Ser Asp Val Leu Val Glu
                85                  90                  95

Ala Leu Glu Arg Glu Gly Val Thr Asn Val Phe Ala Tyr Pro Gly Gly
            100                 105                 110

Ala Ser Met Glu Ile His Gln Ala Leu Thr Arg Ser Lys Thr Ile Arg
        115                 120                 125

Asn Val Leu Pro Arg His Glu Gln Gly Gly Val Phe Ala Ala Glu Gly
    130                 135                 140

Tyr Ala Arg Ala Thr Gly Lys Val Gly Val Cys Ile Ala Thr Ser Gly
145                 150                 155                 160

Pro Gly Ala Thr Asn Leu Val Ser Gly Leu Ala Asp Ala Leu Leu Asp
                165                 170                 175

Ser Val Pro Leu Val Ala Ile Thr Gly Gln Val Pro Arg Arg Met Ile
            180                 185                 190

Gly Thr Asp Ala Phe Gln Glu Thr Pro Ile Val Glu Val Thr Arg Ser
        195                 200                 205

Ile Thr Lys His Asn Tyr Leu Val Leu Asp Val Glu Asp Ile Pro Arg
    210                 215                 220

Ile Val Lys Glu Ala Phe Phe Leu Ala Asn Ser Gly Arg Pro Gly Pro
225                 230                 235                 240

Val Leu Ile Asp Leu Pro Lys Asp Ile Gln Gln Gln Leu Val Val Pro
                245                 250                 255

Asp Trp Asp Arg Pro Phe Lys Leu Gly Gly Tyr Met Ser Arg Leu Pro
            260                 265                 270

Lys Ser Lys Phe Ser Thr Asn Glu Val Gly Leu Leu Glu Gln Ile Val
        275                 280                 285

Arg Leu Met Ser Glu Ser Lys Lys Pro Val Leu Tyr Val Gly Gly Gly
    290                 295                 300

Cys Leu Asn Ser Ser Glu Glu Leu Arg Arg Phe Val Glu Leu Thr Gly
305                 310                 315                 320

Ile Pro Val Ala Ser Thr Leu Met Gly Leu Gly Ser Tyr Pro Cys Asn
                325                 330                 335

Asp Glu Leu Ser Leu His Met Leu Gly Met His Gly Thr Val Tyr Ala
            340                 345                 350

Asn Tyr Ala Val Asp Lys Ala Asp Leu Leu Leu Ala Phe Gly Val Arg
        355                 360                 365

Phe Asp Asp Arg Val Thr Gly Lys Leu Glu Ala Phe Ala Ser Arg Ala
    370                 375                 380

Lys Ile Val His Ile Asp Ile Asp Ser Ala Glu Ile Gly Lys Asn Lys
385                 390                 395                 400

Gln Pro His Val Ser Ile Cys Ala Asp Val Lys Leu Ala Leu Arg Gly

                405                 410                 415

Met Asn Lys Ile Leu Glu Ser Arg Ile Gly Lys Leu Asn Leu Asp Phe
            420                 425                 430

Ser Lys Trp Arg Glu Glu Leu Gly Glu Gln Lys Lys Glu Phe Pro Leu
        435                 440                 445

Ser Phe Lys Thr Phe Gly Asp Ala Ile Pro Pro Gln Tyr Ala Ile Gln
    450                 455                 460

Val Leu Asp Glu Leu Thr Asn Gly Asn Ala Ile Ile Ser Thr Gly Val
465                 470                 475                 480

Gly Gln His Gln Met Trp Ala Ala Gln His Tyr Lys Tyr Arg Asn Pro
                485                 490                 495

Arg Gln Trp Leu Thr Ser Gly Gly Leu Gly Ala Met Gly Phe Gly Leu
            500                 505                 510

Pro Ala Ala Ile Gly Ala Ala Val Ala Arg Pro Asp Ala Val Val Val
        515                 520                 525

Asp Ile Asp Gly Asp Gly Ser Phe Ile Met Asn Val Gln Glu Leu Ala
    530                 535                 540

Thr Ile Arg Val Glu Asn Leu Pro Val Lys Ile Met Leu Leu Asn Asn
545                 550                 555                 560

Gln His Leu Gly Met Val Val Gln Leu Glu Asp Arg Phe Tyr Lys Ala
                565                 570                 575

Asn Arg Ala His Thr Tyr Leu Gly Asn Pro Ser Lys Ser Ala Asp Ile
            580                 585                 590

Phe Pro Asp Met Leu Lys Phe Ala Glu Ala Cys Asp Ile Pro Ser Ala
        595                 600                 605

Arg Val Ser Asn Val Ala Asp Leu Arg Ala Ala Ile Gln Thr Met Leu
    610                 615                 620

Asp Thr Pro Gly Pro Tyr Leu Leu Asp Val Ile Val Pro His Gln Glu
625                 630                 635                 640

His Val Leu Pro Met Ile Pro Ser Gly Ala Gly Phe Lys Asp Thr Ile
                645                 650                 655

Thr Glu Gly Asp Gly Arg Thr Ser Tyr
            660                 665

<210>  10
<211>  4501
<212>  DNA
<213>  Artificial Sequence

<220>
<223>  genomic DNA of mutated als gene (P188S and W569L)

<400>  10
cgtggtaagg tttttcttcc tattgggcct aggaagtttt ccagccttgt aaaaatcttg        60

tgtgcttttt ctctttcgtt ttttagttat tttcattccg caatctaaat tcgaaaattt       120

tccctcaact acaattcaac cccttcttgt atttggtcta gtgttcatac tagaataaca       180

caaaatcgtg attaaatcta tgtattggat tgatagagaa acataaactc tttgaagaaa       240

cctaattatg ttaggacttc taatagtttc cgtcacgttt tcatttgtat tagaatttta       300

gaggtttaac tactataaag aattcatgtt ataatggaac ttgagtaata caagtcctgt       360

aagatgagaa gactaatcat gttagaagta taatcatgat agaagtctaa ttttattaat       420

agtcttttaa tgttagaatt ctaaagtttt taggagctta attaaggtag ttttcctacc       480

tatataagat tcctagtttc ctattacttg taataggatt cttagtttta taattatgaa       540

ataatttcta atcctaattg ttttcttata aataggctac ggctggtcac ctacaccaat       600

aaaattcaaa agtttactga ttaaatttga tagtttttct tcttatatga tctataagac       660

tagcttaatt agaatattaa gatttccccc ttattccacc ttcaaattag tgtacataac       720

tttctccatt aaaaatttca agaaaccttt tcctaattaa accatatatt ctaaaactcc       780

attaatagga acccctcgtt cctcatcaat tttttttttt taaaaggctt ttttttcttc       840

aacccatcat atccacattt acaagagcag ggtattttgg taagtttcca tatatagaaa       900

gtggaatcga gcgcctccac tcatttcctc ctcaaaagaa caagaacaag aacgagaaca       960

agaacaacca tcctcattct ctctccaaaa ctccaaacaa caacaatggc ggctaccttc      1020

acaaacccaa catttccccc ttcctcaact ccattaacca aaaccctaaa atcccaatct      1080

tccatctctt caaccctccc cttttccacc cctcccaaaa ccccaactcc actctttcac      1140

cgtcccctcc aaatctcatc ctcccaatcc cacaaatcat ccgccattaa aacacaaact      1200

caagcacctt cttctccagc tattgaagat tcatctttcg tttctcgatt tggccctgat      1260

gaacccagaa aagggtccga tgtcctcgtt gaagctcttg agcgtgaagg tgttaccaat      1320

gtgtttgctt accctggtgg tgcatctatg gaaatccacc aagctctcac acgctctaaa      1380

accatccgca atgtcctccc tcgccatgaa caaggcgggg ttttcgccgc cgagggatat      1440

gctagagcta ctggaaaggt tggtgtctgc attgcgactt ctggtcctgg tgctaccaac      1500

ctcgtatcag gtcttgctga cgctctcctt gattctgtcc ctcttgttgc catcactggc 1560

caagtttcac gccgtatgat tggcactgat gcttttcagg agactccaat tgttgaggtg 1620

acaaggtcta ttactaagca taattattta gttttggatg tagaggatat tcctagaatt 1680

gttaaggaag cctttttttt agctaattct ggtaggcctg gacctgtttt gattgatctt 1740

cctaaagata ttcagcagca attggttgtt cctgattggg ataggccttt taagttgggt 1800

gggtatatgt ctaggctgcc aaagtccaag ttttcgacga atgaggttgg acttcttgag 1860

cagattgtga ggttgatgag tgagtcgaag aagcctgtct tgtatgtggg aggtgggtgt 1920

ttgaattcta gtgaggagtt gaggagattt gttgagttga cagggattcc ggtggctagt 1980

actttgatgg ggttggggtc ttacccttgt aatgatgaac tgtctcttca tatgttgggg 2040

atgcacggga ctgtttatgc caattatgcg gtggataagg cggatttgtt gcttgctttc 2100

ggggttaggt ttgatgatcg tgtgaccggg aagctcgagg cgtttgctag ccgtgctaag 2160

attgtgcata ttgatattga ctctgctgag attgggaaga acaagcagcc ccatgtgtcc 2220

atttgtgctg atgttaaatt ggcattgcgg ggtatgaata agattctgga gtctagaata 2280

gggaagctga atttggattt ctccaagtgg agagaagaat taggtgagca gaagaaggaa 2340

ttcccactga gttttaagac atttggggat gcaattcctc cacaatatgc cattcaggtg 2400

cttgatgagt tgaccaatgg taatgctatt ataagtactg gtgttgggca gcaccaaatg 2460

tgggctgcgc agcattacaa gtacagaaac cctcgccaat ggctgacctc tggtgggttg 2520

ggggctatgg ggtttgggct accagccgcc attggagctg cagttgctcg accagatgca 2580

gtggttgtcg atattgatgg ggatggcagt tttattatga atgttcaaga gttggctaca 2640

attagggtgg aaaatctccc agttaagata atgctgctaa acaatcaaca tttaggtatg 2700

gttgtccaat tggaagatag gttctataaa gctaaccggg cacatacata ccttggaaac 2760

ccttccaaat ctgctgatat cttccctgat atgctcaaat tcgctgaggc atgtgatatt 2820

ccttctgccc gtgttagcaa cgtggctgat ttgagggccg ccattcaaac aatgttggat 2880

actccagggc cgtacctgct cgatgtgatt gtaccgcatc aagagcatgt gttgcctatg 2940

attccaagtg gtgccggttt caaggatacc attacagagg gtgatggaag aacctcttat 3000

tgatcggttt aatgacggtt ggaaccattt aaagagggta agctatatta ctgtatgtat 3060

attagtatgt tcctggataa tttagaagct tttgtctgtt gtcttttgca gtttatgaag 3120

ttagtttgct gttgtcatgt tacttgttac tttaaaaagc tttttgtagt ttttgagcaa 3180

ctagtatgga atgctcttcc tgtattgctt ggaaaattca caaaagtggt ttttcggcta 3240

tggatgttgt gttgcatcat gcatatatag cttgatatac tagttggctt ggtgcatctt 3300

taacatatac taatgagact acgacagcaa ttgccaatta gttggcttga cataattctt 3360

agtctgccac tagaaatctt gcttcttttt tttccctcat ttgttgaaag tccctgttgc 3420

gacctgacat gggagcattg gagattgttt agcaatagca gttgcagtta ggtgactaca 3480

gtcatcttcc aaatatgaat actctctgga ggggaggagg ttttacaaaa tatgagtttt 3540

taacacatga gaaacttata ttaaacaagg ttgagtcacc ccatattttt caaagttgac 3600

ttttgtctga tttgggtgac tatgcctgtt gtatgcaata aactcgatgt acataagact 3660

tgtataatcc aatctaaccc ttcctggctg attatgaaac cgagtcggct aatttgttgc 3720

ttgatcttca tgtgtgagcc tgatgccagg tgaccactag aggagtacct ttcattgaga 3780

tatcgattcg gttaattggt tcctcatatg ggtctcaaaa ctgaattttt ctcaggctct 3840

cttctaacca gttgttgaat tttatgaaac ttcagtccag ttaaaacttt gatcccggat 3900

cagaatttct ctgagttgtt cttccgcctc tcagttccac tgttccagtg ttcttggtcc 3960

tcaacctgta ctctgagcta tttctgcaaa cacctgaagt tcctgctgcc cttggacaaa 4020

tacagaatgc agcattagca tttatttaag acaagatgaa tacttgctcc cctgttagtt 4080

atgctaattc tgcattagta ttctttaatt ttaattagat actgcaatcc gtgaactctg 4140

cagtttcttg atctctctct gtttcaattt ctcctatctt atcgcccatt tctttaggct 4200

ttcgttatct atcgtctaat tcaagagtag gtaactaggt acgggataaa actttttcat 4260

gaagaaccta tgttcccgta ttatgctgtc ccacaagctt caactttcta ccttgttttc 4320

tatacgttgg acaacttctt ttgtgttgaa ctgatttaat tgataaatga ataattttgg 4380

ataaagaaaa ttgattgacc aataatttat ttattttagt ttacgttttg tatataccga 4440

ctgggctcat gtgagcacat ttatgtgcac aatttttttt atgtgaaaac aaaactaagc 4500

t 4501

<210> 11
<211> 1998
<212> DNA
<213> Artificial Sequence

<220>
<223> cDNA of mutated als gene (P188S and W569L)

<400> 11
atggcggcta ccttcacaaa cccaacattt tccccttcct caactccatt aaccaaaacc 60

ctaaaatccc aatcttccat ctcttcaacc ctcccctttt ccacccctcc caaaacccca 120

actccactct ttcaccgtcc cctccaaatc tcatcctccc aatcccacaa atcatccgcc 180

attaaaacac aaactcaagc accttcttct ccagctattg aagattcatc tttcgtttct 240

cgatttggcc ctgatgaacc cagaaaaggg tccgatgtcc tcgttgaagc tcttgagcgt 300

gaaggtgtta ccaatgtgtt tgcttaccct ggtggtgcat ctatggaaat ccaccaagct 360

ctcacacgct ctaaaaccat ccgcaatgtc ctccctcgcc atgaacaagg cggggttttc 420

gccgccgagg gatatgctag agctactgga aaggttggtg tctgcattgc gacttctggt 480

cctggtgcta ccaacctcgt atcaggtctt gctgacgctc tccttgattc tgtccctctt 540

gttgccatca ctggccaagt ttcacgccgt atgattggca ctgatgcttt tcaggagact 600

ccaattgttg aggtgacaag gtctattact aagcataatt atttagtttt ggatgtagag 660

gatattccta gaattgttaa ggaagccttt tttttagcta attctggtag gcctggacct 720

gttttgattg atcttcctaa agatattcag cagcaattgg ttgttcctga ttgggatagg 780

ccttttaagt tgggtgggta tatgtctagg ctgccaaagt ccaagttttc gacgaatgag 840

gttggacttc ttgagcagat tgtgaggttg atgagtgagt cgaagaagcc tgtcttgtat 900

gtgggaggtg ggtgtttgaa ttctagtgag gagttgagga gatttgttga gttgacaggg 960

attccggtgg ctagtacttt gatggggttg gggtcttacc cttgtaatga tgaactgtct 1020

cttcatatgt tggggatgca cgggactgtt tatgccaatt atgcggtgga taaggcggat 1080

ttgttgcttg ctttcggggt taggtttgat gatcgtgtga ccgggaagct cgaggcgttt 1140

gctagccgtg ctaagattgt gcatattgat attgactctg ctgagattgg gaagaacaag 1200

cagccccatg tgtccatttg tgctgatgtt aaattggcat tgcggggtat gaataagatt 1260

ctggagtcta gaatagggaa gctgaatttg gatttctcca agtggagaga agaattaggt 1320

gagcagaaga aggaattccc actgagtttt aagacatttg gggatgcaat tcctccacaa 1380

tatgccattc aggtgcttga tgagttgacc aatggtaatg ctattataag tactggtgtt 1440

gggcagcacc aaatgtgggc tgcgcagcat tacaagtaca gaaaccctcg ccaatggctg 1500

acctctggtg ggttgggggc tatggggttt gggctaccag ccgccattgg agctgcagtt 1560

gctcgaccag atgcagtggt tgtcgatatt gatggggatg gcagttttat tatgaatgtt 1620

caagagttgg ctacaattag ggtggaaaat ctcccagtta agataatgct gctaaacaat 1680

caacatttag gtatggttgt ccaattggaa gataggttct ataaagctaa ccgggcacat 1740

acataccttg gaaacccttc caaatctgct gatatcttcc ctgatatgct caaattcgct 1800

gaggcatgtg atattccttc tgcccgtgtt agcaacgtgg ctgatttgag ggccgccatt 1860

caaacaatgt tggatactcc agggccgtac ctgctcgatg tgattgtacc gcatcaagag 1920

catgtgttgc ctatgattcc aagtggtgcc ggtttcaagg ataccattac agagggtgat 1980

ggaagaacct cttattga 1998

<210> 12
<211> 665
<212> PRT
<213> Artificial Sequence

<220>
<223> protein of mutated als gene (P188S and W569L)

<400>  12

Met Ala Ala Thr Phe Thr Asn Pro Thr Phe Ser Pro Ser Ser Thr Pro
1               5                   10                  15

Leu Thr Lys Thr Leu Lys Ser Gln Ser Ser Ile Ser Ser Thr Leu Pro
            20                  25                  30

Phe Ser Thr Pro Pro Lys Thr Pro Thr Pro Leu Phe His Arg Pro Leu
        35                  40                  45

Gln Ile Ser Ser Ser Gln Ser His Lys Ser Ser Ala Ile Lys Thr Gln
    50                  55                  60

Thr Gln Ala Pro Ser Ser Pro Ala Ile Glu Asp Ser Ser Phe Val Ser
65                  70                  75                  80

Arg Phe Gly Pro Asp Glu Pro Arg Lys Gly Ser Asp Val Leu Val Glu
                85                  90                  95

Ala Leu Glu Arg Glu Gly Val Thr Asn Val Phe Ala Tyr Pro Gly Gly
            100                 105                 110

Ala Ser Met Glu Ile His Gln Ala Leu Thr Arg Ser Lys Thr Ile Arg
        115                 120                 125

Asn Val Leu Pro Arg His Glu Gln Gly Gly Val Phe Ala Ala Glu Gly
    130                 135                 140

Tyr Ala Arg Ala Thr Gly Lys Val Gly Val Cys Ile Ala Thr Ser Gly
145                 150                 155                 160

Pro Gly Ala Thr Asn Leu Val Ser Gly Leu Ala Asp Ala Leu Leu Asp
                165                 170                 175

Ser Val Pro Leu Val Ala Ile Thr Gly Gln Val Ser Arg Arg Met Ile
            180                 185                 190

Gly Thr Asp Ala Phe Gln Glu Thr Pro Ile Val Glu Val Thr Arg Ser
        195                 200                 205

Ile Thr Lys His Asn Tyr Leu Val Leu Asp Val Glu Asp Ile Pro Arg
    210                 215                 220

Ile Val Lys Glu Ala Phe Phe Leu Ala Asn Ser Gly Arg Pro Gly Pro
225                 230                 235                 240

Val Leu Ile Asp Leu Pro Lys Asp Ile Gln Gln Gln Leu Val Val Pro

                245                 250                 255

Asp Trp Asp Arg Pro Phe Lys Leu Gly Gly Tyr Met Ser Arg Leu Pro
            260                 265                 270

Lys Ser Lys Phe Ser Thr Asn Glu Val Gly Leu Leu Glu Gln Ile Val
        275                 280                 285

Arg Leu Met Ser Glu Ser Lys Lys Pro Val Leu Tyr Val Gly Gly Gly
    290                 295                 300

Cys Leu Asn Ser Ser Glu Glu Leu Arg Arg Phe Val Glu Leu Thr Gly
305                 310                 315                 320

Ile Pro Val Ala Ser Thr Leu Met Gly Leu Gly Ser Tyr Pro Cys Asn
                325                 330                 335

Asp Glu Leu Ser Leu His Met Leu Gly Met His Gly Thr Val Tyr Ala
            340                 345                 350

Asn Tyr Ala Val Asp Lys Ala Asp Leu Leu Leu Ala Phe Gly Val Arg
        355                 360                 365

Phe Asp Asp Arg Val Thr Gly Lys Leu Glu Ala Phe Ala Ser Arg Ala
    370                 375                 380

Lys Ile Val His Ile Asp Ile Asp Ser Ala Glu Ile Gly Lys Asn Lys
385                 390                 395                 400

Gln Pro His Val Ser Ile Cys Ala Asp Val Lys Leu Ala Leu Arg Gly
                405                 410                 415

Met Asn Lys Ile Leu Glu Ser Arg Ile Gly Lys Leu Asn Leu Asp Phe
            420                 425                 430

Ser Lys Trp Arg Glu Glu Leu Gly Glu Gln Lys Lys Glu Phe Pro Leu
        435                 440                 445

Ser Phe Lys Thr Phe Gly Asp Ala Ile Pro Pro Gln Tyr Ala Ile Gln
    450                 455                 460

Val Leu Asp Glu Leu Thr Asn Gly Asn Ala Ile Ile Ser Thr Gly Val
465                 470                 475                 480

Gly Gln His Gln Met Trp Ala Ala Gln His Tyr Lys Tyr Arg Asn Pro
                485                 490                 495

Arg Gln Trp Leu Thr Ser Gly Gly Leu Gly Ala Met Gly Phe Gly Leu
            500                 505                 510

Pro Ala Ala Ile Gly Ala Ala Val Ala Arg Pro Asp Ala Val Val Val
        515                 520                 525

Asp Ile Asp Gly Asp Gly Ser Phe Ile Met Asn Val Gln Glu Leu Ala
    530                 535                 540

Thr Ile Arg Val Glu Asn Leu Pro Val Lys Ile Met Leu Leu Asn Asn
545                 550                 555                 560

Gln His Leu Gly Met Val Val Gln Leu Glu Asp Arg Phe Tyr Lys Ala
                565                 570                 575

Asn Arg Ala His Thr Tyr Leu Gly Asn Pro Ser Lys Ser Ala Asp Ile
            580                 585                 590

Phe Pro Asp Met Leu Lys Phe Ala Glu Ala Cys Asp Ile Pro Ser Ala
        595                 600                 605

Arg Val Ser Asn Val Ala Asp Leu Arg Ala Ala Ile Gln Thr Met Leu
    610                 615                 620

Asp Thr Pro Gly Pro Tyr Leu Leu Asp Val Ile Val Pro His Gln Glu
625                 630                 635                 640

His Val Leu Pro Met Ile Pro Ser Gly Ala Gly Phe Lys Asp Thr Ile
                645                 650                 655

Thr Glu Gly Asp Gly Arg Thr Ser Tyr
            660                 665

<210>  13
<211>  21
<212>  DNA
<213>  Artificial Sequence

<220>
<223>  BvEPSPS_Ex_2_for

<400>  13
ggaaatttcc atcctaacga g                                                21

<210>  14
<211>  21
<212>  DNA
<213>  Artificial Sequence

<220>
<223>  BvEPSPS_Ex_2_rev

<400>  14
gcaagaggaa acaagtctcc a                                                 21


<210>  15
<211>  20
<212>  DNA
<213>  Artificial Sequence

<220>
<223>  BvEPSPS_Ex2_Seqf

<400>  15
catcctaacg agaattatgc                                                   20


<210>  16
<211>  20
<212>  DNA
<213>  Artificial Sequence

<220>
<223>  BvEPSPS_Ex2_Seqr

<400>  16
gtctccacac aaaataaaag                                                   20


<210>  17
<211>  43
<212>  DNA
<213>  Artificial Sequence

<220>
<223>  KASP-marker s1txepss02 - Primer_Allel_C

<400>  17
gaaggtgacc aagttcatgc tcagcaactg cagctgtcaa tgg                         43


<210>  18
<211>  45
<212>  DNA
<213>  Artificial Sequence

<220>
<223>  KASP-marker s1txepss02 - Primer_Allel_T

<400>  18
gaaggtcgga gtcaacggat taacagcaac tgcagctgtc aatga                       45


<210>  19
<211>  29
<212>  DNA
<213>  Artificial Sequence

<220>
<223>  KASP-marker s1txepss02 - Primer_Common

<400>  19
ctttttcttg gaaatgcagg aacagcaat                                         29

<210>  20
<211>  517
<212>  PRT
<213>  Beta vulgaris

<400>  20

Met Ala Gln Ala Ser Thr Ile Asn Asn Gly Val Lys Ser Thr Gln Leu
1               5                   10                  15

Cys Pro Asn Leu Pro Lys Thr His Leu Ser Lys Ser Ser Lys Ser Val
            20                  25                  30

Lys Phe Gly Ser Asn Leu Arg Phe Ser Pro Lys Leu Lys Ser Phe Asn
        35                  40                  45

Asn Glu Arg Val Gly Gly Asn Ser Ser Val Val Phe Arg Val Arg Ala
    50                  55                  60

Ser Val Ala Ala Ala Ala Glu Lys Ser Ser Thr Val Pro Glu Ile Val
65                  70                  75                  80

Leu Gln Pro Ile Lys Glu Ile Ser Gly Thr Ile Gln Leu Pro Gly Ser
                85                  90                  95

Lys Ser Leu Ser Asn Arg Ile Leu Leu Leu Ala Ala Leu Ser Gln Gly
            100                 105                 110

Thr Thr Val Val Asp Asn Leu Leu Tyr Ser Asp Asp Ile Arg Tyr Met
        115                 120                 125

Leu Asp Ala Leu Arg Thr Leu Gly Leu Asn Val Glu Asp Asp Asn Ile
    130                 135                 140

Ala Lys Arg Ala Ile Val Glu Gly Cys Gly Gly Leu Phe Pro Val Gly
145                 150                 155                 160

Lys Asp Gly Lys Glu Ile Glu Leu Phe Leu Gly Asn Ala Gly Thr Ala
                165                 170                 175

Met Arg Pro Leu Thr Ala Ala Val Ala Val Ala Gly Gly Asn Ser Ser
            180                 185                 190

Tyr Val Leu Asp Gly Val Pro Arg Met Arg Glu Arg Pro Ile Gly Asp
        195                 200                 205

Leu Val Ala Gly Leu Lys Gln Leu Gly Ala Asp Val Asp Cys Tyr Leu
    210                 215                 220

Gly Thr Asn Cys Pro Pro Val Arg Val Asn Ala Lys Gly Gly Leu Pro
225                 230                 235                 240

Gly Gly Lys Val Lys Leu Ser Gly Ser Val Ser Ser Gln Tyr Leu Thr
                245                 250                 255

Ala Leu Leu Met Ala Thr Pro Leu Gly Leu Gly Asp Val Glu Val Glu
            260                 265                 270

Ile Ile Asp Lys Leu Ile Ser Val Pro Tyr Val Glu Met Thr Ile Lys
        275                 280                 285

Leu Met Glu Arg Phe Gly Val Ser Val Glu His Ser Ala Asp Trp Gly
    290                 295                 300

Arg Phe Leu Ile Arg Gly Gly Gln Lys Tyr Lys Ser Pro Gly Asn Ala
305                 310                 315                 320

Tyr Val Glu Gly Asp Ala Ser Ser Ala Ser Tyr Phe Ile Gly Gly Ala
                325                 330                 335

Ala Val Thr Gly Gly Thr Val Thr Val Glu Gly Cys Gly Thr Ser Ser
            340                 345                 350

Leu Gln Gly Asp Val Lys Phe Ala Glu Val Leu Glu Lys Met Gly Cys
        355                 360                 365

Lys Val Ser Trp Thr Glu Asn Ser Val Thr Val Thr Gly Pro Pro Arg
    370                 375                 380

Asp Ala Ser Gly Arg Lys His Leu Arg Ala Val Asp Val Asn Met Asn
385                 390                 395                 400

Lys Met Pro Asp Val Ala Met Thr Leu Ala Val Val Ala Leu Tyr Ala
                405                 410                 415

Asp Gly Pro Thr Thr Ile Arg Asp Val Ala Ser Trp Arg Val Lys Glu
            420                 425                 430

Thr Glu Arg Met Ile Ala Ile Cys Thr Glu Leu Arg Lys Leu Gly Ala
        435                 440                 445

Thr Val Glu Glu Gly Ser Asp Tyr Cys Val Ile Thr Pro Pro Glu Lys
    450                 455                 460

Leu Asn Val Thr Ala Ile Asp Thr Tyr Asp Asp His Arg Met Ala Met

465                 470                 475                 480

Ala Phe Ser Leu Ala Ala Cys Ala Asp Val Pro Val Thr Ile Lys Asp
                485                 490                 495

Pro Gly Cys Thr Arg Lys Thr Phe Pro Asp Tyr Phe Asp Val Leu Glu
            500                 505                 510

Arg Phe Ala Lys His
        515

<210>  21
<211>  665
<212>  PRT
<213>  Beta vulgaris

<400>  21

Met Ala Ala Thr Phe Thr Asn Pro Thr Phe Ser Pro Ser Ser Thr Pro
1               5                   10                  15

Leu Thr Lys Thr Leu Lys Ser Gln Ser Ser Ile Ser Ser Thr Leu Pro
            20                  25                  30

Phe Ser Thr Pro Pro Lys Thr Pro Thr Pro Leu Phe His Arg Pro Leu
        35                  40                  45

Gln Ile Ser Ser Ser Gln Ser His Lys Ser Ser Ala Ile Lys Thr Gln
    50                  55                  60

Thr Gln Ala Pro Ser Ser Pro Ala Ile Glu Asp Ser Ser Phe Val Ser
65                  70                  75                  80

Arg Phe Gly Pro Asp Glu Pro Arg Lys Gly Ser Asp Val Leu Val Glu
                85                  90                  95

Ala Leu Glu Arg Glu Gly Val Thr Asn Val Phe Ala Tyr Pro Gly Gly
            100                 105                 110

Ala Ser Met Glu Ile His Gln Ala Leu Thr Arg Ser Lys Thr Ile Arg
        115                 120                 125

Asn Val Leu Pro Arg His Glu Gln Gly Gly Val Phe Ala Ala Glu Gly
    130                 135                 140

Tyr Ala Arg Ala Thr Gly Lys Val Gly Val Cys Ile Ala Thr Ser Gly
145                 150                 155                 160

Pro Gly Ala Thr Asn Leu Val Ser Gly Leu Ala Asp Ala Leu Leu Asp

                165                 170                 175

Ser Val Pro Leu Val Ala Ile Thr Gly Gln Val Pro Arg Arg Met Ile
            180                 185                 190

Gly Thr Asp Ala Phe Gln Glu Thr Pro Ile Val Glu Val Thr Arg Ser
        195                 200                 205

Ile Thr Lys His Asn Tyr Leu Val Leu Asp Val Glu Asp Ile Pro Arg
    210                 215                 220

Ile Val Lys Glu Ala Phe Phe Leu Ala Asn Ser Gly Arg Pro Gly Pro
225                 230                 235                 240

Val Leu Ile Asp Leu Pro Lys Asp Ile Gln Gln Gln Leu Val Val Pro
                245                 250                 255

Asp Trp Asp Arg Pro Phe Lys Leu Gly Gly Tyr Met Ser Arg Leu Pro
            260                 265                 270

Lys Ser Lys Phe Ser Thr Asn Glu Val Gly Leu Leu Glu Gln Ile Val
        275                 280                 285

Arg Leu Met Ser Glu Ser Lys Lys Pro Val Leu Tyr Val Gly Gly Gly
    290                 295                 300

Cys Leu Asn Ser Ser Glu Glu Leu Arg Arg Phe Val Glu Leu Thr Gly
305                 310                 315                 320

Ile Pro Val Ala Ser Thr Leu Met Gly Leu Gly Ser Tyr Pro Cys Asn
                325                 330                 335

Asp Glu Leu Ser Leu His Met Leu Gly Met His Gly Thr Val Tyr Ala
            340                 345                 350

Asn Tyr Ala Val Asp Lys Ala Asp Leu Leu Leu Ala Phe Gly Val Arg
        355                 360                 365

Phe Asp Asp Arg Val Thr Gly Lys Leu Glu Ala Phe Ala Ser Arg Ala
    370                 375                 380

Lys Ile Val His Ile Asp Ile Asp Ser Ala Glu Ile Gly Lys Asn Lys
385                 390                 395                 400

Gln Pro His Val Ser Ile Cys Ala Asp Val Lys Leu Ala Leu Arg Gly
                405                 410                 415

Met Asn Lys Ile Leu Glu Ser Arg Ile Gly Lys Leu Asn Leu Asp Phe
            420                 425                 430

Ser Lys Trp Arg Glu Glu Leu Gly Glu Gln Lys Lys Glu Phe Pro Leu
        435                 440                 445

Ser Phe Lys Thr Phe Gly Asp Ala Ile Pro Pro Gln Tyr Ala Ile Gln
    450                 455                 460

Val Leu Asp Glu Leu Thr Asn Gly Asn Ala Ile Ile Ser Thr Gly Val
465                 470                 475                 480

Gly Gln His Gln Met Trp Ala Ala Gln His Tyr Lys Tyr Arg Asn Pro
                485                 490                 495

Arg Gln Trp Leu Thr Ser Gly Gly Leu Gly Ala Met Gly Phe Gly Leu
            500                 505                 510

Pro Ala Ala Ile Gly Ala Ala Val Ala Arg Pro Asp Ala Val Val Val
        515                 520                 525

Asp Ile Asp Gly Asp Gly Ser Phe Ile Met Asn Val Gln Glu Leu Ala
    530                 535                 540

Thr Ile Arg Val Glu Asn Leu Pro Val Lys Ile Met Leu Leu Asn Asn
545                 550                 555                 560

Gln His Leu Gly Met Val Val Gln Trp Glu Asp Arg Phe Tyr Lys Ala
                565                 570                 575

Asn Arg Ala His Thr Tyr Leu Gly Asn Pro Ser Lys Ser Ala Asp Ile
            580                 585                 590

Phe Pro Asp Met Leu Lys Phe Ala Glu Ala Cys Asp Ile Pro Ser Ala
        595                 600                 605

Arg Val Ser Asn Val Ala Asp Leu Arg Ala Ala Ile Gln Thr Met Leu
    610                 615                 620

Asp Thr Pro Gly Pro Tyr Leu Leu Asp Val Ile Val Pro His Gln Glu
625                 630                 635                 640

His Val Leu Pro Met Ile Pro Ser Gly Ala Gly Phe Lys Asp Thr Ile
                645                 650                 655

Thr Glu Gly Asp Gly Arg Thr Ser Tyr
            660                 665

## Description

### FIELD OF THE INVENTION

The invention relates to the use of herbicides, in particular glyphosate, for controlling weeds in Beta vulgaris growing areas as well as glyphosate tolerant Beta vulgaris plants.

### BACKGROUND OF THE INVENTION

Sugar beet (Beta vulgaris) is grown as a commercial crop in many countries, with a combined harvest of over 240 million metric tons. N-phosphonomethyl-glycine, commonly referred to as glyphosate, is a broad-spectrum herbicide, which is widely used due to its high efficiency, biodegradability, and low toxicity to animals and humans. Glyphosate inhibits the shikimic acid pathway which leads to the biosynthesis of aromatic compounds including amino acids and vitamins. Specifically, glyphosate inhibits the conversion of phosphoenolpyruvic acid and 3-phosphoshikimic acid to 5-enolpyruvyl-3-phosphoshikimic acid by inhibiting the enzyme 5-enolpyruvyl-3-phosphoshikimic acid synthase (EPSP synthase or EPSPS). When conventional plants are treated with glyphosate, the plants cannot produce the aromatic amino acids (e.g. phenylalanine and tyrosine) needed to grow and survive. EPSPS is present in all plants, bacteria, and fungi. It is not present in animals, which do not synthesize their own aromatic amino acids. Because the aromatic amino acid biosynthetic pathway is not present in mammals, birds or aquatic life forms, glyphosate has little if any toxicity for these organisms.

Glyphosate is the active ingredient in herbicides such as Roundup@, produced by Monsanto Company, USA. Typically, it is formulated as a water-soluble salt such as an ammonium, alkylamine, alkali metal or trimethylsulfonium salt. One of the most common formulations is the isopropylamine salt of glyphosate, which is the form employed in Roundup® herbicide.

It has been shown that glyphosate tolerant plants can be produced by inserting into the genome of the plant the capacity to produce an EPSP synthase which is glyphosate tolerant, e.g. the CP4-EPSPS from Agrobacterium sp. strain CP4. A glyphosate tolerant sugar beet plant may be produced by Agrobacterium mediated transformation, introducing a gene coding for a glyphosate tolerant EPSPS such as CP4-EPSPS into the plant's genome. Such a sugar beet plant, expressing CP4-EPSPS, has been described in WO 99/23232 or WO 2004/074492 A1. However, sugar beet plants grown from cells transformed with the gene for CP4-EPSPS in this manner, differ widely in their characteristics, due to the fact that the gene is inserted at a random position in the plant genome. Such transgenic sugar beets are considered as being genetically modified organisms (GMO). Therefore, the acceptance in the public domain is very low while the costs for deregulation are extremely high. Furthermore, there are strict legal limitation with respect to the commercialization of such plants or seeds thereof.

That is why, even though the glyphosate is a well-studied and highly efficient herbicide system it is not commercially applicable in sugar beet in many territories worldwide. It would thus be highly desirable to use glyphosate herbicides for control of unwanted vegetation in Beta vulgaris plants, preferably sugar beet plants which are tolerant to such glyphosate herbicides. The present invention has the objective to solve this problem.

### SUMMARY OF THE INVENTION

The inventors developed methods for controlling unwanted vegetation in Beta vulgaris, in particular sugar beet, growing areas and thereby increasing the yield of these areas. One essential element is the provision of a new glyphosate resistant Beta vulgaris plant, in particular sugar beet. This herbicide resistance trait does not rely on the transgenic modification of the genome like in the sugar beets as disclosed in WO 2004/074492 A1, but on a single point mutation in the endogenous epsp synthase gene, such as created by use of mutagenic agents. Such mutant Beta vulgaris plants are not considered as being genetically modified organisms (GMO). Therefore, the acceptance in the public domain is high as well as the legal limitation with respect to the commercialization of such plants are minimal.

Furthermore, in certain embodiments the inventive methods also make use of another herbicide resistant sugar beet which entered the market recently (WO 2012/049268 A1). These sugar beet plants carry a point mutation in the endogenous acetolactate gene which confers resistance to another herbicide class, so called ALS-inhibitors. The invention is in particular captured by the appended claims, which are incorporated herein explicitly by reference.

In an aspect, the invention relates to the use of a glyphosate herbicide for controlling unwanted vegetation in growing areas of Beta vulgaris whereby the Beta vulgaris plants comprise an endogenous allele which is modified to encode a modified epsp synthase. More particularly, the Beta vulgaris plants comprise an allele encoding an epsp synthase having at position 179 an amino acid different from proline.

In a further aspect, the invention relates to a sugar beet plant or plant part comprising an endogenous allele encoding an epsp synthase having at position 179 an amino acid different from proline.

In a further aspect, the invention relates to a method for producing sugar, comprising:
a) providing the root beet of the beet plant according to the invention as described above, b) extracting sugar from said root beet.

In a further aspect, the invention relates to the use of the sugar beet plant of plant part according to the invention as described above in a method of sugar production, anaerobic digestion, or fermentation.

In a further aspect, the invention relates to the use of the sugar beet plant of plant part according to the invention as described above in a method of sugar, biogas or biofuel production.

In a further aspect, the invention relates to a method comprising identifying in a sugar beet plant or plant part an endogenous allele encoding an epsp synthase having at position 179 an amino acid different from proline.

### DETAILED DESCRIPTION OF THE INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of", as well as the terms "consisting essentially of", "consists essentially" and "consists essentially of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-20% or less, preferably +/-10% or less, more preferably +/-5% or less, and still more preferably +/-1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates) ("Ausubel et al. 1992"); the series Methods in Enzymology (Academic Press, Inc.); Innis et al., PCR Protocols: A Guide to Methods and Applications, Academic Press: San Diego, 1990; PCR 2: A Practical Approach (M.J. MacPherson, B.D. Hames and G.R. Taylor eds. (1995); Harlow and Lane, eds. (1988) Antibodies, a Laboratory Manual; and Animal Cell Culture (R.I. Freshney, ed. (1987). General principles of microbiology are set forth, for example, in Davis, B. D. et al., Microbiology, 3rd edition, Harper & Row, publishers, Philadelphia, Pa. (1980).

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

It is to be understood that other embodiments may be utilised and structural or logical changes may be made without departing from the scope of the present invention. The following detailed description, therefore, is not to be taken in a limiting sense, and the scope of the present invention is defined by the appended claims.

Preferred statements (features) and embodiments of this invention are set herein below. Each statements and embodiments of the invention so defined may be combined with any other statement and/or embodiments unless clearly indicated to the contrary. In particular, any feature indicated as being preferred or advantageous may be combined with any other feature or features or statements indicated as being preferred or advantageous. Hereto, the present invention is in particular captured by any one or any combination of one or more of the below numbered aspects and embodiments 1 to 48, with any other statement and/or embodiments.
1. A method for controlling bolters, weed beets, or annual beets in sugar beet growing areas or for increasing the beet yield in Beta vulgaris growing areas, in particular in sugar beet growing areas, comprising the steps of: a) planting Beta vulgaris plants, in particular sugar beet plants, or sowing Beta vulgaris seeds, in particular sugar beet seeds, comprising an endogenous allele encoding an epsp synthase having at position 179 an amino acid different from proline, b) applying a glyphosate herbicide to the growing plants, preferably at a dosage sufficient for inhibiting the growth of the bolters, weed beets, or annual beets, more preferably at a dosage sufficient for killing the bolters, weed beets or annual beets, and c) optionally, repeating step b) during the growing season.
2. Use of a glyphosate herbicide for controlling unwanted vegetation, in particular weed beets, annual beets, bolters and/or weeds, in Beta vulgaris growing areas, in particular sugar beet growing areas, in which the Beta vulgaris plants, in particular sugar beet plants comprise an endogenous allele encoding an epsp synthase having at position 179 an amino acid different from proline.
3. The method or use of statement 1 or 2, wherein the glyphosate herbicide is selected from glyphosate or a derivative thereof, preferably wherein said derivative is a salt, ester, amide, or alkylamide; preferably wherein said salt is an alkali metal salt such as (mono-, di-, or tri-) sodium or (mono-, di-, or tri-) potassium, an ammonium salt, a di-ammonium salt such as dimethylammonium, an alkylamine salt such as C1-C16 alkylamine salts such as dimethylamine, ethylamine, ethylenediamine, hexamethylenediamine, n-propylamine, and isopropylamine salts, an alkylammonium salt such as C1-C16 alkylammonium salts such as dimethylammonium and isopropylammonium salts such as monoisopropylammonium salt (IPA), an alkanolamine salt such as C1-C16 alkanolamine salts such as (mono-, di-, or tri-) ethanolamine salts such as monoethanolammonium salt (MEA), an alkylsulfonium salt such as trimethylsulfonium salts (TMS), a sulfoxonium salt, and mixtures or combinations thereof.
4. The method or use of any of statements 1 to 3, wherein all endogenous alleles encoding epsp synthases have at position 179 an amino acid different from proline.
5. The method or use of any of statements 1 to 4, wherein the endogenous allele(s) encoding the epsp synthase(s) having at position 179 an amino acid selected from the group of Serine, Threonine, Alanine and Leucine, preferably the amino acid is Serine.
6. The method or use of any of statements 1 to 5, wherein the epsp synthase having at position 179 an amino acid different from proline, comprises an amino acid sequence selected from i) the sequence of SEQ ID NO: 3, ii) the sequence of i) having at position 179 an amino acid different from serine and proline, or iii) a sequence having an identity of at least 90%, preferably at least 95%, more preferably at least 98%, such as at least 99% to the sequence of i) or ii), preferably over the entire length of the sequence, and preferably having EPSP synthase activity.
7. The method or use of any of statements 1 to 6, wherein the endogenous allele(s) encoding an epsp synthase having at position 179 an amino acid different from proline, comprises a nucleotide sequence selected from: i) the sequence of SEQ ID NO: 1,ii) a sequence having the coding sequence of SEQ ID NO: 2,iii) the sequence of i) or ii) having nucleotides corresponding to amino acid position 179 of SEQ ID NO: 3 or corresponding to the codon of amino acid position 179 of SEQ ID NO: 3, and encoding an amino acid different from serine and proline at said position 179,iv) a sequence having an identity of at least 90%, preferably at least 95%, more preferably at least 98%, such as at least 99% to the sequence of i), ii), or iii), preferably over the entire length of the sequence, and preferably having EPSP synthase activity, and/or which hybridizes under stringent conditions with the reverse complement of the sequences of i), ii), or iii); or v) a nucleotide sequence encoding the epsp synthase according to statement 6.
8. The method or use of any of statements 1 to 7, wherein the endogenous allele or all endogenous alleles encoding the epsp synthase(s) has/have additionally at position 175 an amino acid different from threonine, preferably the amino acid is isoleucine.
9. The method or use of statement 8, wherein the epsp synthase(s) having additionally at position 175 an amino acid different from threonine, comprise(s) an amino acid sequence selected from: i) the sequence of SEQ ID NO: 6, or ii) the sequence of i) having at position 175 an amino acid different from isoleucine and threonine, or iii) a sequence having an identity of at least 90%, preferably at least 95%, more preferably at least 98%, such as at least 99% to the sequence of i) or ii), preferably over the entire length of the sequence, and preferably having EPSP synthase activity.
10. The method or use of statement 8 or 9, wherein the endogenous allele(s) encoding the epsp synthase(s) having additionally at position 175 an amino acid different from threonine, comprises a nucleotide sequence selected from: i) the sequence of SEQ ID NO: 4, ii) a sequence having the coding sequence of SEQ ID NO: 5, iii) the sequence of i) or ii) having nucleotides corresponding to amino acid position 175 of SEQ ID NO: 6 or corresponding to the codon of amino acid position 175 of SEQ ID NO: 6, and encoding an amino acid different from isoleucine and threonine at said position 175, iv) a sequence having an identity of at least 90%, preferably at least 95%, more preferably at least 98%, such as at least 99% to the sequence of i), ii), or iii), preferably over the entire length of the sequence, and preferably having EPSP synthase activity, and/or which hybridizes under stringent conditions with the reverse complement of the sequences of i), ii), or iii); or v) a nucleotide sequence encoding the epsp synthase according to statement 9.
11. The method or use of any of statements 1 to 10, wherein (in step b) of the method) at least 300 g/ha active ingredient glyphosate is applied to the growing plants, preferably at least 600 g/ha active ingredient glyphosate, more preferably at least 1200 g/ha active ingredient glyphosate, preferably wherein said active ingredient is glyphosate acid equivalent.
12. The method of any of statements 1 to 11, wherein step b) is performed before pollination of flowers of bolters, weed beets, or annual beets, preferably during pre-flowering stage or latest at the time when flowers open.
13. The method or use of any of statements 1 to 12, wherein the Beta vulgaris plants or Beta vulgaris seeds further comprise an endogenous allele encoding an acetolactate synthase (ALS) having at position 569 an amino acid different from tryptophan, preferably the amino acid is selected from the group of alanine, glycine, isoleucine, leucine, methionine, phenylalanine, proline, valine or arginine, preferably the amino acid is leucine.
14. The method or use of statement 13, wherein the endogenous allele encoding the acetolactate synthase has additionally at position 188 an amino acid different from proline, preferably the amino acid is selected from the group consisting of serine, threonine, arginine, leucine, glutamine, alanine, more preferably the amino acid is serine.
15. The method of any of statements 13 to 14, further comprising: a) in a prior or subsequent growing season planting Beta vulgaris plants or sowing Beta vulgaris seeds comprising an endogenous gene encoding an acetolactate synthase as defined in any of statements 13 to 14, b) applying an ALS inhibitor to the growing plants, and c) optionally, repeating step b) during the other growing season.
16. A Method for controlling bolters, weed beets, or annual beets in Beta vulgaris growing areas, in particular sugar beet growing areas, or for increasing the beet yield in Beta vulgaris growing areas, in particular sugar beet growing areas, comprising the steps of: a) planting Beta vulgaris plants, in particular sugar beet plants, or sowing Beta vulgaris seeds, in particular sugar beet seeds, comprising the endogenous allele encoding an epsp synthase as defined in any of statements 1 to 12 and an endogenous allele encoding an acetolactate synthase having at position 569 an amino acid different from tryptophan, b) applying glyphosate and/or an ALS inhibitor to the growing plants, preferably at a dosage sufficient for inhibiting the growth of the bolters, weed beets, or annual beets, more preferably at a dosage sufficient for killing the bolters, weed beets or annual beets, and c) optionally, repeating step b) during the growing season.
17. A method for controlling bolters, weed beets, or annual beets in Beta vulgaris growing areas, in particular sugar beet growing areas, or for increasing the beet yield in Beta vulgaris growing areas, in particular sugar beet growing areas, comprising the steps of: a) planting or Beta vulgaris plants, in particular sugar beet plants or sowing Beta vulgaris seeds, in particular sugar beet seeds, comprising an endogenous gene encoding an epsp synthase protein having at position 179 an amino acid different from proline and an endogenous gene encoding an acetolactate synthase having at position 569 an amino acid different from tryptophan, b) applying a first herbicide selected from i) glyphosate or ii) an ALS inhibitor herbicide to the growing plants, preferably at a dosage sufficient for inhibiting the growth of the bolters, weed beets, or annual beets, more preferably at a dosage sufficient for killing the bolters, weed beets or annual beets, c) applying a second herbicide selected from i) glyphosate or ii) an ALS inhibitor herbicide, different to the herbicide applied in step b) to the growing plants, preferably at a dosage sufficient for inhibiting the growth of the bolters, weed beets, or annual beets, more preferably at a dosage sufficient for killing the bolters, weed beets or annual beets, and d) optionally, repeating step b) and/or c) during the growing season.
18. The use of any of statements 13 or 14, in combination with an ALS inhibitor, preferably an ALS inhibitor selected from the group consisting of: sulfonylurea, sulfonylaminocarbonyltriazolinone, triazolopyrimidine, sulfonanilide, imidazolinone, pyrimidinyloxybenzoeacid, pyrimidinylthiobenzoeacid.
19. The use of statement 18, wherein the application of the respective herbicides (i) takes place jointly or simultaneously, or (ii) takes place at different times and/or in a plurality of portions (sequential application), in pre-emergence applications followed by post-emergence applications or early post-emergence applications followed by medium or late post-emergence applications.
20. The method or use of any of statements 13 to 19, wherein all endogenous alleles encoding an acetolactate synthase have at position 569 an amino acid different from tryptophan.
21. The method or use of any of statements 13 to 20, wherein the endogenous allele(s) encoding an acetolactate synthase has/have at position 569 an amino acid selected from the group of alanine, glycine, isoleucine, leucine, methionine, phenylalanine, proline, valine or arginine, preferably the amino acid is leucine.
22. The method or use of any of statements 13 to 21, wherein the acetolactate synthase(s) having at position 569 an amino acid different from tryptophan comprise(s) an amino acid sequence selected from: i) the sequence of SEQ ID NO: 9, ii) the sequence of i) having at position 569 an amino acid different from tryptophan and leucine, or iii) a sequence having an identity of at least 90%, preferably at least 95%, more preferably at least 98%, such as at least 99% to SEQ ID NO: 9, preferably over the entire length of the sequence, preferably having acetolactate synthase activity.
23. The method or use of any of statements 13 to 22, wherein the endogenous allele(s) encoding the acetolactate synthase(s) have a nucleotide sequence selected from: i) the sequence of SEQ ID NO: 7, ii) a sequence having the coding sequence of SEQ ID NO: 8, iii) the sequence of i) or ii) having nucleotides corresponding to amino acid position 569 of SEQ ID NO: 9 or corresponding to the codon of amino acid position 569 of SEQ ID NO: 9, and encoding an amino acid different from tryptophan and leucine at said position 569, iv) a sequence having an identity of at least 90%, preferably at least 95%, more preferably at least 98%, such as at least 99% to the sequence of i) or ii), preferably over the entire length of the sequence, preferably having acetolactate synthase activity, and/or which hybridizes under stringent conditions with the reverse complement of the sequences of i), ii), or iii); or v) a nucleotide sequence encoding the acetolactate synthase according to statement 22.
24. The method or use of any of statements 13 to 23, wherein the endogenous allele or all endogenous alleles encoding the an acetolactate synthase(s) having additionally at position 188 an amino acid different from proline, preferably the amino acid is selected from the group consisting of serine, threonine, arginine, leucine, glutamine, alanine, more preferably the amino acid is serine.
25. The method or use of statement 24, wherein the acetolactate synthase(s) having additionally at position 188 an amino acid different from proline, comprise(s) an amino acid sequence selected from: i) the sequence of SEQ ID NO: 12, ii) the sequence of i) having at position 188 an amino acid different from serine and proline, or iii) a sequence having an identity of at least 90%, preferably at least 95%, more preferably at least 98%, such as at least 99% to SEQ ID NO: 12, preferably over the entire length of the sequence, preferably having acetolactate synthase activity.
26. The method or use of statement 24 or 25, wherein the endogenous allele(s) encoding the acetolactate synthase(s) having additionally at position 188 an amino acid different from proline, comprise(s) a nucleotide sequence selected from: i) the sequence of SEQ ID NO: 10, ii) a sequence having the coding sequence of SEQ ID NO: 11, iii) the sequence of i) or ii) having nucleotides corresponding to amino acid position 188 of SEQ ID NO: 12 or corresponding to the codon of amino acid position 188 of SEQ ID NO: 12, and encoding an amino acid different from serine and proline at said position 188, iv) a sequence having an identity of at least 90%, preferably at least 95%, more preferably at least 98%, such as at least 99% to the sequence of i), ii), or iii), preferably over the entire length of the sequence, preferably having acetolactate synthase activity, and/or which hybridizes under stringent conditions with the reverse complement of the sequences of i), ii), or iii); or v) a nucleotide sequence encoding the acetolactate synthase according to statement 25.
27. The method or use of any one of statement 13 to 26, wherein (in step b) of the method) at least 300 g/ha active ingredient glyphosate is applied to the growing plants, preferably at least 600 g/ha active ingredient glyphosate is applied, more preferably at least 1200 g/ha, preferably wherein said active ingredient is glyphosate acid equivalent; and/or the ALS inhibitor is applied to the growing plants with a minimal dosage which is an equivalent to the mixtures of 35 g/ha foramsulfuron and 7 g/ha iodosulfuron-methyl-sodium.
28. The method or use of any one of statements 13 to 27, wherein the glyphosate herbicide and/or ALS inhibitor is applied before pollination of flowers of bolters, weed beets, or annual beets, preferably during pre-flowering stage or latest at the time when flowers open.
29. Method for controlling bolters, weed beets, or annual beets in Beta vulgaris growing areas, in particular sugar beet growing areas, or for increasing the beet yield in Beta vulgaris growing areas, in particular sugar beet growing areas, comprising I) conducting the method according to any of statements 1 to 12 in a growing season, and II) conducting the following steps in another growing season: a) planting sugar beet plants comprising an endogenous gene encoding an acetolactate synthase as defined in any one of statements 13 to 26, b) applying an ALS inhibitor to the growing plants, and c) optionally, repeating step b) during the other growing season.
30. The method of statement 29, wherein the other growing season of II) is before and/or after the growing season of I).
31. The method of statement 29 or 30, wherein in step II) b) the ALS inhibitor is applied to the growing plants with a minimal dosage which is an equivalent to the mixtures of 35 g/ha foramsulfuron and 7 g/ha iodosulfuron-methyl-sodium.
32. The method of any of statements 29 to 31, wherein step II) b) is performed before pollination of flowers of bolters, weed beets, or annual beets, preferably during pre-flowering stage or latest at the time when flowers open.
33. A method for producing Beta vulgaris beet roots, in particular sugar beets, in Beta vulgaris, in particular sugar beet growing areas, comprising the steps of: a) conducting the method of any of statements 1 to 32, and b) havesting Beta vulgaris beet roots, in particular sugar beets, preferably by the end of the growing season.
34. A method for providing a glyphosate resistant Beta vulgaris plant, in particular a sugar beet plant, comprising the steps of: a) mutagenizing Beta vulgaris, in particular sugar beet, cell or tissue with at least 0.5% EMS or at least 0.3% ENU, b) producing stecks from the mutagenized cells or tissue (M0), c) replanting stecks for producing a population of seeds (M1), d) producing seeds (M2) from plants grown from M1 seeds, e) sowing M2 seeds and applying at least 600 g/ha glyphosate active ingredient to growing plants, preferably wherein said active ingredient is glyphosate acid equivalent, f) optionally, replant surviving plants in pots and applying at least 600 g/ha glyphosate active ingredient to the growing plants, preferably wherein said active ingredient is glyphosate acid equivalent; and g) selecting surviving plants without herbicide damages or with minimal herbicide damages.
35. Method of statement 34, further comprise the steps of: h) optionally sequencing the endogenous epsp synthase alleles of the plants of g) or using the marker s1txepss02 on the plants of g), and i) selecting sugar beet plants comprising an endogenous allele encoding an epsp synthase having at position 179 an amino acid different from proline.
36. A glyphosate resistant Beta vulgaris plant, in particular sugar beet plant, or plant part obtained by the method of statement 34 or 35; or the progeny or seed thereof.
37. The Beta vulgaris plant, in particular sugar beet plant, of statement 36, wherein the plant is not exclusively obtained by means of an essentially biological method.
38. A Beta vulgaris plant, in particular sugar beet plant, or plant part or seed thereof comprising an endogenous allele encoding an epsp synthase having at position 179 an amino acid different from proline.
39. The Beta vulgaris plant, in particular sugar beet plant, of any of statements 36 to 38, wherein all endogenous alleles encoding epsp synthases have at position 179 an amino acid different from proline.
40. The Beta vulgaris plant, in particular sugar beet plant, of any of statements 36 to 39, wherein the endogenous allele(s) encoding (an) epsp synthase(s) are as defined in any of statements 5 to 10.
41. The Beta vulgaris plant, in particular sugar beet plant, of any of statements 36 to 40, further comprising an endogenous allele encoding an acetolactate synthase having at position 569 an amino acid different from tryptophan.
42. The Beta vulgaris plant, in particular sugar beet plant, of statement 41, wherein all endogenous alleles encoding acetolactate synthases have at position 569 an amino acid different from tryptophan.
43. The Beta vulgaris plant, in particular sugar beet plant, of any of statements 41 to 42, wherein the endogenous allele(s) encoding (an) acetolactate synthase(s) are as defined in any of statements 13 to 14, or 21 to 26.
44. The Beta vulgaris plant, such as sugar beet plant, or plant part of any of statements 36 to 43, wherein said plant part is a root beet, seed, cell, or tissue.
45. A method for producing sugar, comprising: a) providing the root beet of the sugar beet plant of any of statements 36 to 44, b) extracting sugar from said root beet.
46. Use of the sugar beet plant of plant part of any of statements 36 to 44 in a method of sugar production, anaerobic digestion, or fermentation.
47. Use of the sugar beet plant of plant part of any of statements 36 to 44 in a method of sugar, biogas or biofuel production.
48. A method comprising identifying in a Beta vulgaris plant, in particular a sugar beet plant, or plant part an endogenous allele encoding an epsp synthase having at position 179 an amino acid different from proline and/or having at position 175 an amino acid different from threonine.

A plant of the species Beta vulgaris is, in particular, a plant of the subspecies Beta vulgaris subsp. vulgaris. For example, numbering among these are Beta vulgaris subsp. vulgaris var. altissima (sugar beet in a narrower sense), Beta vulgaris ssp. vulgaris var. vulgaris (chard), Beta vulgaris ssp. vulgaris var. conditiva (beetroot / red beet), Beta vulgaris ssp. vulgaris var. crassa/alba (fodder beet). In a preferred embodiment, Beta vulgaris as referred to herein is Beta vulgaris subsp. Vulgaris, more preferably Beta vulgaris subsp. vulgaris var. altissima (i.e. sugar beet).

The cultivated sugar beet is a biennial plant which forms a storage root and a leaf rosette in the first year. Shoot elongation (bolting) and flower formation starts after a period of low temperature, whereas many wild beets of the genus B. vulgaris ssp. maritima show an annual growing habit due to the presence of the bolting gene B at the B locus. The BOLTING gene (B gene) is responsible for the determination of the annual habit in sugar beet. Annuality in the Beta species is considered a monogenic and dominant trait. Plants carrying the dominant B allele are able to switch from juvenile to reproductive stages in a vernalization-independent manner, contrary to biennial plants carrying the b allele that obligatory require vernalization for bolting and subsequent flowering to occur. The dominant allele of locus B is abundant in wild beets and causes bolting under long days without the cold requirement usually essential for biennial cultivars carrying the recessive allele. "B gene" as used herein refers to a gene that is responsible for the determination of the annual habit (early bolting) in Beta vulgaris, such as sugar beet. Plants carrying the dominant allele B are able to switch from juvenile to reproductive stages in a vernalization-independent manner, i.e. make shoot elongation followed by flowering without prior exposure to cold temperatures.

In certain embodiments, the methods for controlling bolters, weed beets, or annual beets as described herein relate to methods for controlling bolters, weed beets, or annual beets in Beta vulgaris growing areas, preferably Beta vulgaris subsp. vulgaris growing areas, in particular Beta vulgaris subsp. vulgaris var. altissima growing areas. In certain embodiments, the methods for controlling bolters, weed beets, or annual beets as described herein relate to methods for controlling bolters, weed beets, or annual beets in biennial Beta vulgaris growing areas, preferably biennial Beta vulgaris subsp. vulgaris growing areas, in particular biennial Beta vulgaris subsp. vulgaris var. altissima growing areas.

In certain embodiments, the uses as described herein relate to uses in Beta vulgaris growing areas, preferably Beta vulgaris subsp. vulgaris growing areas, in particular Beta vulgaris subsp. vulgaris var. altissima growing areas. In certain embodiments, the uses as described herein relate to uses in biennial Beta vulgaris growing areas, preferably biennial Beta vulgaris subsp. vulgaris growing areas, in particular biennial Beta vulgaris subsp. vulgaris var. altissima growing areas.

A "biennial" or "biannual" Beta vulgaris refers to a Beta vulgaris plant that takes two years to complete its biological lifecycle. An "annual" Beta vulgaris refers to a Beta vulgaris plant that germinates, flowers, and dies in one year. An "annual Beta vulgaris" refers to a Beta vulgaris plant containing the dominant allele B at the B locus in a heterozygous or homozygous state. A "biennial Beta vulgaris" refers to a Beta vulgaris plant containing the recessive allele b at the B locus in a homozygous state

"Bolting" refers to the transition from the vegetative rosette stage to the inflorescence or reproductive growth stage, in particular shoot formation. Bolting (stem elongation) is the first step clearly visible in the transition from vegetative to reproductive growth. Bolting can be characterized by an (unwanted) emergence of shoots during the first year of growing, which is disadvantageously in harvesting and processing, but also reduces crop yield. Indeed, bolting and flowering of Beta vulgaris plants is undesirable, since in the case of for instance sugar beets it is not the seeds or fruits, but rather the underground part of the plant, the storage root, that is used, and the energy stored in the root would be consumed during the bolting and flowering of the plant.

As used herein, the term "bolters" refers to Beta vulgaris plants that bolt during the growing season, in particular the same year as the Beta vulgaris plants are planted or sown, preferably before the time the beets are or need to be harvested. In certain embodiments, the bolters are annual Beta vulgaris plants. In certain embodiments, the bolters are weed beets. In certain embodiments, the bolters are sea beets (i.e. Beta vulgaris subsp. maritima). In certain embodiments, the bolters are not Beta vulgaris subsp. vulgaris. In certain embodiments, the bolters are not Beta vulgaris subsp. vulgaris var. altissima. In certain embodiments, the bolters comprise the dominant bolting gene (B gene). As used herein, the term "weed beets" refers to unwanted beet plants, as opposed to the intended cultivated beet plants in the beet growing areas. Weed beets typically are wild beets. Weed beets are preferably annual beets, optionally Beta vulgaris subsp. maritima.

As used herein, "controlling" in the context of controlling bolters or unwanted plants or vegetation etc. includes inhibiting or preventing the growth of bolters, weed beets, or annual beets or unwanted plants or inhibiting bolting of weed beets or annual beets, or at least inhibiting seed production of weed beets or annual beets. "Controlling" may also include killing bolters, weed beets or annual beets, or unwanted plants, preferably before bolting occurs, or at least before seed production of the bolters, weed beets, or annual beets. "Controlling" may also include reducing the amount of bolters, weed beets, or annual beets, or unwanted plants in beet growing areas, preferably before bolting occurs, or at least before seed production of the bolters, weed beets, or annual beets. Controlling bolters or unwanted plants etc. in certain embodiments refers to a reduction of at least 50% of the amount of bolters or unwanted plants etc. or a reduction of at least 50% of the biomass of bolters or unwanted plants etc., such as preferably at least 60%, more preferably at least 70%, such as at least 80% or at least 90%.

As used herein, "unwanted plants" or "unwanted vegetation" are to be understood as meaning all plants which grow in locations where they are unwanted. This can, for example, be harmful plants (for example monocotyledonous or dicotyledonous weeds or unwanted crop plants).

As used herein "Beta vulgaris growing areas" refers to agricultural areas where Beta vulgaris plants are cultivated (i.e. deliberately planted or sown), with the aim of harvesting, such as beet root harvesting or seed harvesting.

The methods and uses according to the invention as described herein, in certain aspects may be for increasing the yield of Beta vulgaris plants or plant parts (i.e. the cultivated Beta vulgaris plants, as opposed to for instance the weed beets). An increased yield may for instance be an increased amount of (cultivated) Beta vulgaris or an increased biomass of (cultivated) Beta vulgaris, such as increase amount of biomass of harvested or harvestable plant parts, such as the beet root. An increased yield may also be for instance in the case of sugar beets an overall increase sugar amount or content (e.g. an increased sugar yield per hectare).

As used herein, the term "growing season" generally refers to the time period between planting or sowing the Beta vulgaris plants or seeds and harvesting the Beta vulgaris plants, in particular the beet roots. Usually, the growing season is from March/April to September/October/November in the northern hemisphere. The skilled person will understand however, that the growing season may be longer or shorter depending on for instance climate or weather conditions or geological conditions or geographical location. It will be further understood that the growing season may shift, such as for instance in the production of winter beets or spring beets.

As used herein unless clearly indicated otherwise, the term "plant" intended to mean a plant at any developmental stage.

It is preferred that the Beta vulgaris plant of the present invention is orthoploid or anorthoploid. An orthoploid plant may preferably be haploid, diploid, tetraploid, hexaploid, octaploid, decaploid or dodecaploid, while an anorthoploid plant may preferably be triploid or pentaploid. In certain preferred embodiments, the Beta vulgaris plant according to the invention is diploid.

The term "plant" according to the present invention includes whole plants or parts of such a whole plant. Whole plants preferably are seed plants, or a crop. "Parts of a plant" are e.g. shoot vegetative organs/structures, e.g., leaves, stems and tubers; roots, flowers and floral organs/structures, e.g. bracts, sepals, petals, stamens, carpels, anthers and ovules; seed, including embryo, endosperm, and seed coat; fruit and the mature ovary; plant tissue, e.g. vascular tissue, ground tissue, and the like; and cells, e.g. guard cells, egg cells, pollen, trichomes and the like; and progeny of the same. Parts of plants may be attached to or separate from a whole intact plant. Such parts of a plant include, but are not limited to, organs, tissues, and cells of a plant, and preferably seeds. A "plant cell" is a structural and physiological unit of a plant, comprising a protoplast and a cell wall. The plant cell may be in form of an isolated single cell or a cultured cell, or as a part of higher organized unit such as, for example, plant tissue, a plant organ, or a whole plant. "Plant cell culture" means cultures of plant units such as, for example, protoplasts, cell culture cells, cells in plant tissues, pollen, pollen tubes, ovules, embryo sacs, zygotes and embryos at various stages of development. "Plant material" refers to leaves, stems, roots, flowers or flower parts, fruits, pollen, egg cells, zygotes, seeds, cuttings, cell or tissue cultures, or any other part or product of a plant. This also includes callus or callus tissue as well as extracts (such as extracts from taproots/root beets) or samples. A "plant organ" is a distinct and visibly structured and differentiated part of a plant such as a root, stem, leaf, flower bud, or embryo. "Plant tissue" as used herein means a group of plant cells organized into a structural and functional unit. Any tissue of a plant in planta or in culture is included. This term includes, but is not limited to, whole plants, plant organs, plant seeds, tissue culture and any groups of plant cells organized into structural and/or functional units. The use of this term in conjunction with, or in the absence of, any specific type of plant tissue as listed above or otherwise embraced by this definition is not intended to be exclusive of any other type of plant tissue. In certain preferred embodiments, the plant parts or plant organs as referred to herein are root beet (or rootbeet) or seed. The term root beet (or beetroot) refers to the taproot or hypocotyl or the beet which has been transformed into a fleshy storage organ.

Accordingly, the Beta vulgaris plant of the present invention is preferably non-transgenic with regard to the epsp synthase gene (and/or with regard to the ALS gene), as the epsp synthase gene is an endogenous gene. Of course, foreign genes can be transferred to the plant either by genetic engineering, by mutagenesis or by conventional methods such as crossing. Said genes can be genes conferring herbicide tolerances, preferably conferring herbicide tolerances different from glyphosate or ALS inhibitor herbicide tolerances, genes improving yield, genes improving resistances to biological organisms (fungi, bacteria or viruses), genes improving tolerance to abiotic stress like drought, frost, heat etc. and/or genes concerning content or ingredient modifications.

The term "transgenic" here means genetically modified by the introduction of a non-endogenous nucleic acid sequence. Typically, a species-specific nucleic acid sequence is introduced in a form, arrangement or quantity into the cell in a location where the nucleic acid sequence does not occur naturally in the cell. While the Beta vulgaris plants according to the invention are preferably non-transgenic with respect to the mutated epsp synthase (or with respect to the mutated ALS), it will be understood that such Beta vulgaris plants may be transgenic for other traits.

Glyphosate is a unique herbicide, because it is the only herbicide known to inhibit synthesis of the aromatic amino acids phenylalanine, tyrosine, and tryptophan. Plants that cannot synthesize these three amino acids are not vital. The affected enzyme of the biosynthetic pathway leading towards aromatic amino acids is 5-enolpyruvylshikimate-3-phosphate synthase (EPSPS), which catalyzes the reaction of shikimate-3-phosphate (S3P) and phosphoenolpyruvate (PEP) to form 5-enolpyruvylshikimate-3-phosphate (EPSP). Glyphosate shares structural similarities to PEP, binds to EPSPS and inhibits the enzyme's reaction in a competitive manner. Glyphosate is the only known herbicide acting on EPSPS (Schonbrunn E, Eschenburg S, Shuttleworth WA, Schloss JV, Amrhein N, Evans JN, Kabsch W: Interaction of the herbicide glyphosate with its target enzyme 5-enolpyruvylshikimate 3-phosphate synthase in atomic detail. Proc Natl Acad Sci U S A 2001, 98(4):1376-1380.; Pollegioni L, Schonbrunn E, Siehl D: Molecular basis of glyphosate resistance-different approaches through protein engineering. FEBS J 2011, 278(16):2753-2766.). Inhibition of synthesis of aromatic amino acids causes more or less immediate stop of growth and eventually kills plants within days after application. Therefore, glyphosate is generally a non-selective herbicide and will severely injure or kill any living plant tissue that it comes in contact with. However, it can be used selectively in glyphosate-resistant crops, including sugar beet, corn, soybean, cotton, and canola.

In certain embodiments, the wild type Beta vulgaris epsp synthase has an amino acid sequence as provided in NCBI reference sequence XP_010692222.1 (SEQ ID NO: 20). In certain embodiments, the wild type or native Beta vulgaris epsp synthase has an amino acid sequence having at least 90%, preferably at least 95%, more preferably at least 98%, such as at least 99% sequence identity, preferably over the entire length, to the sequence of NCBI reference sequence XP_010692222.1, and preferably has epsp synthase activity, with the proviso that amino acid residue at position 179 is proline, and optionally that the amino acid residue at position 175 is threonine.

In certain embodiments, the wild type Beta vulgaris epsp synthase gene has a sequence encoding an amino acid sequence as provided in NCBI reference sequence XP_010692222.1. In certain embodiments, the wild type or native Beta vulgaris epsp synthase gene has a sequence encoding an amino acid sequence having at least 90%, preferably at least 95%, more preferably at least 98%, such as at least 99% sequence identity, preferably over the entire length, to the sequence of NCBI reference sequence XP_010692222.1, and preferably has epsp synthase activity, with the proviso that amino acid residue at position 179 is proline, and optionally that the amino acid residue at position 175 is threonine.

Preferably, as used herein, where amino acid residue positions are referred to for the epsp synthase, the numbering corresponds to the amino acid positions in reference sequence XP_010692222.1. SEQ ID NO: 3 corresponds to the sequence of XP_010692222.1 having the P179S mutation. SEQ ID NO: 6 corresponds to the sequence of XP_010692222.1 having the P179S mutation and the T175I mutation.

As used herein, the term "epsp synthase activity" refers to the enzymatic activity of epsp synthase. The term "having epsp synthase activity" in the context of variant epsp synthases as described above in certain preferred embodiments refers to an epsp synthase of which the enzymatic activity is unaffected or substantially unaffected compared to wild type or native epsp synthase. In certain embodiments, the enzymatic activity is at least 50% of the wild type epsp synthase activity, preferably at least 60%, more preferably at least 70%, even more preferably at least 80%, most preferably at least 90%, such as at least 95%. Enzymatic activity can be measured by means known in the art.

Roundup Ready plants carry the gene coding for a glyphosate-insensitive form of EPSPS, obtained from Agrobacterium sp. strain CP4 (Funke T, Han H, Healy-Fried ML, Fischer M, Schonbrunn E: Molecular basis for the herbicide resistance of Roundup Ready crops. Proc Natl Acad Sci U S A 2006, 103(35):13010-13015.; Padgette SR, Kolacz KH, Delannay X, Re DB, LaVallee BJ, Tinius CN, Rhodes WK, Otero YI, Barry GF, Eichholtz DA et al: Development, Identification, and Characterization of a Glyphosate-Tolerant Soybean Line. Crop Sci 1995, 35(5):1451-1461.). The Agrobacterium sp. strain CP4, isolated from a waste-fed column at a glyphosate production facility, yielded a glyphosate resistant, kinetically efficient EPSP synthase suitable for the production of transgenic, glyphosate-tolerant crops. Other classes II EPSP synthases have been described since then, typically from Gram-positive bacteria, including pathogenic species such as Streptococcus pneumonia and Staphylococcus aureus. Interestingly, a single residue in the active site (Ala-100, numbering based on the E. coli protein) renders the CP4 EPSP synthase insensitive to glyphosate, whereas a highly conserved glycine residue is found at this position in known natural plant and bacterial enzymes.

As used herein, the term "glyphosate herbicide" refers to a composition comprising glyphosate or a glyphosate derivative, including compounds which can be converted into active glyphosate. Glyphosate is also known as N-phosphonomethylglycine and in its acid form has the structure:

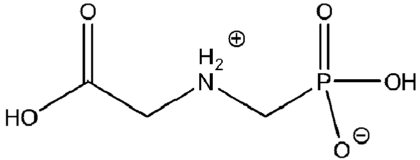

Since glyphosate in its acid form is relatively insoluble in water (1.16% by weight at 25°C), it is typically formulated as a water-soluble salt. It is typically formulated as a monobasic, dibasic, or tribasic salt. Various salts of glyphosate, methods for preparing salts of glyphosate, formulations of glyphosate or its salts and methods of use of glyphosate or its salts for killing and controlling weeds and other plants are disclosed in U.S. Pat. No. 4,507,250, U.S. Pat. No. 4,481,026, U.S. Pat. No. 4,405,531, U.S. Pat. No. 4,315,765, U.S. Pat. No. 4,140,513, U.S. Pat. No. 3,977,860, U.S. Pat. No. 3,853,530, and U.S. Pat. No. 3, 799, 758.

Typical glyphosate salts include, for example, the mono(isopropylammonium) ("IPA"), potassium, sodium, monoethanolammonium ("MEA"), trimethylsulfonium ("TMS"), ammonium, diammonium salts, n-propylamine, ethylamine, ethylenediamine, and hexamethylenediamine salts. The most widely used salt of glyphosate is the IPA salt. Commercial herbicides of Monsanto Company having the IPA salt of glyphosate as active ingredient include Roundup®, Roundup® Ultra, Roundup® Xtra, and Rodeo® herbicides. These are aqueous solution concentrate formulations and are generally diluted in water by the user prior to application to plant foliage. Commercially formulated TMS salt is used, for example, in Touchdown® herbicide of Zeneca (Syngenta), formulated potassium salt is used, for example, in Roundup® PowerMAX, and formulated ammonium salt is used, for example, in Roundup® Max (www.roundup.it/roundup_max.php) as used in the present invention. Glyphosate salts are typically co-formulated with a surfactant to maximize herbicidal efficacy. For example, see WO 96/032839.

In certain embodiments, a glyphosate herbicide as used herein comprises glyphosate or a derivative thereof, wherein said derivative is selected from a salt, ester, amide, or alkylamide. In certain embodiments the glyphosate salt is an alkali metal salt such as (mono-, di-, or tri-) sodium or (mono-, di-, or tri-) potassium. In certain embodiments, the glyphosate salt is an ammonium salt or a di-ammonium salt such as dimethylammonium, an alkylamine salt such as C1-C16 alkylamine salts such as dimethylamine, ethylamine, ethylenediamine, hexamethylenediamine, n-propylamine, and isopropylamine salts, an alkylammonium salt such as C1-C16 alkylammonium salts such as dimethylammonium and isopropylammonium salts such as monoisopropylammonium salt (IPA), an alkanolamine salt such as C1-C16 alkanolamine salts such as (mono-, di-, or tri-) ethanolamine salts such as monoethanolammonium salt (MEA). In certain embodiments, the glyphosate salt is an alkylsulfonium salt such as trimethylsulfonium salts (TMS), a sulfoxonium salt. In certain embodiments, the glyphosate herbicide comprises a mixture or combination of glyphosate or any of its derivatives, in particular salts as described above.

In certain aspects, the glyphosate herbicide is applied in the methods and uses according to the invention as described herein at a dosage sufficient for controlling (e.g. inhibiting growth) bolters, weed beets, or annual beets. In certain embodiments, such dosage is at least 300 g/ha (glyphosate acid equivalent), such as at least 600 g/ha, preferably at a dose of at least 900 g/ha, more preferably at a dose of at least 1000 g/ha, even more preferably at a dose of at least 1100 g/ha, most preferably at a dose of at least 1200 g/ha or a dose of 1200 g/ha. This dosage preferably refers to a single application dose. It will be understood that more than one application may be needed during the growing season, such as two applications or three applications. The dose of such subsequent applications may be the same or may be different than the dose of the first application.

As used herein, the term "glyphosate acid equivalent" refers to that portion of a formulation of glyphosate herbicide that theoretically could be converted back to the corresponding or parent acid, or the theoretical yield of the glyphosate acid from a glyphosate herbicide which has been formulated as a derivative (such as esters, salts, amines, etc.).The glyphosate acid equivalent can be calculated from the ratio of the molecular mass of the glyphosate parent acid (having a molecular mass of 168 in its deprotonated state) and the molecular mass of the formulated glyphosate product, typically a derivative such as a salt). For instance, glyphosate isopropylamine has a molecular mass of 228, which has a fraction of 168/228=0.7368 glyphosate parent acid (deprotonated). Multiplying the concentration or amount of glyphosate isopropylamine by the fraction 0.7368 results in the concentration or amount of glyphosate acid equivalent. Accordingly, for instance 5 kg glyphosate isopropylamine has a glyphosate acid equivalent of 3.684 kg.

In certain aspects, the invention relates to Beta vulgaris plants which tolerate glyphosate (and optionally ALS inhibitors), in particular in doses sufficiently high to effect optimal herbicidal activity. In certain embodiments, the Beta vulgaris plants as described herein tolerate glyphosate (e.g. glyphosate acid equivalent) at a dose of at least 300 g/ha, such as at least 600 g/ha, preferably at a dose of at least 900 g/ha, more preferably at a dose of at least 1000 g/ha, even more preferably at a dose of at least 1100 g/ha, most preferably at a dose of at least 1200 g/ha or a dose of 1200 g/ha. In certain embodiments, the Beta vulgaris plants as described herein tolerate an ALS inhibitor dose equivalent to the mixtures of 35 g/ha foramsulfuron and 7 g/ha iodosulfuron-methyl-sodium glyphosate. Preferably, said dose is a single application dose. It will be understood that if multiple applications are needed during the growing season, the Beta vulgaris plant according to the invention are preferably tolerant to said multiple applications. Preferably, the glyphosate (and optionally ALS inhibitor) tolerant Beta vulgaris plant according to the invention has no disadvantages with respect to other important agronomic properties such as growth, yield, quality, pathogen resistance, physiological functions, etc.

Glyphosate (or ALS inhibitor) tolerance can for instance be determined by visual injury ratings for plant vigour and plant chlorosis based on a scale from 0 (dead plant) to 9 (completely unaffected plant), such as for instance ratings taken on individual plants 2 weeks after glyphosate application. Ratings of 0 to 3 are characteristic of susceptible plants. Ratings of 3 to 7 indicate a low to intermediate level of tolerance, and ratings of 8 or 9 indicate good levels of tolerance. In particular the ratings have the following meaning: 9. Unaffected plant identical to untreated control; 8. Only very small necrosis on the tips of the leaves with less than 5% of the leaf area affected and yellow; 7. Very small necrosis on the tips of the leaves which start to curl; less than 5% of the leaf area are affected and yellow; 6,5,4. Increasing necrosis and leaf curl; leaves are becoming smaller than normal; 3,2. No or very limited leaf growth; all leaves are curled and affected by necrosis; 1. No growth of the plant; up to 5% of the plant stay green; 0. Dead plant. In certain preferred embodiments, the Beta vulgaris plants according to the invention have a rating of at least 3, preferably at least 7, more preferably at least 8, even more preferably 9.

In certain embodiments, the Beta vulgaris plants according to the invention are less sensitive to glyphosate (or a glyphosate herbicide), and optionally an ALS inhibitor herbicide, than the corresponding wild type Beta vulgaris plants or bolters, weed beets, annual beets as described herein elsewhere. In certain embodiments, the Beta vulgaris plant according to the invention are at least 10 times less sensitive, such as 100 times less sensitive, more preferably, 500 times, even more preferably 1000 times and most preferably less than 2000 times. Less sensitive when used herein may, vice versa, be seen as "more tolerable" or "more resistant". Similarly, "more tolerable" or "more resistant" may, vice versa, be seen as "less sensitive".

In certain embodiments, the Beta vulgaris plants according to the invention have an epsp synthase of which the enzymatic activity is unaffected or substantially unaffected by glyphosate. In certain embodiments, the Beta vulgaris plants according to the invention have an epsp synthase of which the enzymatic activity is at most 50% less in the presence of glyphosate compared to the absence of glyphosate, preferably at most 40% less, more, preferably at most 30% less, even more preferably at most 20 less, most preferably at most 10% less, such as at most 5% less. Enzymatic activity can be measured by means known in the art. Enzymatic activity is preferably determined in the presence of glyphosate (or a glyphosate herbicide) at a relevant applicable herbicidal dose, such as a dose corresponding to a field application of at least 300 g/ha, such as at least 600 g/ha, preferably at a dose of at least 900 g/ha, more preferably at a dose of at least 1000 g/ha, even more preferably at a dose of at least 1100 g/ha, most preferably at a dose of at least 1200 g/ha or a dose of 1200 g/ha.

As used herein, ALS (acetolactate synthase; also known as AHAS (acetohydroxyacid synthase); EC 2.2.1.6; formerly EC 4.1.3.18)) is involved in the conversion of two pyruvate molecules to an acetolactate molecule and carbon dioxide. The reaction uses thyamine pyrophosphate in order to link the two pyruvate molecules. The resulting product of this reaction, acetolactate, eventually becomes valine, leucine and isoleucine (Singh (1999) "Biosynthesis of valine, leucine and isoleucine", in Plant Amino Acids, Singh, B.K., ed., Marcel Dekker Inc. New York, New York, pp. 227-247). Inhibitors of the ALS interrupt the biosynthesis of valine, leucine and isoleucine in plants. The consequence is an immediate depletion of the respective amino acid pools causing a stop of protein biosynthesis leading to a cessation of plant growth and eventually the plant dies, or - at least - is damaged.

In certain embodiments, the wild type Beta vulgaris ALS has an amino acid sequence as provided in NCBI reference sequence XP_010695365.1 (SEQ ID NO: 21). In certain embodiments, the wild type or native Beta vulgaris ALS has an amino acid sequence having at least 90%, preferably at least 95%, more preferably at least 98%, such as at least 99% sequence identity, preferably over the entire length, to the sequence of NCBI reference sequence XP_010695365.1, and preferably has ALS activity, with the proviso that amino acid residue at position 569 is tryptophan, and optionally that the amino acid residue at position 188 is proline.

In certain embodiments, the wild type Beta vulgaris ALS gene has a sequence encoding an amino acid sequence as provided in NCBI reference sequence XP_010695365.1. In certain embodiments, the wild type or native Beta vulgaris ALS gene has a sequence encoding an amino acid sequence having at least 90%, preferably at least 95%, more preferably at least 98%, such as at least 99% sequence identity, preferably over the entire length, to the sequence of NCBI reference sequence XP_010695365.1, and preferably has epsp synthase activity, with the proviso that amino acid residue at position 569 is tryptophan, and optionally that the amino acid residue at position 188 is proline.

Preferably, as used herein, where amino acid residue positions are referred to for the ALS, the numbering corresponds to the amino acid positions in reference sequence XP_010695365.1. SEQ ID NO: 9 corresponds to the sequence of XP_010695365.1 having the W569L mutation. SEQ ID NO: 12 corresponds to the sequence of XP_010695365.1 having the W569L mutation and the P188S mutation.

Herbicidal compounds belonging to the class of ALS inhibitors, which can be used in certain embodiments of the invention include (a) sulfonylurea herbicides (Beyer E.M et al. (1988), Sulfonylureas in Herbicides: Chemistry, Degradation, and Mode of Action; Marcel Dekker, New York, 1988, 117-189), (b) sulfonylaminocarbonyltriazolinone herbicides (Pontzen, R., Pflanz.- Nachrichten Bayer, 2002, 55, 37-52), (c) imidazolinone herbicides (Shaner, D.L., et al., Plant Physiol., 1984, 76, 545-546; Shaner, D.L., and O'Connor, S.L. (Eds.) The Imidazolinone Herbicides, CRC Press, Boca Rato, FL, 1991), (d) triazolopyrimidine herbicides (Kleschick, W.A. et al., Agric. FoodChem, 1992, 40, 1083-1085), and (e) pyrimidinyl(thio)benzoate herbicides (Shimizu, T.J., Pestic. Sci.,1997, 22, 245-256; Shimizu, T. et al., Acetolactate Syntehase Inhibitors in Herbicide Classes in Development, Boger, P., Wakabayashi. K., Hirai, K., (Eds.), Springer Verlag, Berlin, 2002, 1-41).

In certain embodiments, the ALS inhibitor is selected from sulfonylurea, sulfonylaminocarbonyltriazolinone, triazolopyrimidine, sulfonanilide, imidazolinone, pyrimidinyloxybenzoeacid, pyrimidinylthiobenzoeacid. Further ALS inhibitors which may be used in certain aspects of the invention are described for instance in WO 2014/090760, WO 2012/049268, WO 2012/049266, EP 2 627 183, and WO 2014/091021, each of which incorporated herein by reference in their entirety.

In certain embodiments, the ALS inhibitor is selected from the ALS inhibitors listed in claims 2 to 4 of WO/2012049266, all of which are explicitly incorporated herein by reference.

In certain embodiments, suitable mutated ALS conferring resistance to ALS inhibitors are as described in EP 2 931 902 and WO 2012/049268, which are incorporated herein in their entirety by reference.

In certain embodiments, non-glyphosate or non-ALS inhibitor herbicides may be applied in combination with the glyphosate and/or ALS-inhibitor herbicides. In certain embodiments, the application of the respective herbicides (i) takes place jointly or simultaneously, or (ii) takes place at different times and/or in a plurality of portions (sequential application), in pre-emergence applications followed by post-emergence applications or early post-emergence applications followed by medium or late post-emergence applications. In certain embodiments, the herbicides are selected from chloridazon, clethodim, clodinafop, clodinafop-propargyl, clopyralid, cycloxydim, desmedipham, dimethenamid, dimethenamid-P, ethofumesate, fenoxaprop, fenoxaprop-P, fenoxaprop-ethyl, fenoxaprop-P-ethyl, fluazifop, fluazifop-P, fluazifop-butyl, fluazifop-P-butyl, glufosinate, glufosinate-ammonium, glufosinate-P, glufosinate-P-ammonium, glufosinate-P-sodium, haloxyfop, haloxyfop-P, haloxyfop-ethoxyethyl, haloxyfop-P-ethoxyethyl, haloxyfop-methyl, haloxyfop-P-methyl, lenacil, metamitron, phenmedipham, phenmedipham-ethyl, propaquizafop, quinmerac, quizalofop, quizalofop-ethyl, quizalofop-P, quizalofop-P-ethyl, quizalofop-P-tefuryl, sethoxydim.

In certain aspects, the invention relates to a vacuole of a cell of the Beta vulgaris plant of the invention, and the content substances stored therein. Furthermore, the invention also relates to the cell extract from a cell - preferably, from a cell of the Beta vulgaris plant of the invention, and preferably from a cell of one of the following crops: sugar beet, chard, or beetroot. No plant can be regenerated from the cell extract. Likewise encompassed by the invention is a plant genome containing the nucleic acid according to the invention. No plant can be regenerated from the plant genome. The sugar concentration from the cell extract may thereby be increased relative to a cell that is not a cell according to the invention, but that belongs to the same species or crop. This applies, in particular under the conditions when Glyphosate herbicide is applied. Also encompassed by the invention is the use of the cell extract for the production of sugar (saccharose) or for the production of juice - preferably, beetroot juice.

An additional aspect of the invention is a seed stock comprising seeds of the Beta vulgaris plant of the invention. The seed stock and the seeds may be technically treated. The invention thus also comprises technically-treated seed stock and technically-treated seeds. The various embodiments of technically-treated seed stock are explained in detail in the following whereby the term seed stock also includes seeds: Technically-treated seed stock may be present in polished form. The outermost layer of the seed is thereby removed, so that the seed assumes a more rounded form. This is helpful in sowing, where an optimally uniform shape leads to a uniform distribution of the seed stock grains by sowing machines. Technically-treated seed stock furthermore encompasses pelleted seed stock. The seed stock is thereby embedded in a pelleting mass that protects the seed stock contained therein and leads to a larger mass, such that the pelleted seed stock shows a greater resistance capability with regard to wind drift and is thus less susceptible to being blown away by the wind, and, at the same time, a more precise positioning during sowing is enabled. In a preferred embodiment of the invention, all pelleted seed stock grains of a batch or unit designated for sale have essentially the same shape and the same mass. Deviations of 5% in diameter and mass are possible. However, the deviations preferably do not exceed 1%. As one of the main components, the pelleting mass may contain for example a mineral compound such as clay, bentonite, kaolin, humus and/or peat, for example. It is possible to add an adhesive material like polyacylamide. Additional possible components are cited in US 4,067,141. Moreover, the pelleting mass may contain additional chemical agents that positively influence the cultivation in practice. These may here be substances that are counted among fertilizing agents. These include compounds rich of one or more of the following elements: Nitrogen, Phosphorus and Potassium (macronutrients). Therefore, the fertilizing ingredients may contain for example Nitrate nitrogen, Ammonium nitrogen, Magnesium Nitrate, Calcium Ammonium Nitrate, Mono Ammonium Phosphate, Mono Potassium Phosphate and Potassium Nitrate. Furthermore, pelleting mass may contain fungicides, insecticides, and/or antifeedants. The fungicides may be thiram and/or hymexazol and/or other fungicides. The insecticide may be a substance from the neonicotinoid group. The substance from the neonicotinoid group is preferably imidacloprid (ATC Code: QP53AX17) and/or clothianidin (CAS number 210880-92-5). Furthermore, the insecticide may also be cyfluthrin (CAS number 68359-37-5), beta-cyfluthrin or tefluthrin. It is worth mentioned that the compound included in the dressing or pelleting mass are taken up by the plant and show systemic effect thereby providing suitable protection of the whole plant. Plants resulting from pelleted seed including one or more pesticides therefore differ from naturally occurring plants and show better performance under biotic stress conditions. In this context the invention also encompasses a mixture of a pelleting mass and a seed according to the invention. Furthermore, the invention also encompasses a method for producing a pelleted seed according to the invention comprising the following steps: a) providing a seed of the Beta vulgaris plant of the invention, b) embedding the sugar beet plant seed in a pelleting mass, and c) allowing the pelleting mass to dry, wherein the seed may be optionally a primed or pregerminated seed or the seed may be allowed to be primed during step b).

The pelleted seed stock is a specific embodiment of dressed seed stock. In this context technically-treated seed stock encompasses also the dressed seed stock. However, the invention is not limited to pelleted seed stock, but, rather, may be applied with any form of dressed seed stock. The invention thus also relates to dressed seed stock, which includes pelleted seed stock, but is not limited to this. Dry dressing, wet dressing, and suspension dressing are thus also encompassed. The dressing may thereby also contain at least one dye (coloring), such that the dressed seed stock may be quickly differentiated from undressed seed stock, and, furthermore, good visibility in the environment is ensured after sowing. The dressing may also contain those agrochemicals which are described in the context of the pilling mass. The invention includes thus such dressed seed stock whereby the dressing contains at least one anti-feedant such as an insecticide and / or at least one fungicide. Optionally, so called electonical dressing (dressing by application of electric energy) may be applied. However, electronic dressing is not a dressing in the strict sense of the word.

An additional form of technically-treated seed stock is encrusted seed stock. What is known as coating is also spoken of in this context as well as of seed stock treated with a coating. The difference to pelleted seed stock is that the seed grains retain their original shape, wherein this method is especially economical. The method is described in EP 0 334 258 A1, for example. An additional form of technically-treated seed stock is sprouted or primed seed stock. Sprouted seed stock is pretreated via a pre-germination, whereas primed seed stock has been pretreated via a priming ("germination"). Pre-germinated and primed seed stock have the advantage of a shorter emergence time. The point in time of the emergence after sowing is, at the same time, more strongly synchronized. This enables better agrotechnical processing during cultivation and especially during the harvest, and, additionally, increases the yield quantity. In pre-germination, the seed stock is germinated until the radicle exits the seed stock shell, and the process is subsequently stopped. In the priming, the process is stopped before the radicle exits the seed stock shell. Compared to pre-germinated seed stock, seed stock that has been subjected to a priming is insensitive to the stress of a re-drying and, after such a re-drying, has a longer storage life in comparison to pre-germinated seed stock, for which a re-drying is generally not advised. In this context, technically pre-treated seed stock also includes primed and re-dried seed stock. The process of pre-germination is explained in US 4,905,411 A. Various embodiments of priming are explained in EP 0 686 340 A1. In addition to this, it is also possible to simultaneously pill and prime seed stock in one process. This method is described in EP 2 002 702 B1. Primed seed stock which is moreover pelleted, is encompassed by the present invention.

In addition to this, the invention also encompasses a mixture containing the seed stock according to the invention or the seeds according to the invention, and a dressing mass as defined above. The dressing mass is thereby preferably embodied as a pelleting mass, as defined above.

With storage of seed stock according to the invention, storage conditions are preferably to be chosen that do not negatively affect the stability or storage life of the seed stock. Fluctuations in humidity may, especially, have a disadvantageous effect here. Part of the invention is a method for the storage of the seed stock in a bag or container that is via simultaneously water-repellent and breathable. Such a bag or container may be designed as a carton or packing. Such a carton or packing may optionally possess an inner vapor barrier. If the carton or packing is designed as a duplex carton, its stability increases. A container, bag, carton or packing comprising the seed stock according to the invention, or technically-treated seed stock according to the invention, is likewise a part of the invention. It is likewise part of the invention to store seed stock according to the invention or technically-treated seed stock according to the invention in such a bag, container, packing or carton.

The term "sequence" when used herein relates to nucleotide sequence(s), polynucleotide(s), nucleic acid sequence(s), nucleic acid(s), nucleic acid molecule, peptides, polypeptides and proteins, depending on the context in which the term "sequence" is used. The terms "nucleotide sequence(s)", "polynucleotide(s)", "nucleic acid sequence(s)", "nucleic acid(s)", "nucleic acid molecule" are used interchangeably herein and refer to nucleotides, either ribonucleotides or deoxyribonucleotides or a combination of both, in a polymeric unbranched form of any length. Nucleic acid sequences include DNA, cDNA, genomic DNA, RNA, synthetic forms and mixed polymers, both sense and antisense strands, or may contain non-natural or derivatized nucleotide bases, as will be readily appreciated by those skilled in the art.

An "isolated nucleic acid" is understood to be a nucleic acid isolated from its natural or original environment. The term also includes a synthetic manufactured nucleic acid.

When used herein, the term "polypeptide" or "protein" (both terms are used interchangeably herein) means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The term polypeptide also refers to, and does not exclude, modifications of the polypeptide, e.g., glycosylation, acetylation, phosphorylation and the like. Such modifications are well described in basic texts and in more detailed monographs, as well as in the research literature.

Amino acid substitutions encompass amino acid alterations in which an amino acid is replaced with a different naturally-occurring amino acid residue. Such substitutions may be classified as "conservative", in which an amino acid residue contained in the wild-type protein is replaced with another naturally-occurring amino acid of similar character, for example Gly↔Ala, Val↔Ille↔Leu, Asp↔Glu, Lys↔Arg, Asn↔Gln or Phe↔Trp↔Tyr. Substitutions encompassed by the present invention may also be "non-conservative", in which an amino acid residue which is present in the wild-type protein is substituted with an amino acid with different properties, such as a naturally-occurring amino acid from a different group (e.g. substituting a charged or hydrophobic amino acid with alanine. "Similar amino acids", as used herein, refers to amino acids that have similar amino acid side chains, i.e. amino acids that have polar, non-polar or practically neutral side chains. "Non-similar amino acids", as used herein, refers to amino acids that have different amino acid side chains, for example an amino acid with a polar side chain is non-similar to an amino acid with a non-polar side chain. Polar side chains usually tend to be present on the surface of a protein where they can interact with the aqueous environment found in cells ("hydrophilic" amino acids). On the other hand, "non-polar" amino acids tend to reside within the center of the protein where they can interact with similar non-polar neighbours ("hydrophobic" amino acids"). Examples of amino acids that have polar side chains are arginine, asparagine, aspartate, cysteine, glutamine, glutamate, histidine, lysine, serine, and threonine (all hydrophilic, except for cysteine which is hydrophobic). Examples of amino acids that have non-polar side chains are alanine, glycine, isoleucine, leucine, methionine, phenylalanine, proline, and tryptophan (all hydrophobic, except for glycine which is neutral).

The term "gene" when used herein refers to a polymeric form of nucleotides of any length, either ribonucleotides or desoxyribonucleotides. The term includes double- and single-stranded DNA and RNA. It also includes known types of modifications, for example, methylation, "caps", substitutions of one or more of the naturally occurring nucleotides with an analog. Preferably, a gene comprises a coding sequence encoding the herein defined polypeptide. A "coding sequence" is a nucleotide sequence which is transcribed into mRNA and/or translated into a polypeptide when placed or being under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

A used herein, the term "endogenous" refers to a gene or allele which is present in its natural genomic location. The term "endogenous" can be used interchangeably with "native". This does not however exclude the presence of one or more nucleic acid differences with the wild-type allele. In particular embodiments, the difference with a wild-type allele can be limited to less than 9 preferably less than 6, more particularly less than 3 nucleotide differences. More particularly, the difference with the wildtype sequence can be in only one nucleotide. Preferably, the endogenous allele encodes a modified protein having less than 9, preferably less than 6, more particularly less than 3 and even more preferably only one amino acid difference with the wild-type protein.

As used herein, the term "homozygote" refers to an individual cell or plant having the same alleles at one or more or all loci. When the term is used with reference to a specific locus or gene, it means at least that locus or gene has the same alleles. As used herein, the term "homozygous" means a genetic condition existing when identical alleles reside at corresponding loci on homologous chromosomes. As used herein, the term "heterozygote" refers to an individual cell or plant having different alleles at one or more or all loci. When the term is used with reference to a specific locus or gene, it means at least that locus or gene has different alleles. As used herein, the term "heterozygous" means a genetic condition existing when different alleles reside at corresponding loci on homologous chromosomes.

As used herein, an "allele" refers to alternative forms of various genetic units associated with different forms of a gene or of any kind of identifiable genetic element, which are alternative in inheritance because they are situated at the same locus in homologous chromosomes. In a diploid cell or organism, the two alleles of a given gene (or marker) typically occupy corresponding loci on a pair of homologous chromosomes.

A "marker" is a (means of finding a position on a) genetic or physical map, or else linkages among markers and trait loci (loci affecting traits). The position that the marker detects may be known via detection of polymorphic alleles and their genetic mapping, or else by hybridization, sequence match or amplification of a sequence that has been physically mapped. A marker can be a DNA marker (detects DNA polymorphisms), a protein (detects variation at an encoded polypeptide), or a simply inherited phenotype (such as the 'waxy' phenotype). A DNA marker can be developed from genomic nucleotide sequence or from expressed nucleotide sequences (e.g., from a spliced RNA or a cDNA). Depending on the DNA marker technology, the marker may consist of complementary primers flanking the locus and/or complementary probes that hybridize to polymorphic alleles at the locus. The term marker locus is the locus (gene, sequence or nucleotide) that the marker detects. "Marker" or "molecular marker" or "marker locus" may also be used to denote a nucleic acid or amino acid sequence that is sufficiently unique to characterize a specific locus on the genome. Any detectable polymorphic trait can be used as a marker so long as it is inherited differentially and exhibits linkage disequilibrium with a phenotypic trait of interest.

Markers that detect genetic polymorphisms between members of a population are well-established in the art. Markers can be defined by the type of polymorphism that they detect and also the marker technology used to detect the polymorphism. Marker types include but are not limited to, e.g., detection of restriction fragment length polymorphisms (RFLP), detection of isozyme markers, randomly amplified polymorphic DNA (RAPD), amplified fragment length polymorphisms (AFLPs), detection of simple sequence repeats (SSRs), detection of amplified variable sequences of the plant genome, detection of self-sustained sequence replication, or detection of single nucleotide polymorphisms (SNPs). SNPs can be detected e.g. via DNA sequencing, PCR-based sequence specific amplification methods, detection of polynucleotide polymorphisms by allele specific hybridization (ASH), dynamic allele-specific hybridization (DASH), molecular beacons, microarray hybridization, oligonucleotide ligase assays, Flap endonucleases, 5' endonucleases, primer extension, single strand conformation polymorphism (SSCP) or temperature gradient gel electrophoresis (TGGE). DNA sequencing, such as the pyrosequencing technology has the advantage of being able to detect a series of linked SNP alleles that constitute a haplotype. Haplotypes tend to be more informative (detect a higher level of polymorphism) than SNPs.

A "marker allele", alternatively an "allele of a marker locus", can refer to one of a plurality of polymorphic nucleotide sequences found at a marker locus in a population. With regard to a SNP marker, allele refers to the specific nucleotide base present at that SNP locus in that individual plant.

As used herein, the term "sequence identity" refers to the degree of identity between any given nucleic acid sequence and a target nucleic acid sequence. Percent sequence identity is calculated by determining the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. Percent sequence identity also can be determined for any amino acid sequence. To determine percent sequence identity, a target nucleic acid or amino acid sequence is compared to the identified nucleic acid or amino acid sequence using the BLAST 2 Sequences (BI2seq) program from the stand-alone version of BLASTZ containing BLASTN and BLASTP. This stand-alone version of BLASTZ can be obtained from Fish & Richardson's web site (World Wide Web at fr.com/blast) or the U.S. government's National Center for Biotechnology Information web site (World Wide Web at ncbi.nlm.nih.gov). Instructions explaining how to use the BI2seq program can be found in the readme file accompanying BLASTZ. BI2seq performs a comparison between two sequences using either the BLASTN or BLASTP algorithm.

BLASTN is used to compare nucleic acid sequences, while BLASTP is used to compare amino acid sequences. To compare two nucleic acid sequences, the options are set as follows: -i is set to a file containing the first nucleic acid sequence to be compared (e.g. , C:\seq I .txt); -j is set to a file containing the second nucleic acid sequence to be compared (e.g. , C:\seq2.txt); -p is set to blastn; -o is set to any desired file name (e.g. , C :\output.txt); -q is set to - 1 ; -r is set to 2; and all other options are left at their default setting. The following command will generate an output file containing a comparison between two sequences: C:\B12seq -i c:\seqI .txt -j c:\seq2.txt -p blastn -o c:\output.txt -q - 1 -r 2. If the target sequence shares homology with any portion of the identified sequence, then the designated output file will present those regions of homology as aligned sequences. If the target sequence does not share homology with any portion of the identified sequence, then the designated output file will not present aligned sequences. Once aligned, a length is determined by counting the number of consecutive nucleotides from the target sequence presented in alignment with the sequence from the identified sequence starting with any matched position and ending with any other matched position. A matched position is any position where an identical nucleotide is presented in both the target and identified sequences. Gaps presented in the target sequence are not counted since gaps are not nucleotides. Likewise, gaps presented in the identified sequence are not counted since target sequence nucleotides are counted, not nucleotides from the identified sequence. The percent identity over a particular length is determined by counting the number of matched positions over that length and dividing that number by the length followed by multiplying the resulting value by 100. For example, if (i) a 500-base nucleic acid target sequence is compared to a subject nucleic acid sequence, (ii) the BI2seq program presents 200 bases from the target sequence aligned with a region of the subject sequence where the first and last bases of that 200-base region are matches, and (iii) the number of matches over those 200 aligned bases is 180, then the 500-base nucleic acid target sequence contains a length of 200 and a sequence identity over that length of 90% (i.e. , 180 / 200 x 100 = 90). It will be appreciated that different regions within a single nucleic acid target sequence that aligns with an identified sequence can each have their own percent identity. It is noted that the percent identity value is rounded to the nearest tenth. For example, 78.11, 78.12, 78.13, and 78.14 are rounded down to 78.1, while 78.15, 78.16, 78.17, 78.18, and 78.19 are rounded up to 78.2. It also is noted that the length value will always be an integer.

An "isolated nucleic acid sequence" or "isolated DNA" refers to a nucleic acid sequence which is no longer in the natural environment from which it was isolated, e.g. the nucleic acid sequence in a bacterial host cell or in the plant nuclear or plastid genome. When referring to a "sequence" herein, it is understood that the molecule having such a sequence is referred to, e.g. the nucleic acid molecule. A "host cell" or a "recombinant host cell" or "transformed cell" are terms referring to a new individual cell (or organism) arising as a result of at least one nucleic acid molecule, having been introduced into said cell. The host cell is preferably a plant cell or a bacterial cell. The host cell may contain the nucleic acid as an extra-chromosomally (episomal) replicating molecule, or comprises the nucleic acid integrated in the nuclear or plastid genome of the host cell, or as introduced chromosome, e.g. minichromosome.

When reference is made to a nucleic acid sequence (e.g. DNA or genomic DNA) having "substantial sequence identity to" a reference sequence or having a sequence identity of at least 80%, e.g. at least 85%, 90%, 95%, 98% or 99% nucleic acid sequence identity to a reference sequence, in one embodiment said nucleotide sequence is considered substantially identical to the given nucleotide sequence and can be identified using stringent hybridisation conditions. In another embodiment, the nucleic acid sequence comprises one or more mutations compared to the given nucleotide sequence but still can be identified using stringent hybridisation conditions. "Stringent hybridisation conditions" can be used to identify nucleotide sequences, which are substantially identical to a given nucleotide sequence. Stringent conditions are sequence dependent and will be different in different circumstances. Generally, stringent conditions are selected to be about 5°C lower than the thermal melting point (Tm) for the specific sequences at a defined ionic strength and pH. The Tm is the temperature (under defined ionic strength and pH) at which 50% of the target sequence hybridises to a perfectly matched probe. Typically, stringent conditions will be chosen in which the salt concentration is about 0.02 molar at pH 7 and the temperature is at least 60°C. Lowering the salt concentration and/or increasing the temperature increases stringency. Stringent conditions for RNA-DNA hybridisations (Northern blots using a probe of e.g. 100 nt) are for example those which include at least one wash in 0.2X SSC at 63°C for 20min, or equivalent conditions. Stringent conditions for DNA-DNA hybridisation (Southern blots using a probe of e.g. 100 nt) are for example those which include at least one wash (usually 2) in 0.2X SSC at a temperature of at least 50°C, usually about 55°C, for 20 min, or equivalent conditions. See also Sambrook et al. (1989) and Sambrook and Russell (2001).

The term "hybridizing" or "hybridization" means a process in which a single-stranded nucleic acid molecule attaches itself to a complementary nucleic acid strand, i.e. agrees with this base pairing. Standard procedures for hybridization are described, for example, in Sambrook et al. (Molecular Cloning. A Laboratory Manual, Cold Spring Harbor Laboratory Press, 3rd edition 2001). Preferably this will be understood to mean an at least 50%, more preferably at least 55%, 60%, 65%, 70%, 75%, 80% or 85%, more preferably 90%, 91%, 92%, 93%, 94%, 95%, 96%, 97%, 98% or 99% of the bases of the nucleic acid strand form base pairs with the complementary nucleic acid strand. The possibility of such binding depends on the stringency of the hybridization conditions. The term "stringency" refers to hybridization conditions. High stringency is if base pairing is more difficult, low stringency, when a base-pairing is facilitated. The stringency of hybridization conditions depends for example on the salt concentration or ionic strength and temperature. Generally, the stringency can be increased by increasing the temperature and / or decreasing salinity. "Stringent hybridization conditions" are defined as conditions in which hybridization occurs predominantly only between homologous nucleic acid molecules. The term "hybridization conditions" refers not only to the actual binding of the nucleic acids at the prevailing conditions, but also in the subsequent washing steps prevailing conditions. Stringent hybridization conditions are, for example, conditions under which predominantly only those nucleic acid molecules having at least 70%, preferably at least 75%, at least 80%, at least 85%, at least 90% or at least 95% sequence identity hybridize. Less stringent hybridization conditions include: hybridization in 4 x SSC at 37 ° C, followed by repeated washing in 1 x SSC at room temperature. Stringent hybridization conditions include: hybridization in 4 x SSC at 65 ° C, followed by repeated washing in 0.1 x SSC at 65 ° C for a total of about 1 hour.

In an aspect, the invention also relates to a method for providing a glyphosate resistant or tolerant Beta vulgaris plant. Such method may involve mutagenesis. In certain embodiments, the nucleic acid modification of the epsp synthase gene is effected by random mutagenesis. Cells or organisms may be exposed to mutagens such as UV radiation or mutagenic chemicals (such as for instance such as ethyl methanesulfonate (EMS)), and mutants with desired characteristics are then selected. Mutants can for instance be identified by TILLING (Targeting Induced Local Lesions in Genomes). The method combines mutagenesis, such as mutagenesis using a chemical mutagen such as ethyl methanesulfonate (EMS) with a sensitive DNA screening-technique that identifies single base mutations/point mutations in a target gene. The TILLING method relies on the formation of DNA heteroduplexes that are formed when multiple alleles are amplified by PCR and are then heated and slowly cooled. A "bubble" forms at the mismatch of the two DNA strands, which is then cleaved by a single stranded nuclease. The products are then separated by size, such as by HPLC. See also McCallum et al. "Targeted screening for induced mutations"; Nat Biotechnol. 2000 Apr;18(4):455-7 and McCallum et al. "Targeting induced local lesions IN genomes (TILLING) for plant functional genomics"; Plant Physiol. 2000 Jun;123(2):439-42. In certain embodiments, the mutant epsp synthase can be obtained by targeted mutagenesis, such as gene editing techniques, including CRISPR/Cas (such as CRISPR/Cas9 or CRISPRCpf1), zinc finger nucleases, meganucleases, or TALEN gene editing techniques, as are known in the art.

In an aspect, the invention relates to a method for detecting or identifying the epsp (or ALS) mutations according to the invention as described herein. Any means of detection can be applied, as described herein elsewhere, and include for instance sequencing, hybridization based methods (such as (dynamic) allele-specific hybridization, molecular beacons, SNP microarrays), enzyme based methods (such as PCR, KASP (Kompetitive Allele Specific PCR), RFLP, ALFP, RAPD, Flap endonuclease, primer extension, 5'-nuclease, oligonucleotide ligation assay), post-amplification methods based on physical properties of DNA (such as single strand conformation polymorphism, temperature gradient gel electrophoresis, denaturing high performance liquid chromatography, high-resolution melting of the entire amplicon, use of DNA mismatch-binding proteins, SNPlex, surveyor nuclease assay), etc.

In certain embodiments, detection is performed by KASP. In certain embodiments, a KASP-marker is s1txepss02 (SEQ ID NOs: 17-19). Preferably, this KASP marker is useful for detecting single nucleotide point mutation in the endogenous epsps gene of Beta vulgaris causing P179S amino acid change in BvEPSPS.

"Fermentation" as used herein refers to the process of transforming an organic molecule into another molecule using a micro-organism. For example, "fermentation" can refer to aerobic transforming sugars or other molecules from plant material, such as the plant material of the present invention, to produce alcohols (e.g., ethanol, methanol, butanol); organic acids (e.g., citric acid, acetic acid, itaconic acid, lactic acid, gluconic acid); ketones (e.g., acetone), amino acids (e.g., glutamic acid); gases (e.g., H2 and CO2), antibiotics (e.g., penicillin and tetracycline); enzymes; vitamins (e.g., riboflavin, B12, beta-carotene); and/or hormones. Fermentation include fermentations used in the consumable alcohol industry (e.g., beer and wine). Fermentation also includes anaerobic fermentations, for example, for the production of biofuels. Fermenting can be accomplished by any organism suitable for use in a desired fermentation step, including, but not limited to, bacteria, fungi, archaea, and protists. Suitable fermenting organisms include those that can convert mono-, di-, and trisaccharides, especially glucose and maltose, or any other biomass-derived molecule, directly or indirectly to the desired fermentation product (e.g., ethanol, butanol, etc.).

Suitable fermenting organisms also include those which can convert non-sugar molecules to desired fermentation products. Such organisms and fermentation methods are known to the person skilled in the art.

The term "biofuel", as used herein, refers to a fuel that is derived from biomass, i.e., a living or recently living biological organism, such as a plant or an animal waste. Biofuels include, but are not limited to, biodiesel, biohydrogen, biogas, biomass-derived dimethylfuran (DMF), and the like. In particular, the term "biofuel" can be used to refer to plant-derived alcohols, such as ethanol, methanol, propanol, or butanol, which can be denatured, if desired prior to use. The term "biofuel" can also be used to refer to fuel mixtures comprising plant-derived fuels, such as alcohol/gasoline mixtures (i.e., gasohols). Gasohols can comprise any desired percentage of plant-derived alcohol (i.e., about 10%, 15%, 20%, 25%, 30%, 35%, 40%, 45%, 50%, 55%, 60%, 65%, 70%, 75%, 80%, 85%, 90%, or 95% plant -derived alcohol). For example, one useful biofuel-based mixture is E85, which comprises 85% ethanol and 15% gasoline. The biofuel can be any biofuel produced by aerobic or anaerobic fermentation of plant material. A non-limiting example of a biofuel obtained by aerobic fermentation is bioethanol. Biofuels that can be obtained by anaerobic fermentation include, but are not limited to biogas and/or biodiesel. Methods of aerobic and/or anaerobic fermentation are known to the person skilled in the art. Further encompassed by the present invention are biofuels selected from the group comprising ethanol, biogas and/or biodiesel as produced by the method for producing one or more biofuel(s) or the present invention.

The present invention includes other industrial applications such as the production of antibodies or bioplastic in the sugar beet plants of the present invention. Further, sugar beet plants of the present invention or parts thereof can also be used without further processing, such as, for example, as cattle feed.

The term "sugar" refers to fermentable monosaccharides disaccharides, and trisaccharides, particularly to mono- and disaccharides. Thus, in the present invention sugars include, but are not limited to, sucrose, fructose, glucose, galactose, maltose, lactose, and mannose, preferably sucrose.

The aspects and embodiments of the invention are further supported by the following non-limiting examples.

### EXAMPLES

6 kg seeds of sugar beet elite line T807 (3BT1760, M0 population) were mutagenized with 0.5% EMS and 0.3% and 0.5%, respectively ENU, and subsequently drilled for steck production. Stecks were replanted for seed production occupying an area of 5.8 ha. M1 population sizes were 75,000 seed producing plants for EMS and 19,000 plants for ENU mutagenized seeds. The following M2 seed amounts (values given for purified seed) has been obtained: 240 kg for 0.5% EMS (idents A and B), 548 kg for 0.3% ENU (idents C and D), and 256 kg 0.5% ENU (idents E and F).

176 kg of idents A and B and 75 kg of idents C, D, E and F were sown on an approximately 15 ha spanning field site. Approximately 1 month after sowing, the entire field was sprayed with 0.88 l/ha ROUNDUP® MAX (680 g/kg glyphosate acid equivalent). Approximately 6 weeks later, plants surviving the glyphosate treatment were collected in the field, transplanted to pots and further cultivated in the greenhouses. Surviving plants were treated with 600 g/ha glyphosate acid equivalent about 2 months later. 172 plants survived that treatment in a very healthy stage without any signs of herbicide damage.

In order to identify the causative mutation behind the observed herbicide resistance sequencing analyses have been performed. Inter alia, the exon 2 of the epsp synthase gene in sugar beet was PCR amplified (primers BvEPSPS_Ex_2_for, 5'-ggaaatttccatcctaacgag-3' (SEQ ID NO: 13), and BvEPSPS_Ex_2_rev, 5'-gcaagaggaaacaagtctcca-3' (SEQ ID NO: 14)) and Sanger sequenced (primers BvEPSPS_Ex2_Seqf, 5'- catcctaacgagaattatgc -3' (SEQ ID NO: 15), and BvEPSPS_Ex2_Seqr, 5'- gtctccacacaaaataaaag -3' (SEQ ID NO: 16)) in all 172 surviving plants. The sugar beet plant with identifier 6MS1008-109 carried a C to T mutation that causes the P179S amino acid change in BvEPSPS. This artificial SNP has been independently confirmed by KASP marker assay using an in silico developed KASP-marker s1txepss02 (SEQ ID NOs: 17-19):
SEQ ID NO: 17 KASP-marker s1txepss02 - Primer_Allel_C
   GAAGGTGACCAAGTTCATGCTCAGCAACTGCAGCTGTCAATGG
SEQ ID NO: 18 KASP-marker s1txepss02 - Primer_Allel_T
   GAAGGTCGGAGTCAACGGATTAACAGCAACTGCAGCTGTCAATGA
SEQ ID NO: 19 KASP-marker s1txepss02 - Primer_Common
   CTTTTTCTTGGAAATGCAGGAACAGCAAT

The genomic DNA nucleotide sequence of a mutant epsp synthase gene, carrying a mutation causing the P179S amino acid change according certain embodiments of the invention is provided in SEQ ID NO: 1. The cDNA nucleotide sequence of a mutant epsp synthase gene, carrying a mutation causing the P179S amino acid change according certain embodiments of the invention is provided in SEQ ID NO: 2. The protein sequence of a mutant epsp synthase gene, carrying a P179S mutation according certain embodiments of the invention is provided in SEQ ID NO: 3. The genomic DNA nucleotide sequence of a mutant epsp synthase gene, carrying a mutation causing the P179S amino acid change and a mutation causing the T175I amino acid change according certain embodiments of the invention is provided in SEQ ID NO: 4. The cDNA nucleotide sequence of a mutant epsp synthase gene, carrying a mutation causing the P179S amino acid change and a mutation causing the T175I amino acid change according certain embodiments of the invention is provided in SEQ ID NO: 5. The protein sequence of a mutant epsp synthase gene, carrying a P179S mutation and a T175I mutation according certain embodiments of the invention is provided in SEQ ID NO: 6:
SEQ ID NO: 1 genomic DNA epsps P179S 
aggaagtatttgaatttgatatagatattgtgtctttgtgtgtgttgaatttcaattcccagttccctaaaaaaatttacaattgca
atttcgagattatgatgtaaattaaatttgagagactagaaagtatttggtcaacccaaaaaaaaatatcaatacttatata
aatcaaaaacataatagagaatccaattttactaaaaatattagtaattttgattaaaataatctattaaaatgaactctaacc
ttcacataatttccacatattattaatcaacaaaataagcatcacaaattattagaataggcgatctaattttaacataaaatt
agacgaattcaaattgaatttttctaacaagctcattccatttcacgcaacccaaaattatcctagtcagtagtcatccattcttt
tctcattcctttattcttgattatcgaactacaacagataatttcaaaaaaaaactaaattggtagtcttaactgattaaactactt
actaaatggattaaagaatgtcattactgaatagattaaactgattacgaaatagattaacttggtccctaaatagattaaat
tagttactatattaaaattaggcgatctcttacaaaaccaactgaataagcatagctctgtatattacctagatttcaactaaat
caaaacccccttacagttcaatctagagctgatcattttggctcggcccgtcccatttttgggccgggttttagtcagattttttgg
cccgcggtcgggcccggcccgatttttttggctttgggcaagccaaaaacgacttttcagtttatttttggcccgacccgttttt
acccgcaaaagcccgctaatttaggtccgcactttgggcacaaaaatttagcccgaacttaaacctggcccgacccatg
atcacctctagtttaatccaaactaaaaaactacacaagttagccaaaaattatgtctactttgtacaactttataaaataca
cacagtagttgatatcttgatgattaactccttttgaagtttgactacacaccaaccccaaacacacccactttttccccctct
tgtcaccaacccccccctcctctttagccaccaaagtttggttggtgagtcctccataactgctaaattctctctttttctctctcct
 
aaaaaactaaaacccaccaaaatttcagacatcaaaaaaattacaagtgaaggaaacaataatggctcaagctagca
ccataaacaatggtgtcaaaagcacccaattatgccccaatttacccaaaacccacttatccaaatcttcaaaatctgtta
aatttggatcaaatttgagattttctccaaagttgaaatcttttaacaatgaaagagttggtgggaattcatcagttgttttcagg
gttagggcttcagttgcagcagcagctgaaaaatcatcaactgtaccagaaattgtgttacaacccatcaaagagatctc
gggtaccattcaattgcccggatccaagtcattatctaatcggattctacttcttgctgccctttctcaggtacttttcaattgtttg
atttctttttttcttagaacttgtgaatttgtatactttatccgtttctaaatacgtgcaacatttgaatagtaacgagtatttatctacc
aacttatttaatattctctcacgaatgtatatgaaaaaatatagtcatgcgtggttttatttgattgatctgcggacttttataatatc
aactttttataatttagaggacaaagtagtgtattgggtagcgtgtaaggaggttgggaaactggaggaatttttttaacaattc
aagtttgatatttttcatagtgaaatgtttattagcatagaatcatgcttttagtttttagtggagtgtgcatttattctttaacttgttgg
atggctatgattaagaattggattctggttatttgcttgagtatttagaaattaattgtgggtgttggtgataatgtaacaaaattg
ttttgaaggtgtgagaatgtgattttaattatatgaggaaatgatgggttatttattgtaatgtgggaatttatgataatatgttgga
ggatgaaacaattgatgattttgaagtggggtatgaaggctttcccccatttttttacctttcatgtgttgtatgtagcagactgc
caaagcaattcttctgagcagcttggcatatttccattgcaactcataatctcaatccaagcacaatcaattacctgccacgc
tagcactttaagtagcaaaatcgcgcttgaagaaaagataataccacaggcttctatagttctattccttgtgatataggaca
caagtatcatcctagatgacttccctagccctgcaaataattcctggaaggaagtccgttgagtatccttgatcaagtaaaa
ttagcgctttcttttgggtaacacaaactatactcgaaccactttatgctaagggatcacattgcgtctcttgcagaacttttaac
atttctaacatggcataagcacggaaacaccctgtgaagttgctagtgaccatctttttgataaatcaagaatccctgttctt
atttgcatacaaagggttaagtcttgtctgagagggattgacatatctcctgctggatagactttgtgttcatctctaatgttattc
tgagtttgcattgtatgtgttgtgttttcgtttttcttcttgtttgtgtgtgtgtgtgtgtgtgtgtgtgtgttacccaaacttgtacgga
aatttccatcctaacgagaattatgctgcagggaacaacagtggttgacaacttgctatatagtgatgatatccgttacatgc
tggatgctttgagaactcttgggttgaatgtggaggatgataatatagccaaaagggcaatcgtggagggttgcggtggttt
gtttcctgttggaaaagatggaaagaaattgaactttttcttggaaatgcaggaacagcaatgcgctcattgacagctgc
agttgctgttgctggaggaaattctaggtttgtctactaaggccttccatgtcatcgtgaggctgttaatattcttttattttgtgtgg
agacttgtttcctcttgctacaattgcaaagggggctgggattgattaacaatgcagaactcaaacactatctttggtatcac
cgtctggcaggtggcatctgatcttgcaaaacagatatggagggttttgccggaaaggggttgggggtgggacttgggag
tatagcctcaatgttatagaagattgtgctatactactgtaaaattaacttttggttggaaccagatatagttaataaagtattttt
ttatctggtgaggggtgcttctaggtcctgaaaagttccaccatagaaaaaaattgaatttcattgtgaaaagctaaggtga
attcattaaattcaatacatgttgttgattgcttgaactagaatacttcagatatcagttaattgaatttatttcttttggagaacatg
catgaagagcttttaactcgcctctactatcctgctcttaattatttttataaatagatttagcaattgggaatatcctgtaatttgct
ggttgctttttcatatggtatatattgaattggtgtggaggcgttttacaaagagtgaaggtaattgttgatgggtggatattgaa
tggaaatactttgatggcggatagcatattcatctgattggatgtctgaaatttgatgagctctagtaaatgttcctccatgtatg
cttgtgtattgggaatttgcaatccagtagtagagaacacccctgataattgcactattattcctgagttgtgagagctctcaa
gaagatagatggggttcttcaagtttttaattgtctttaggtagtgtaaaggttgcaatatttcaaaggatctgtctgatccaac
aagtctgctaaatccttcaccaatctgaaaggggagaagatactgctctctctgacctgcttgcatgcatataaagcttctaa
aacaaataggttaaatgtaaaacaagcgcagaagttttcacttctcttgggaaatgtgtgccaagatctattgcatgacatg
 
aattcatatttattgattatatgctcaacatgaatgggattaagaatgaaataaagtagcatgagtaggagacaatccgcct
gctcttttaataggcatatacaatcagtcatcaacatcattgctgctttagaatttgatttatgttgatcttggtttttgcagctatgt
acttgatggagtgccgagaatgagggagcgacccattggggatttggtagcgggtctaaagcaacttggcgccgatgtt
gactgttatcttggcacaaattgtcctcctgttcgagtgaatgctaaaggaggccttcccgggggcaaggtgaattctcttgtt
ttgattagagaaaaattataagttagtgttctatcatgcttgacacgaacaagtttttttatctgaaagagaaagaagtgtatg
cctaatgattaacagtcatattcaaaatgatataaaagagggtggaaggttacttgctctatgatgatttatggtagtaccgtg
ttaccaatagcagaattcaagcaatattctttgaactggtaataacaagttcaagcaatcaagcatagtttagattagattct
atcaatctcttagcatagtttaaattcgcttccatcaatctcttttgtgacgtaagaagttctgttccattatacttatgctttttgtga
aattttatttgcaggtcaagctctctggatcagttagtagccaatatttgactgcactgcttatggctactcctttgggtcttggag
acgtggaggttgaaatcattgataaattgatttctgtaccatatgtggagatgacaataaagctaatggaaaggtttggagt
gtctgtagagcatagtgctgactggggtaggttcttgatccgaggtggtcagaagtacaagtaagtctctctttttttactacgt
gtcctgaaaaatctatgttttagtagactaagatactataaaatacatcagatctcctggaaatgcgtatgttgagggtgatg
cttcaagtgctagttacttcataggaggggccgcagtcactggtgggactgtgactgttgagggttgtggaacaagtagttt
acaggtatgatttaaagccttatttcacatccttttacttctctccttactgcatctcagcttaattctgaagaactcttgttgctcaa
tctctaggcataagcttgttctttcgaccttctaaatttgcaaaatcattctgttaatgtaacaaacagaagatgagatggtgttt
ttgatcctgataaaaaaaattaggacattcatgattggtttatgttctatgacaatttcgatgatctcttcaattgtaacttgagga
cccttttctgctagtaaagattgtgtataagctattctagactgttgtatacacctcatattccggtattgtttatacctaataaactt
cttatgattagtatcttacttgtctcatgtgttttgcatagggtgatgtaaaatttgctgaagttcttgagaagatgggttgcaagg
tatcctggacagagaacagtgtcactgtcactggacccacccagggatgcatctggaagaaacacttgcgcgccgttga
tgtcaacatgaacaaaatgccagatgttgcaatgactcttgctgttgttgctctttatgcagatggacccaccaccattagag
acggtatgcttaattcctttctgtgaacatgacactcttcttgtgccataggtagattccgtagttattggtcacaatccaaatttg
cattggtatttaaagcaagtcttaggtggtgagaccgcttgaaacccataatataaaagaaaggtcaccttggtctcaaag
ttaattcttgtgagaccatctcatgcaagattttgccgggtatctaatccttgtttgcactgaggaaaaggaaaaaaattaac
atgacctattagtaagccaaaaaaattctaacaagagaagtagccgaataggtaataatttttgtgatataatgatcagtg
gctagctggagagtgaaggaaacagaacggatgattgcgatttgcacagagctcagaaaggttagccattttggatata
cacattcttgaaggttttcaaccttgtagaagattgtactattgataaaaaaaattgatgtttatccggtgtacttcgaaatttcttt
tcagctgggggcaacagttgaggaaggatcagattactgtgtgatcactccacctgagaaactaaatgtgacggccattg
atacatacgatgatcaccgaatggccatggcattctctcttgctgcctgcgccgatgttcctgttaccatcaaggacccgggt
tgcactcgcaagactttcccagactactttgatgtgttggaaaggtttgcaaagcattaagtggtctcctacatattctataaa
gcataagctgagatttttgagagaattaggagatgaaaaatgctttctgcttgagttatcatcacattctttgtattatgattgta
agattattatagtatagagtttacaaagtactactaataattgttatgtatccgattgatcagaaataagttaattggaaggctg
gactttgaaaatgtgaccaagacactagtgtgaccaagtcattttgttaatgtgagttcaatgttattgattcaacatgtagag
ccaaatctcaattctatcgtcacttcatatgaccaaaaatctaaagatgaaaaagtaaaaaagagcatgttggatcaaact
ctagctgtatctctgaaattcaatcacgcagttagatcaaatgaggattaaagggagt
 
SEQ ID NO: 2 cDNA epsps P179S
atggctcaagctagcaccataaacaatggtgtcaaaagcacccaattatgccccaatttacccaaacccacttatccaa
atcttcaaaatctgttaaatttggatcaaatttgagattttctccaaagttgaaatcttttaacaatgaaagagttggtgggaatt
catcagttgttttcagggttagggcttcagttgcagcagcagctgaaaaatcatcaactgtaccagaaattgtgttacaacc
catcaaagagatctcgggtaccattcaattgcccggatccaagtcattatctaatcggattctacttcttgctgccctttctcag
ggaacaacagtggttgacaacttgctatatagtgatgatatccgttacatgctggatgctttgagaactcttggttgaatgtg
gaggatgataatatagccaaaagggcaatcgtggagggttgcggtggtttgtttcctgttggaaaagatggaaagaaat
tgaactttttcttggaaatgcaggaacagcaatgcgctcattgacagctgcagttgctgttgctggaggaaattctagctatgt
acttgatggagtgccgagaatgagggagcgacccattggggatttggtagcgggtctaaagcaacttggcgccgatgtt
gactgttatcttggcacaaattgtcctcctgttcgagtgaatgctaaaggaggccttcccgggggcaaggtcaagctctctg
gatcagttagtagccaatatttgactgcactgcttatggctactcctttgggtcttggagacgtggaggttgaaatcattgata
aattgatttctgtaccatatgtggagatgacaataaagctaatggaaaggtttggagtgtctgtagagcatagtgctgactg
ggtaggttcttgatccgaggtggtcagaagtacaaatctcctggaaatgcgtatgttgagggtgatgcttcaagtgctagtt
acttcataggaggggccgcagtcactggtgggactgtgactgttgagggttgtggaacaagtagtttacagggtgatgtaa
aatttgctgaagttcttgagaagatgggttgcaaggtatcctggacagagaacagtgtcactgtcactggaccacccagg
gatgcatctggaagaaaacacttgcgcgccgttgatgtcaacatgaacaaaatgccagatgttgcaatgactcttgctgtt
gttgctctttatgcagatggacccaccaccattagagacgtggctagctggagagtgaaggaaacagaacggatgattg
cgatttgcacagagctcagaaagctgggggcaacagttgaggaaggatcagattactgtgtgatcactccacctgagaa
actaaatgtgacggccattgatacatacgatgatcaccgaatggccatggcattctctcttgctgcctgcgccgatgttcctg
ttaccatcaaggacccgggttgcactcgcaagactttcccagactactttgatgtgttggaaaggtttgcaaagcattaa
SEQ ID NO: 3 protein epsps P179S 
MAQASTINNGVKSTQLCPNLPKTHLSKSSKSVKFGSNLRFSPKLKSFNNERVGGNSS
VVFRVRASVAAAAEKSSTVPEIVLQPIKEISGTIQLPGSKSLSNRILLLAALSQGTTVVD
NLLYSDDIRYMLDALRTLGLNVEDDNIAKRAIVEGCGGLFPVGKDGKEIELFLGNAGTA
MRSLTAAVAVAGGNSSYVLDGVPRMRERPIGDLVAGLKQLGADVDCYLGTNCPPVR
VNAKGGLPGGKVKLSGSVSSQYLTALLMATPLGLGDVEVEIIDKLISVPYVEMTIKLME
RFGVSVEHSADWGRFLIRGGQKYKSPGNAYVEGDASSASYFIGGAAVTGGTVTVEG
CGTSSLQGDVKFAEVLEKMGCKVSWTENSVTVTGPPRDASGRKHLRAVDVNMNKM
PDVAMTLAVVALYADGPTTIRDVASWRVKETERMIAICTELRKLGATVEEGSDYCVITP
PEKLNVTAIDTYDDHRMAMAFSLAACADVPVTIKDPGCTRKTFPDYFDVLERFAKH
SEQ ID NO: 4 genomic DNA epsps T175I P179S 
aggaagtatttgaatttgatatagatattgtgtctttgtgtgtgttgaatttcaattcccagttccctaaaaaaatttacaattgca
atttcgagattatgatgtaaattaaatttgagagactagaaagtatttggtcaacccaaaaaaaaaatatcaatacttatata
 
aatcaaaaacataatagagaatccaatttactaaaatattagtaatttgattaaataatctattaaatgaactctaacc
ttcacataatttccacatattattaatcaacaaaataagcatcacaaattattagaataggcgatctaattttaacataaaatt
agacgaattcaaattgaatttttctaacaagctcattccatttcacgcaacccaaaattatcctagtcagtagtcatccattcttt
tctcattcctttattcttgattatcgaactacaacagataatttcaaaaaaaaactaaattggtagtcttaactgattaaactactt
actaaatggattaaagaatgtcattactgaatagattaaactgattacgaaatagattaacttggtccctaaatagattaaat
tagttactatattaaaattaggcgatctcttacaaaaccaactgaataagcatagctctgtatattacctagatttcaactaaat
caaaaccccttacagttcaatctagagctgatcattttggctcggcccgtcccattttgggccgggtttagtcagatttttttgg
cccgcggtcgggcccggcccgatttttttggctttgggcaagccaaaaacgactttcagtttatttttggcccgacccgttttt
acccgcaaaagcccgctaatttaggtccgcactttgggcacaaaaatttagcccgaacttaaacctggcccgacccatg
atcacctctagtttaatccaaactaaaaaactacacaagttagccaaaaattatgtctactttgtacaactttataaaataca
cacagtagttgatatcttgatgattaactccttttgaagtttgactacacaccaaccccaaacacacccactttttccccccctct
tgtcaccaacccccccctcctctttagccaccaaagtttggttggtgagtcctccataactgctaaattctctctttttctctctcct
aaaaaactaaaacccaccaaaatttcagacatcaaaaaaattacaagtgaaggaaacaataatggctcaagctagca
ccataaacaatggtgtcaaaagcacccaattatgccccaatttacccaaaacccacttatccaaatcttcaaaatctgtta
aatttggatcaaatttgagattttctccaaagttgaaatcttttaacaatgaaagagttggtgggaattcatcagttgttttcagg
gttagggcttcagttgcagcagcagctgaaaaatcatcaactgtaccagaaattgtgttacaacccatcaaagagatctc
gggtaccattcaattgcccggatccaagtcattatctaatcggattctacttcttgctgccctttctcaggtacttttcaattgtttg
atttcttttttcttagaacttgtgaattgtatactttatccgtttctaaatacgtgcaacatttgaatagtaacgagtatttatctacc
aacttatttaatattctctcacgaatgtatatgaaaaaatatagtcatgcgtggttttatttgattgatctgcggacttttataatatc
aactttttataatttagaggacaaagtagtgtattgggtagcgtgtaaggaggttgggaaactggaggaatttttaacaattc
aagtttgatatttttcatagtgaaatgtttattagcatagaatcatgcttttagtttttagtggagtgtgcatttattctttaacttgttgg
atggctatgattaagaattggattctggttatttgcttgagtatttagaaattaattgtgggtgttggtgataatgtaacaaaattg
ttttgaaggtgtgagaatgtgattttaattatatgaggaaatgatgggttatttattgtaatgtgggaatttatgataatatgttgga
ggatgaaacaattgatgattttgaagtggggtatgaaggctttcccccatttttttacctttcatgtgttgtatgtagcagactgc
caaagcaattcttctgagcagcttggcatatttccattgcaactcataatctcaatccaagcacaatcaattacctgccacgc
tagcactttaagtagcaaaatcgcgcttgaagaaaagataataccacaggcttctatagttctattccttgtgatataggaca
caagtatcatcctagatgacttccctagccctgcaaataattcctggaaggaagtccgttgagtatccttgatcaagtaaaa
ttagcgctttcttttgggtaacacaaactatactcgaaccactttatgctaagggatcacattgcgtctcttgcagaacttttaac
atttctaacatggcataagcacggaaacaccctgtgaagttgctagtgaccatctttttgataaatcaagaatccctgttctt
atttgcatacaaagggttaagtcttgtctgagagggattgacatatctcctgctggatagactttgtgttcatctctaatgttattc
tgagtttgcattgtatgtgttgtgttttcgtttttcttcttgtttgtgtgtgtgtgtgtgtgtgtgtgtgtgttacccaaacttgtacgga
aatttccatcctaacgagaattatgctgcagggaacaacagtggttgacaacttgctatatagtgatgatatccgttacatgc
tggatgctttgagaactcttgggttgaatgtggaggatgataatatagccaaaagggcaatcgtggagggttgcggtggttt
gtttcctgttggaaaagatggaaagaaattgaactttttcttggaaatgcaggaatagcaatgcgctcattgacagctgca
gttgctgttgctggaggaaattctaggtttgtctactaaggccttccatgtcatcgtgaggctgttaatattcttttattttgtgtgga
 
gacttgtttcctcttgctacaattgcaaagggggctgggattgattaacaatgcagaactcaaacactatctttggtatcacc
gtctggcaggtggcatctgatcttgcaaaacagatatggagggttttgccggaaaggggttgggggtgggacttgggagt
atagcctcaatgttatagaagattgtgctatactactgtaaaattaacttttggttggaaccagatatagttaataaagtatttttt
tatctggtgaggggtgcttctaggtcctgaaaagttccaccatagaaaaaaattgaatttcattgtgaaaagctaaggtgaa
ttcattaaattcaatacatgttgttgattgcttgaactagaatacttcagatatcagttaattgaatttatttcttttggagaacatgc
atgaagagcttttaactcgcctctactatcctgctcttaattatttttataaatagatttagcaattgggaatatcctgtaatttgctg
gttgctttttcatatggtatatattgaattggtgtggaggcgttttacaaagagtgaaggtaattgttgatgggtggatattgaat
ggaaatactttgatggcggatagcatattcatctgattggatgtctgaaatttgatgagctctagtaaatgttcctccatgtatg
cttgtgtattgggaatttgcaatccagtagtagagaacacccctgataattgcactattattcctgagttgtgagagctctcaa
gaagatagatggggttcttcaagtttttaattgtctttaggtagtgtaaaggttgcaatatttcaaaggatctgtctgatccaac
aagtctgctaaatccttcaccaatctgaaaggggagaagatactgctctctctgacctgcttgcatgcatataaagcttctaa
aacaaataggttaaatgtaaaacaagcgcagaagttttcacttctcttgggaaatgtgtgccaagatctattgcatgacatg
aattcatatttattgattatatgctcaacatgaatgggattaagaatgaaataaagtagcatgagtaggagacaatccgcct
gctcttttaataggcatatacaatcagtcatcaacatcattgctgctttagaatttgatttatgttgatcttggtttttgcagctatgt
acttgatggagtgccgagaatgagggagcgacccattggggatttggtagcgggtctaaagcaacttggcgccgatgtt
gactgttatcttggcacaaattgtcctcctgttcgagtgaatgctaaaggaggccttcccgggggcaaggtgaattctcttgtt
ttgattagagaaaaattataagttagtgttctatcatgcttgacacgaacaagtttttttatctgaaagagaaagaagtgtatg
cctaatgattaacagtcatattcaaaatgatataaaagagggtggaaggttacttgctctatgatgatttatggtagtaccgtg
ttaccaatagcagaattcaagcaatattctttgaactggtaataacaagttcaagcaatcaagcatagtttagattagattct
atcaatctcttagcatagtttaaattcgcttccatcaatctcttttgtgacgtaagaagttctgttccattatacttatgctttttgtga
aattttatttgcaggtcaagctctctggatcagttagtagccaatatttgactgcactgcttatggctactccttttgggtcttggag
acgtggaggttgaaatcattgataaattgatttctgtaccatatgtggagatgacaataaagctaatggaaaggtttggagt
gtctgtagagcatagtgctgactggggtaggttcttgatccgaggtggtcagaagtacaagtaagtctctctttttttactacgt
gtcctgaaaaatctatgttttagtagactaagatactataaaatacatcagatctcctggaaatgcgtatgttgagggtgatg
cttcaagtgctagttacttcataggaggggccgcagtcactggtgggactgtgactgttgagggttgtggaacaagtagttt
acaggtatgatttaaagccttatttcacatcctttttacttctctccttactgcatctcagcttaattctgaagaactcttgttgctcaa
tctctaggcataagcttgttctttcgaccttctaaatttgcaaaatcattctgttaatgtaacaaacagaagatgagatggtgttt
ttgatcctgataaaaaaaattaggacattcatgattggtttatgttctatgacaatttcgatgatctcttcaattgtaacttgagga
cccttttctgctagtaaagattgtgtataagctattctagactgttgtatacacctcatattccggtattgtttatacctaataaactt
cttatgattagtatcttacttgtctcatgtgttttgcatagggtgatgtaaaatttgctgaagttcttgagaagatgggttgcaagg
tatcctggacagagaacagtgtcactgtcactggaccacccagggatgcatctggaagaaaacacttgcgcgccgttga
tgtcaacatgaacaaaatgccagatgttgcaatgactcttgctgttgttgctctttatgcagatggacccaccaccattagag
acggtatgcttaattcctttctgtgaacatgacactcttcttgtgccataggtagattccgtagttattggtcacaatccaaatttg
cattggtatttaaagcaagtcttaggtggtgagaccgcttgaaacccataatataaagaaaggtcaccttggtctcaaag
ttaattcttgtgagaccatctcatgcaagattttgccgggtatctaatccttgtttgcactgaggaaaaggaaaaaaaattaac
 
atgacctattagtaagccaaaaaaattctaacaagagaagtagccgaataggtaataatttttgtgatataatgatcagtg
gctagctggagagtgaaggaaacagaacggatgattgcgatttgcacagagctcagaaaggttagccattttggatata
cacattcttgaaggttttcaaccttgtagaagattgtactattgataaaaaaattgatgtttatccggtgtacttcgaaatttcttt
tcagctgggggcaacagttgaggaaggatcagattactgtgtgatcactccacctgagaaactaaatgtgacggccattg
atacatacgatgatcaccgaatggccatggcattctctcttgctgcctgcgccgatgttcctgttaccatcaaggacccgggt
tgcactcgcaagactttcccagactactttgatgtgttggaaaggtttgcaaagcattaagtggtctcctacatattctataaa
gcataagctgagatttttgagagaattaggagatgaaaaatgctttctgcttgagttatcatcacattctttgtattatgattgta
agattattatagtatagagtttacaaagtactactaataattgttatgtatccgattgatcagaaataagttaattggaaggctg
gactttgaaaatgtgaccaagacactagtgtgaccaagtcattttgttaatgtgagttcaatgttattgattcaacatgtagag
ccaaatctcaattctatcgtcacttcatatgaccaaaaatctaaagatgaaaaagtaaaaaagagcatgttggatcaaact
ctagctgtatctctgaaattcaatcacgcagttagatcaaatgaggattaaagggagt
SEQ ID NO: 5 cDNA epsps T175I P179S 
atggctcaagctagcaccataaacaatggtgtcaaaagcacccaattatgccccaatttacccaaacccacttatccaa
atcttcaaaatctgttaaatttggatcaaatttgagattttctccaaagttgaaatctttaacaatgaaagagttggtgggaatt
catcagttgttttcagggttagggcttcagttgcagcagcagctgaaaaatcatcaactgtaccagaaattgtgttacaacc
catcaaagagatctcgggtaccattcaattgcccggatccaagtcattatctaatcggattctacttcttgctgccctttctcag
ggaacaacagtggttgacaacttgctatatagtgatgatatccgttacatgctggatgctttgagaactcttgggttgaatgtg
gaggatgataatatagccaaaagggcaatcgtggagggttgcggtggtttgtttcctgttggaaaagatggaaagaaat
tgaactttttcttggaaatgcaggaatagcaatgcgctcattgacagctgcagttgctgttgctggaggaaattctagctatgt
acttgatggagtgccgagaatgagggagcgacccattggggatttggtagcgggtctaaagcaacttggcgccgatgtt
gactgttatcttggcacaaattgtcctcctgttcgagtgaatgctaaaggaggccttcccgggggcaaggtcaagctctctg
gatcagttagtagccaatatttgactgcactgcttatggctactcctttgggtcttggagacgtggaggttgaaatcattgata
aattgatttctgtaccatatgtggagatgacaataaagctaatggaaaggtttggagtgtctgtagagcatagtgctgactg
ggtaggttcttgatccgaggtggtcagaagtacaaatctcctggaaatgcgtatgttgagggtgatgcttcaagtgctagtt
acttcataggaggggccgcagtcactggtgggactgtgactgttgagggttgtggaacaagtagtttacagggtgatgtaa
aatttgctgaagttcttgagaagatgggttgcaaggtatcctggacagagaacagtgtcactgtcactggaccacccagg
gatgcatctggaagaaaacacttgcgcgccgttgatgtcaacatgaacaaaatgccagatgttgcaatgactcttgctgtt
gttgctctttatgcagatggacccaccaccattagagacgtggctagctggagagtgaaggaaacagaacggatgattg
cgatttgcacagagctcagaaagctgggggcaacagttgaggaaggatcagattactgtgtgatcactccacctgagaa
actaaatgtgacggccattgatacatacgatgatcaccgaatggccatggcattctctcttgctgcctgcgccgatgttcctg
ttaccatcaaggacccgggttgcactcgcaagactttcccagactactttgatgtgttggaaaggtttgcaaagcattaa
SEQ ID NO: 6 protein epsps T175I P179S

MAQASTINNGVKSTQLCPNLPKTHLSKSSKSVKFGSNLRFSPKLKSFNNERVGGNSS
VVFRVRASVAAAAEKSSTVPEIVLQPIKEISGTIQLPGSKSLSNRILLLAALSQGTTVVD
NLLYSDDIRYMLDALRTLGLNVEDDNIAKRAIVEGCGGLFPVGKDGKEIELFLGNAGIA
MRSLTAAVAVAGGNSSYVLDGVPRMRERPIGDLVAGLKQLGADVDCYLGTNCPPVR
VNAKGGLPGGKVKLSGSVSSQYLTALLMATPLGLGDVEVEIIDKLISVPYVEMTIKLME
RFGVSVEHSADWGRFLIRGGQKYKSPGNAYVEGDASSASYFIGGAAVTGGTVTVEG
CGTSSLQGDVKFAEVLEKMGCKVSWTENSVTVTGPPRDASGRKHLRAVDVNMNKM
PDVAMTLAVVALYADGPTTIRDVASWRVKETERMIAICTELRKLGATVEEGSDYCVITP
PEKLNVTAIDTYDDHRMAMAFSLAACADVPVTIKDPGCTRKTFPDYFDVLERFAKH

The genomic DNA nucleotide sequence of a mutant ALS gene, carrying a mutation causing the W569L amino acid change according certain embodiments of the invention is provided in SEQ ID NO: 7. The cDNA nucleotide sequence of a mutant ALS gene, carrying a mutation causing the W569L amino acid change according certain embodiments of the invention is provided in SEQ ID NO: 8. The protein sequence of a mutant ALS gene, carrying a W569L mutation according certain embodiments of the invention is provided in SEQ ID NO: 9. The genomic DNA nucleotide sequence of a mutant ALS gene, carrying a mutation causing the W569L amino acid change and a mutation causing the P188S amino acid change according certain embodiments of the invention is provided in SEQ ID NO: 10. The cDNA nucleotide sequence of a mutant ALS gene, carrying a mutation causing the W569L amino acid change and a mutation causing the P188S amino acid change according certain embodiments of the invention is provided in SEQ ID NO: 11. The protein sequence of a mutant ALS gene, carrying a W569L mutation and a P188S mutation according certain embodiments of the invention is provided in SEQ ID NO: 12:
SEQ ID NO: 7 genomic DNA ALS W569L 
cgtggtaaggtttttcttcctattgggcctaggaagttttccagccttgtaaaatcttgtgtgctttttctctttcgtttttagttattttc
attccgcaatctaaattcgaaaattttccctcaactacaattcaaccccttcttgtatttggtctagtgttcatactagaataaca
caaaatcgtgattaaatctatgtattggattgatagagaaacataaactctttgaagaaacctaattatgttaggacttctaat
agtttccgtcacgttttcatttgtattagaattttagaggtttaactactataaagaattcatgttataatggaacttgagtaataca
agtcctgtaagatgagaagactaatcatgttagaagtataatcatgatagaagtctaattttattaatagtcttttaatgttaga
attctaaagtttttaggagcttaattaaggtagttttcctacctatataagattcctagtttcctattacttgtaataggattcttagttt
tataattatgaaataatttctaatcctaattgttttcttataaataggctacggctggtcacctacaccaataaaattcaaaagtt
tactgattaaatttgatagtttttcttcttatatgatctataagactagcttaattagaatattaagatttccccccttattccaccttca
 
aattagtgtacataactttctccattaaaatttcaagaaaccttttcctaattaaaccatatattctaaaactccattaatagga
acccctcgttcctcatcaatttttttttttaaaaggcttttttttcttcaacccatcatatccacatttacaagagcagggtattttggt
aagtttccatatatagaaagtggaatcgagcgcctccactcatttcctcctcaaaagaacaagaacaagaacgagaac
aagaacaaccatcctcattctctctccaaaactccaaacaacaacaatggcggctaccttcacaaacccaacattttccc
cttcctcaactccattaaccaaaaccctaaaatcccaatcttccatctcttcaaccctccccttttccacccctcccaaaaccc
caactccactctttcaccgtcccctccaaatctcatcctcccaatcccacaaatcatccgccattaaaacacaaactcaag
caccttcttctccagctattgaagattcatctttcgtttctcgatttggccctgatgaacccagaaaaggtccgatgtcctcgtt
gaagctcttgagcgtgaaggtgttaccaatgtgtttgcttaccctggtggtgcatctatggaaatccaccaagctctcacacg
ctctaaaaccatccgcaatgtcctccctcgccatgaacaaggcggggttttcgccgccgagggatatgctagagctactg
gaaaggttggtgtctgcattgcgacttctggtcctggtgctaccaacctcgtatcaggtcttgctgacgctctccttgattctgtc
cctcttgttgccatcactggccaagttccacgccgtatgattggcactgatgcttttcaggagactccaattgttgaggtgaca
aggtctattactaagcataattatttagttttggatgtagaggatattcctagaattgttaaggaagccttttttttagctaattctg
gtaggcctggacctgttttgattgatcttcctaaagatattcagcagcaattggttgttcctgattgggataggccttttaagttg
ggtgggtatatgtctaggctgccaaagtccaagttttcgacgaatgaggttggacttcttgagcagattgtgaggttgatgag
tgagtcgaagaagcctgtcttgtatgtgggaggtgggtgtttgaattctagtgaggagttgaggagatttgttgagttgacag
ggattccggtggctagtactttgatggggttggggtcttacccttgtaatgatgaactgtctcttcatatgttggggatgcacgg
gactgtttatgccaattatgcggtggataaggcggatttgttgcttgctttcggggttaggtttgatgatcgtgtgaccgggaag
ctcgaggcgtttgctagccgtgctaagattgtgcatattgatattgactctgctgagattgggaagaacaagcagccccatg
tgtccatttgtgctgatgttaaattggcattgcggggtatgaataagattctggagtctagaatagggaagctgaatttggattt
ctccaagtggagagaagaattaggtgagcagaagaaggaattcccactgagttttaagacatttggggatgcaattcctc
cacaatatgccattcaggtgcttgatgagttgaccaatggtaatgctattataagtactggtgttgggcagcaccaaatgtg
ggctgcgcagcattacaagtacagaaaccctcgccaatggctgacctctggtgggttgggggctatggggtttgggctac
cagccgccattggagctgcagttgctcgaccagatgcagtggttgtcgatattgatggggatggcagttttattatgaatgttc
aagagttggctacaattagggtggaaaatctcccagttaagataatgctgctaaacaatcaacatttaggtatggttgtcca
attggaagataggttctataaagctaaccgggcacatacataccttggaaacccttccaaatctgctgatatcttccctgata
tgctcaaattcgctgaggcatgtgatattccttctgcccgtgttagcaacgtggctgatttgagggccgccattcaaacaatgt
tggatactccagggccgtacctgctcgatgtgattgtaccgcatcaagagcatgtgttgcctatgattccaagtggtgccggt
ttcaaggataccattacagagggtgatggaagaacctcttattgatcggtttaatgacggttggaaccatttaaagagggta
agctatattactgtatgtatattagtatgttcctggataatttagaagcttttgtctgttgtcttttgcagtttatgaagttagtttgctgt
tgtcatgttacttgttactttaaaaagctttttgtagtttttgagcaactagtatggaatgctcttcctgtattgcttggaaaattcac
aaaagtggtttttcggctatggatgttgtgttgcatcatgcatatatagcttgatatactagttggcttggtgcatctttaacatata
ctaatgagactacgacagcaattgccaattagttggcttgacataattcttagtctgccactagaaatcttgcttcttttttttccct
catttgttgaaagtccctgttgcgacctgacatgggagcattggagattgtttagcaatagcagttgcagttaggtgactaca
gtcatcttccaaatatgaatactctctggaggggaggaggttttacaaaatatgagtttttaacacatgagaaacttatattaa
acaaggttgagtcaccccatatttttcaaagttgacttttgtctgatttgggtgactatgcctgttgtatgcaataaactcgatgta
 
cataagacttgtataatccaatctaacccttcctggctgattatgaaaccgagtcggctaatttgttgcttgatcttcatgtgtga
gcctgatgccaggtgaccactagaggagtacctttcattgagatatcgattcggttaattggttcctcatatgggtctcaaaa
ctgaatttttctcaggctctcttctaaccagttgttgaattttatgaaacttcagtccagttaaaactttgatcccggatcagaattt
ctctgagttgttcttccgcctctcagttccactgttccagtgttcttggtcctcaacctgtactctgagctatttctgcaaacacctg
aagttcctgctgcccttggacaaatacagaatgcagcattagcatttatttaagacaagatgaatacttgctcccctgttagtt
atgctaattctgcattagtattctttaattttaattagatactgcaatccgtgaactctgcagtttcttgatctctctctgtttcaatttct
cctatcttatcgcccatttctttaggctttcgttatctatcgtctaattcaagagtaggtaactaggtacgggataaaactttttcat
gaagaacctatgttcccgtattatgctgtcccacaagcttcaactttctaccttgttttctatacgttggacaacttcttttgtgttga
actgatttaattgataaatgaataattttggataaagaaaattgattgaccaataatttatttattttagtttacgttttgtatatacc
gactgggctcatgtgagcacatttatgtgcacaatttttttttatgtgaaaacaaaactaagct
SEQ ID NO: 8 cDNA ALS W569L 
atggcggctaccttcacaaacccaacattttccccttcctcaactccattaaccaaaaccctaaaatcccaatcttccatctc
ttcaaccctccccttttccacccctcccaaaaccccaactccactctttcaccgtcccctccaaatctcatcctcccaatccca
caaatcatccgccattaaaacacaaactcaagcaccttcttctccagctattgaagattcatctttcgtttctcgatttggccct
gatgaacccagaaaagggtccgatgtcctcgttgaagctcttgagcgtgaaggtgttaccaatgtgtttgcttaccctggtg
gtgcatctatggaaatccaccaagctctcacacgctctaaaaccatccgcaatgtcctccctcgccatgaacaaggcgg
ggttttcgccgccgagggatatgctagagctactggaaaggttggtgtctgcattgcgacttctggtcctggtgctaccaacc
tcgtatcaggtcttgctgacgctctccttgattctgtccctcttgttgccatcactggccaagttccacgccgtatgattggcact
gatgcttttcaggagactccaattgttgaggtgacaaggtctattactaagcataattatttagttttggatgtagaggatattcc
tagaattgttaaggaagcctttttttagctaattctggtaggcctggacctgttttgattgatcttcctaaagatattcagcagca
attggttgttcctgattgggataggccttttaagttgggtgggtatatgtctaggctgccaaagtccaagttttcgacgaatgag
gttggacttcttgagcagattgtgaggttgatgagtgagtcgaagaagcctgtcttgtatgtgggaggtgggtgtttgaattct
agtgaggagttgaggagatttgttgagttgacagggattccggtggctagtactttgatggggttggggtcttacccttgtaat
gatgaactgtctcttcatatgttggggatgcacgggactgtttatgccaattatgcggtggataaggcggatttgttgcttgcttt
cggggttaggtttgatgatcgtgtgaccgggaagctcgaggcgtttgctagccgtgctaagattgtgcatattgatattgact
ctgctgagattgggaagaacaagcagccccatgtgtccatttgtgctgatgttaaattggcattgcggggtatgaataagat
tctggagtctagaatagggaagctgaatttggatttctccaagtggagagaagaattaggtgagcagaagaaggaattc
ccactgagttttaagacatttggggatgcaattcctccacaatatgccattcaggtgcttgatgagttgaccaatggtaatgct
attataagtactggtgttgggcagcaccaaatgtgggctgcgcagcattacaagtacagaaaccctcgccaatggctga
cctctggtgggttgggggctatggggtttgggctaccagccgccattggagctgcagttgctcgaccagatgcagtggttgt
cgatattgatggggatggcagttttattatgaatgttcaagagttggctacaattagggtggaaaatctcccagttaagataa
tgctgctaaacaatcaacatttaggtatggttgtccaattggaagataggttctataaagctaaccgggcacatacatacctt
ggaaacccttccaaatctgctgatatcttccctgatatgctcaaattcgctgaggcatgtgatattccttctgcccgtgttagca
 
acgtggctgatttgagggccgccattcaaacaatgttggatactccagggccgtacctgctcgatgtgattgtaccgcatca
agagcatgtgttgcctatgattccaagtggtgccggtttcaaggataccattacagagggtgatggaagaacctcttattga
SEQ ID NO: 9 protein ALS W569L 
MAATFTNPTFSPSSTPLTKTLKSQSSISSTLPFSTPPKTPTPLFHRPLQISSSQSHKSSA
IKTQTQAPSSPAIEDSSFVSRFGPDEPRKGSDVLVEALEREGVTNVFAYPGGASMEIH
QALTRSKTIRNVLPRHEQGGVFAAEGYARATGKVGVCIATSGPGATNLVSGLADALLD
SVPLVAITGQVPRRMIGTDAFQETPIVEVTRSITKHNYLVLDVEDIPRIVKEAFFLANSG
RPGPVLIDLPKDIQQQLVVPDWDRPFKLGGYMSRLPKSKFSTNEVGLLEQIVRLMSES
KKPVLYVGGGCLNSSEELRRFVELTGIPVASTLMGLGSYPCNDELSLHMLGMHGTVY
ANYAVDKADLLLAFGVRFDDRVTGKLEAFASRAKIVHIDIDSAEIGKNKQPHVSICADV
KLALRGMNKILESRIGKLNLDFSKWREELGEQKKEFPLSFKTFGDAIPPQYAIQVLDEL
TNGNAIISTGVGQHQMWAAQHYKYRNPRQWLTSGGLGAMGFGLPAAIGAAVARPDA
VVVDIDGDGSFIMNVQELATIRVENLPVKIMLLNNQHLGMVVQLEDRFYKANRAHTYL
GNPSKSADIFPDMLKFAEACDIPSARVSNVADLRAAIQTMLDTPGPYLLDVIVPHQEHV
LPMIPSGAGFKDTITEGDGRTSY
SEQ ID NO: 10 genomic DNA ALS P188S W569L 
cgtggtaaggtttttcttcctattgggcctaggaagttttccagccttgtaaaatcttgtgctttttctctttcgtttttagttattttc
attccgcaatctaaattcgaaaattttccctcaactacaattcaacccccttcttgtatttggtctagtgttcatactagaataaca
caaaatcgtgattaaatctatgtattggattgatagagaaacataaactctttgaagaaacctaattatgttaggacttctaat
agtttccgtcacgttttcatttgtattagaattttagaggtttaactactataaagaattcatgttataatggaacttgagtaataca
agtcctgtaagatgagaagactaatcatgttagaagtataatcatgatagaagtctaattttattaatagtcttttaatgttaga
attctaaagtttttaggagcttaattaaggtagttttcctacctatataagattcctagtttcctattacttgtaataggattcttagttt
tataattatgaaataatttctaatcctaattgttttcttataaataggctacggctggtcacctacaccaataaaattcaaaagtt
tactgattaaatttgatagtttttcttcttatatgatctataagactagcttaattagaatattaagatttccccccttattccaccttca
aattagtgtacataactttctccattaaaaatttcaagaaacctttttcctaattaaaccatatattctaaaactccattaatagga
acccctcgttcctcatcaattttttttttttaaaaggctttttttcttcaacccatcatatccacatttacaagagcagggtattttggt
aagtttccatatatagaaagtggaatcgagcgcctccactcatttcctcctcaaaagaacaagaacaagaacgagaac
aagaacaaccatcctcattctctctccaaaactccaaacaacaacaatggcggctaccttcacaaacccaacattttccc
cttcctcaactccattaaccaaaaccctaaaatcccaatcttccatctcttcaaccctccccttttccacccctcccaaaaccc
caactccactctttcaccgtcccctccaaatctcatcctcccaatcccacaaatcatccgccattaaaacacaaactcaag
caccttcttctccagctattgaagattcatctttcgtttctcgatttggccctgatgaacccagaaaagggtccgatgtcctcgtt
gaagctcttgagcgtgaaggtgttaccaatgtgtttgcttaccctggtggtgcatctatggaaatccaccaagctctcacacg
ctctaaaaccatccgcaatgtcctccctcgccatgaacaaggcggggttttcgccgccgagggatatgctagagctactg
 
gaaaggttggtgtctgcattgcgacttctggtcctggtgctaccaacctcgtatcaggtcttgctgacgctctccttgattctgtc
cctcttgttgccatcactggccaagtttcacgccgtatgattggcactgatgcttttcaggagactccaattgttgaggtgaca
aggtctattactaagcataattatttagttttggatgtagaggatattcctagaattgttaaggaagcctttttttagctaattctg
gtaggcctggacctgttttgattgatcttcctaaagatattcagcagcaattggttgttcctgattgggataggccttttaagttg
ggtgggtatatgtctaggctgccaaagtccaagttttcgacgaatgaggttggacttcttgagcagattgtgaggttgatgag
tgagtcgaagaagcctgtcttgtatgtgggaggtgggtgtttgaattctagtgaggagttgaggagatttgttgagttgacag
ggattccggtggctagtactttgatggggttggggtcttacccttgtaatgatgaactgtctcttcatatgttggggatgcacgg
gactgtttatgccaattatgcggtggataaggcggatttgttgcttgctttcggggttaggtttgatgatcgtgtgaccgggaag
ctcgaggcgtttgctagccgtgctaagattgtgcatattgatattgactctgctgagattgggaagaacaagcagccccatg
tgtccatttgtgctgatgttaaattggcattgcggggtatgaataagattctggagtctagaatagggaagctgaatttggattt
ctccaagtggagagaagaattaggtgagcagaagaaggaattcccactgagttttaagacattggggatgcaattcctc
cacaatatgccattcaggtgcttgatgagttgaccaatggtaatgctattataagtactggtgttgggcagcaccaaatgtg
ggctgcgcagcattacaagtacagaaccctcgccaatggctgacctctggtgggttgggggctatggggtttgggctac
cagccgccattggagctgcagttgctcgaccagatgcagtggttgtcgatattgatggggatggcagttttattatgaatgttc
aagagttggctacaattagggtggaaaatctcccagttaagataatgctgctaaacaatcaacatttaggtatggttgtcca
attggaagataggttctataaagctaaccgggcacatacataccttggaaacccttccaaatctgctgatatcttccctgata
tgctcaaattcgctgaggcatgtgatattccttctgcccgtgttagcaacgtggctgatttgagggccgccattcaaacaatgt
tggatactccagggccgtacctgctcgatgtgattgtaccgcatcaagagcatgtgttgcctatgattccaagtggtgccggt
ttcaaggataccattacagagggtgatggaagaacctcttattgatcggtttaatgacggttggaaccatttaaagagggta
agctatattactgtatgtatattagtatgttcctggataatttagaagcttttgtctgttgtcttttgcagtttatgaagttagtttgctgt
tgtcatgttacttgttactttaaaaagctttttgtagtttttgagcaactagtatggaatgctcttcctgtattgcttggaaaattcac
aaaagtggtttttcggctatggatgttgtgttgcatcatgcatatatagcttgatatactagttggcttggtgcatctttaacatata
ctaatgagactacgacagcaattgccaattagttggcttgacataattcttagtctgccactagaaatcttgcttcttttttttccct
catttgttgaaagtccctgttgcgacctgacatgggagcattggagattgtttagcaatagcagttgcagttaggtgactaca
gtcatcttccaaatatgaatactctctggaggggaggaggttttacaaaatatgagtttttaacacatgagaaacttatattaa
acaaggttgagtcaccccatatttttcaaagttgacttttgtctgatttgggtgactatgcctgttgtatgcaataaactcgatgta
cataagacttgtataatccaatctaacccttcctggctgattatgaaaccgagtcggctaatttgttgcttgatcttcatgtgtga
gcctgatgccaggtgaccactagaggagtacctttcattgagatatcgattcggttaattggttcctcatatgggtctcaaaa
ctgaatttttctcaggctctcttctaaccagttgttgaattttatgaaacttcagtccagttaaaactttgatcccggatcagaattt
ctctgagttgttcttccgcctctcagttccactgttccagtgttcttggtcctcaacctgtactctgagctatttctgcaaacacctg
aagttcctgctgcccttggacaaatacagaatgcagcattagcatttatttaagacaagatgaatacttgctcccctgttagtt
atgctaattctgcattagtattctttaattttaattagatactgcaatccgtgaactctgcagtttcttgatctctctctgtttcaatttct
cctatcttatcgcccatttctttaggctttcgttatctatcgtctaattcaagagtaggtaactaggtacgggataaaactttttcat
gaagaacctatgttcccgtattatgctgtcccacaagcttcaactttctaccttgttttctatacgttggacaacttcttttgtgttga
 
actgatttaattgataaatgaataattttggataaagaaaattgattgaccaataatttatttattttagtttacgttttgtatatacc
gactgggctcatgtgagcacatttatgtgcacaattttttttatgtgaaaacaaaactaagct
SEQ ID NO: 11 cDNA ALS P188S W569L 
atggcggctaccttcacaaacccaacattttccccttcctcaactccattaaccaaaaccctaaatcccaatcttccatctc
ttcaaccctccccttttccacccctcccaaaaccccaactccactctttcaccgtcccctccaaatctcatcctcccaatccca
caaatcatccgccattaaaacacaaactcaagcaccttcttctccagctattgaagattcatctttcgtttctcgatttggccct
gatgaacccagaaaagggtccgatgtcctcgttgaagctcttgagcgtgaaggtgttaccaatgtgtttgcttaccctggtg
gtgcatctatggaaatccaccaagctctcacacgctctaaaaccatccgcaatgtcctccctcgccatgaacaaggcgg
ggttttcgccgccgagggatatgctagagctactggaaaggttggtgtctgcattgcgacttctggtcctggtgctaccaacc
tcgtatcaggtcttgctgacgctctccttgattctgtccctcttgttgccatcactggccaagtttcacgccgtatgattggcactg
atgcttttcaggagactccaattgttgaggtgacaaggtctattactaagcataattatttagttttggatgtagaggatattcct
agaattgttaaggaagcctttttttagctaattctggtaggcctggacctgttttgattgatcttcctaaagatattcagcagca
attggttgttcctgattgggataggccttttaagttgggtgggtatatgtctaggctgccaaagtccaagttttcgacgaatgag
gttggacttcttgagcagattgtgaggttgatgagtgagtcgaagaagcctgtcttgtatgtgggaggtgggtgtttgaattct
agtgaggagttgaggagatttgttgagttgacagggattccggtggctagtactttgatggggttggggtcttacccttgtaat
gatgaactgtctcttcatatgttggggatgcacggactgtttatgccaattatgcggtggataaggcggatttgttgcttgcttt
cggggttaggtttgatgatcgtgtgaccgggaagctcgaggcgtttgctagccgtgctaagattgtgcatattgatattgact
ctgctgagattgggaagaacaagcagccccatgtgtccatttgtgctgatgttaaattggcattgcggggtatgaataagat
tctggagtctagaatagggaagctgaatttggatttctccaagtggagagaagaattaggtgagcagaagaaggaattc
ccactgagttttaagacatttggggatgcaattcctccacaatatgccattcaggtgcttgatgagttgaccaatggtaatgct
attataagtactggtgttgggcagcaccaaatgtgggctgcgcagcattacaagtacagaaccctcgccaatggctga
cctctggtgggttgggggctatgggtttgggctaccagccgccattggagctgcagttgctcgaccagatgcagtggttgt
cgatattgatggggatggcagttttattatgaatgttcaagagttggctacaattagggtggaaaatctcccagttaagataa
tgctgctaaacaatcaacatttaggtatggttgtccaattggaagataggttctataaagctaaccgggcacatacatacctt
ggaaacccttccaaatctgctgatatcttccctgatatgctcaaattcgctgaggcatgtgatattccttctgcccgtgttagca
acgtggctgatttgagggccgccattcaaacaatgttggatactccagggccgtacctgctcgatgtgattgtaccgcatca
agagcatgtgttgcctatgattccaagtggtgccggtttcaaggataccattacagagggtgatggaagaacctcttattga
SEQ ID NO: 12 protein ALS P188S W569L

MAATFTNPTFSPSSTPLTKTLKSQSSISSTLPFSTPPKTPTPLFHRPLQISSSQSHKSSA
IKTQTQAPSSPAIEDSSFVSRFGPDEPRKGSDVLVEALEREGVTNVFAYPGGASMEIH
QALTRSKTIRNVLPRHEQGGVFAAEGYARATGKVGVCIATSGPGATNLVSGLADALLD
SVPLVAITGQVSRRMIGTDAFQETPIVEVTRSITKHNYLVLDVEDIPRIVKEAFFLANSG
RPGPVLIDLPKDIQQQLVVPDWDRPFKLGGYMSRLPKSKFSTNEVGLLEQIVRLMSES

KKPVLYVGGGCLNSSEELRRFVELTGIPVASTLMGLGSYPCNDELSLHMLGMHGTVY
ANYAVDKADLLLAFGVRFDDRVTGKLEAFASRAKIVHIDIDSAEIGKNKQPHVSICADV
KLALRGMNKILESRIGKLNLDFSKWREELGEQKKEFPLSFKTFGDAIPPQYAIQVLDEL
TNGNAIISTGVGQHQMWAAQHYKYRNPRQWLTSGGLGAMGFGLPAAIGAAVARPDA
VVVDIDGDGSFIMNVQELATIRVENLPVKIMLLNNQHLGMVVQLEDRFYKANRAHTYL
GNPSKSADIFPDMLKFAEACDIPSARVSNVADLRAAIQTMLDTPGPYLLDVIVPHQEHV
LPMIPSGAGFKDTITEGDGRTSY

There is, based on the screening and selection method, a very strong indication that a heterozygous P179S mutation in BvEPSPS confers complete resistance to 600 g/ha glyphosate acid equivalent which is approx. 50% of standard field treatment level. The heterozygous mutant is backcrossed and selfed and seeds are harvested. A first titration of the glyphosate resistance level for characterization of the selected mutant is performed. The P179S mutation confers resistance to glyphosate in heterozygous and homozygous state.

## Claims

1. Use of a glyphosate herbicide for controlling unwanted vegetation in Beta vulgaris growing areas in which the Beta vulgaris plants comprise an endogenous allele encoding an epsp synthase having at position 179 an amino acid different from proline.

2. Use of a glyphosate herbicide according to claim 1, wherein the glyphosate herbicide is selected from glyphosate or a derivative thereof, preferably wherein said derivative is a salt, ester, amide, or alkylamide; preferably wherein said salt is an alkali metal salt such as (mono-, di-, or tri-) sodium or (mono-, di-, or tri-) potassium, an ammonium salt, a di-ammonium salt such as dimethylammonium, an alkylamine salt such as C1-C16 alkylamine salts such as dimethylamine and isopropylamine salts, an alkylammonium salt such as C1-C16 alkylammonium salts such as dimethylammonium and isopropylammonium salts, an alkanolamine salt such as C1-C16 alkanolamine salts such as (mono-, di-, or tri-) ethanolamine salts, an alkylsulfonium salt such as trimethylsulfonium salts, a sulfoxonium salt, and mixtures or combinations thereof, preferably selected from the following salts: the mono(isopropylammonium) ("IPA"), potassium, sodium, monoethanolammonium ("MEA"), trimethylsulfonium ("TMS"), ammonium, diammonium salts, n-propylamine, ethylamine, ethylenediamine, and hexamethylenediamine salts, preferably the IPA salt.

3. Use of a glyphosate herbicide according to claim 1 or 2, wherein glyphosate herbicide is applied in a dosage of at least 300 g/ha glyphosate acid equivalent, preferably at least 600 g/ha glyphosate acid equivalent, more preferably at least 1200 g/ha glyphosate acid equivalent.

4. Use of a glyphosate herbicide according to any of claims 1 to 3 in combination with a non-glyphosate herbicide (i.e. herbicides showing a mode of action that is different to the inhibition of the epsp synthase), and wherein the non-glyphosate herbicide(s) is/are selected form the group consisting of: clopyralid, cycloxydim, desmedipham, dimethenamid, dimethenamid-P, ethofumesate, fenoxaprop, fenoxaprop-P, fenoxaprop-ethyl, fenoxaprop-P-ethyl, fluazifop, fluazifop-P, fluazifop-butyl, fluazifop-P-butyl, glufosinate, glufosinate-ammonium, glufosinate-P, glufosinate-P-ammonium, glufosinate-P-sodium, haloxyfop, haloxyfop-P, haloxyfop-ethoxyethyl, haloxyfop-P-ethoxyethyl, haloxyfop-methyl, haloxyfop-P-methyl, lenacil, metamitron, phenmedipham, phenmedipham-ethyl, propaquizafop, quinmerac, quizalofop, quizalofop-ethyl, quizalofop-P, quizalofop-P-ethyl, quizalofop-P-tefuryl, sethoxydim.

5. Use of a glyphosate herbicide according to any of claims 1 to 4, wherein all endogenous alleles encoding epsp synthases have at position 179 an amino acid different from proline.

6. Use of a glyphosate herbicide according to any of claims 1 to 5, wherein the endogenous allele encoding the epsp synthase has at position 179 an amino acid selected from the group of Serine, Threonine, Alanine and Leucine, preferably the amino acid is Serine.

7. Use of a glyphosate herbicide according to any of claims 1 to 6, wherein the epsp synthase having at position 179 an amino acid different from proline, comprises an amino acid sequence selected from
i) the sequence of SEQ ID NO: 3,
ii) the sequence i) having at position 179 an amino acid different from serine and proline, or
iii) a sequence having an identity of at least 90% to the sequence of i) or ii), preferably over the entire length of the sequence.

8. Use of a glyphosate herbicide according to any of claims 1 to 7, wherein the endogenous allele encoding an epsp synthase having at position 179 an amino acid different from proline, comprises a nucleotide sequence selected from
i) the sequence of SEQ ID NO: 1,
ii) a sequence having the coding sequence of SEQ ID NO: 2,
iii) the sequence of i) or ii) having nucleotides corresponding to amino acid position 179 and encoding an amino acid different from serine and proline,
iv) a sequence having an identity of at least 90% to the sequence of i), ii), or iii), preferably over the entire length of the sequence, or
v) a nucleotide sequence encoding the epsp synthase according to claim 7.

9. Use of a glyphosate herbicide according to any of claims 1 to 8, wherein the endogenous allele or all endogenous alleles encoding the epsp synthase(s) has/have additionally at position 175 an amino acid different from threonine, preferably the amino acid is isoleucine.

10. Use of a glyphosate herbicide according to any of claims 1 to 9, wherein the Beta vulgaris plants comprise an endogenous allele encoding an acetolactate synthase having at position 569 an amino acid different from tryptophan.

11. Use of a glyphosate herbicide according to claim 10, wherein all endogenous alleles encoding an acetolactate synthase have at position 569 an amino acid different from tryptophan.

12. Use of a glyphosate herbicide according to any of claims 10 to 11, wherein the endogenous allele or all endogenous alleles encoding the an acetolactate synthase(s) having additionally at position 188 an amino acid different from proline, preferably the amino acid is selected from the group consisting of serine, threonine, arginine, leucine, glutamine, alanine, more preferably the amino acid is serine.

13. Use of a glyphosate herbicide according to any of claims 10 to 12 in combination with an ALS inbibitor, preferably selected from the group consisting of: sulfonylurea, sulfonylaminocarbonyltriazolinone, triazolopyrimidine, sulfonanilide, imidazolinone, pyrimidinyloxybenzoeacid, pyrimidinylthiobenzoeacid.

14. Use of a glyphosate herbicide according to any of claims 10 to 13, wherein in step b) at least 300 g ha-1 active ingredient glyphosate is applied to the growing plants, preferably at least 600 g ha-1 active ingredient glyphosate is applied, more preferably at least 1200 g ha-1 and/or the ALS inhibitor is applied to the growing plants with a minimal dosage which is an equivalent to the mixtures of 35 g ha-1 foramsulfuron and 7 g ha-1 iodosulfuron-methyl-sodium.

15. Use of glyphosate herbicide according to any of claims 13, wherein the application of the respective herbicides (i) takes place jointly or simultaneously, or (ii) takes place at different times and/or in a plurality of portions (sequential application), in pre-emergence applications followed by post-emergence applications or early post-emergence applications followed by medium or late post-emergence applications.
